# EUROPEAN PATENT SPECIFICATION

(11) **EP 2 496 270 B1**
(45) Date of publication and mention of the grant of the patent: **31.01.2018**
(21) Application number: 10776064.7
(22) Date of filing: 04.11.2010
(51) Int. Cl.: A61K 51/10, C07K 16/32, C07C 259/06

(54) **ZIRCONIUM-RADIOLABELED, CYSTEINE ENGINEERED ANTIBODY CONJUGATES**
ZIRKONIUM-RADIOMARKIERTE CYSTEIN-MODIFIZIERTE ANTIKÖRPERKONJUGATE
CONJUGUÉS D'ANTICORPS MODIFIÉS PAR CYSTÉINE, RADIOMARQUÉS PAR LE ZIRCONIUM

(30) Priority: 05.11.2009 US 612912
(43) Date of publication of application: 12.09.2012
(73) Proprietor: F. Hoffmann-La Roche AG, 4070 Basel (CH)
(72) Inventor: GILL, Herman, South San Francisco, California 94080 (US); JUNUTULA, Jagath R., South San Francisco, California 94080 (US); LOWMAN, Henry B., South San Francisco, California 94080 (US); MARIK, Jan, South San Francisco, California 94080 (US); TINIANOW, Jeff, South San Francisco, California 94080 (US); WILLIAMS, Simon, South San Francisco, California 94080 (US)
(74) Representative: Brodbeck, Michel
(86) International application number: PCT/US2010/055465
(87) International publication number: WO 2011/056983

(56) References cited:
- WO-A2-2006/034488
- WO-A2-2008/141044
- DIJKERS ELI C F ET AL: "Development and characterization of clinical-grade 89Zr-trastuzumab for HER2/neu immunoPET imaging.", JOURNAL OF NUCLEAR MEDICINE : OFFICIAL PUBLICATION, SOCIETY OF NUCLEAR MEDICINE JUN 2009 LNKD- PUBMED:19443585, vol. 50, no. 6, June 2009 (2009-06), pages 974-981, XP002615664, ISSN: 0161-5505
- GOVINDAN S V ET AL: "Deferoxamine as a chelator for <67>Ga in the preparation of antibody conjugates", NUCLEAR MEDICINE AND BIOLOGY, ELSEVIER, NY, US, vol. 32, no. 5, 1 July 2005 (2005-07-01), pages 513-519, XP004952403, ISSN: 0969-8051, DOI: DOI:10.1016/J.NUCMEDBIO.2005.04.009
- TINIANOW JEFF N ET AL: "Site-specifically 89Zr-labeled monoclonal antibodies for ImmunoPET.", NUCLEAR MEDICINE AND BIOLOGY APR 2010 LNKD- PUBMED:20346868, vol. 37, no. 3, April 2010 (2010-04), pages 289-297, XP002615665, ISSN: 1872-9614
- JUNUTULA J R ET AL: "Site-specific conjugation of a cytotoxic drug to an antibody improves the therapeutic index", NATURE BIOTECHNOLOGY, NATURE PUBLISHING GROUP, NEW YORK, NY, US, vol. 26, no. 8, 1 August 2008 (2008-08-01) , pages 925-932, XP002499771, ISSN: 1087-0156, DOI: DOI:10.1038/NBT.1480 [retrieved on 2008-07-20]
- VEREL IRIS ET AL: "89Zr immuno-PET: comprehensive procedures for the production of 89Zr-labeled monoclonal antibodies.", JOURNAL OF NUCLEAR MEDICINE : OFFICIAL PUBLICATION, SOCIETY OF NUCLEAR MEDICINE AUG 2003 LNKD- PUBMED:12902418, vol. 44, no. 8, August 2003 (2003-08), pages 1271-1281, XP002615666, ISSN: 0161-5505
- JUNUTULA ET AL: "Rapid identification of reactive cysteine residues for site-specific labeling of antibody-Fabs", JOURNAL OF IMMUNOLOGICAL METHODS, ELSEVIER SCIENCE PUBLISHERS B.V.,AMSTERDAM, NL, vol. 332, no. 1-2, 14 January 2008 (2008-01-14), pages 41-52, XP022527824, ISSN: 0022-1759, DOI: DOI:10.1016/J.JIM.2007.12.011
- Greg T Hermanson: "Amine reactive and sulfhydryl-reactive crosslinkers; 1.3. SMCC and Sulfo-SMCC" In: "Bioconjugate Techniques 2nd edition", 1 January 2008 (2008-01-01), Academic Press, XP055200919, vol. 8, pages 283-286,
- Greg T Hermanson: "NHS Ester-Maleimide-Mediated Conjugation" In: "Bioconjugate Techniques, 2nd edition", 1 January 2008 (2008-01-01), Academic Press, XP055200932, vol. 8, pages 788-793,
- Greg T Hermanson: "Amine reactive and sulfhydryl-reactive crosslinkers; 1.3. SMCC and Sulfo-SMCC" In: "Bioconjugate Techniques 2nd edition", 1 January 2008 (2008-01-01), Academic Press, XP55200919, vol. 8, pages 283-286,

## Description

### FIELD OF THE INVENTION

The invention relates generally to methods of making zirconium-labelled, cysteine-engineered antibodies comprising a cysteine engineered antibody conjugated through a free cysteine amino acid to a linker and a zirconium complex.

### BACKGROUND OF THE INVENTION

Molecular imaging is an important tool in the development and evaluation of novel pharmaceuticals. Immuno-positron emission tomography (ImmunoPET) is a rapidly emerging method for tracking and quantifying monoclonal antibodies (mAbs) *in vivo* as it efficiently combines the high sensitivity of PET with the high specificity of mAbs. ImmunoPET aspires to be the clinical method of choice for non-invasive diagnosis providing "comprehensive immunohistochemical staining *in vivo"* (van Dongen GA, et al. "Immuno-PET: a navigator in monoclonal antibody development and applications" Oncologist 2007;12:1379-89). Since ImmunoPET requires a positron-emitting radioisotope to be coupled to a target specific molecule it is essential to match the biological half-life of the molecule with the half-life of the radionuclide (Verel I, et al. "The promise of immuno-PET in radioimmunotherapy" J Nucl Med 2005;46 Suppl 1:164S-71S). Although antibodies (-150 kDa) have plasma half-life ranging from days to weeks, imaging typically provides maximum target-to-background ratios 2-6 days after antibody-based tracer administration demanding the use of radioisotopes such as ⁸⁹Zr and ¹²⁴I with half-life of 3.3 days and 4.2 days, respectively. Unfortunately, the half-life of readily available ⁶⁴Cu (12.7 h) is too short to provide images with good contrast in this time frame.

The development of Positron Emission Tomographic (PET) imaging agents from a Mab template (Immuno-PET) holds promise as a tool for localizing and quantifying molecular targets and may enhance the non-invasive clinical diagnosis of pathological conditions (van Dongen et al (2007) Oncologist 12;1379-89; Williams et a (2001) Cancer Biother Radiopharm 16:25-35; Holliger et al (2005) Nat Biotechnol 23:1126-36). PET is a molecular imaging technology that is increasingly used for detection of disease. PET imaging systems create images based on the distribution of positron-emitting isotopes in the tissue of a patient. The isotopes are typically administered to a patient by injection of probe molecules that comprise a positron-emitting isotope, such as F-18, C-11, N-13, or 0-15, covalently attached to a molecule that is readily metabolized or localized in the body (e.g., glucose) or that chemically binds to receptor sites within the body. In some cases, the isotope is administered to the patient as an ionic solution or by inhalation. Small immuno-PET imaging agents, such as Fab antibody fragments (50 kDa) or diabodies, paired dimers of the covalently associated V_{H}-V_{L} region of Mab, 55 kDa (Shively et al (2007) J Nucl Med 48:170-2), may be particularly useful since they exhibit a short circulation half-life, high tissue permeability, and reach an optimal tumor to background ratio between two to four hours after injection facilitating the use of short half-life isotopes such as the widely available ¹⁸F (109.8 min).

Iodine 124 (¹²⁴I) was coupled to antibody 3F9 and used to estimate the dosimetry for radioimmunotherapy of neuroblastoma (Larson SM, et al "PET scanning of iodine-124-3F9 as an approach to tumor dosimetry during treatment planning for radioimmunotherapy in a child with neuroblastoma" J Nucl Med 1992;33:2020-3). Later, as more sophisticated PET instrumentation and improved techniques for radioiodination emerged, ¹²⁴I was employed in numerous immunoPET studies (Verel I, et al "High-quality 1241-labelled monoclonal antibodies for use as PET scouting agents prior to 131I-radioimmunotherapy" European journal of nuclear medicine and molecular imaging 2004;31:1645-52; Lee FT et al "Immuno-PET of human colon xenograft- bearing BALB/c nude mice using 124I-CDR-grafted humanized A33 monoclonal antibody" J Nucl Med 2001;42:764-9; Sundaresan G, et al. "1241-labeled engineered anti-CEA minibodies and diabodies allow high-contrast, antigen-specific small-animal PET imaging of xenografts in athymic mice" J Nucl Med 2003;44:1962-9; Jain M and Batra SK. "Genetically engineered antibody fragments and PET imaging: a new era of radioimmunodiagnosis" J Nucl Med 2003;44:1970-2; Gonzalez Trotter DE et al. "Quantitation of small-animal (124)1 activity distributions using a clinical PET/CT scanner" J Nucl Med 2004;45:1237-44; Robinson MK, et al. "Quantitative immuno-positron emission tomography imaging of HER2-positive tumor xenografts with an iodine-124 labeled anti-HER2 diabody" Cancer Res 2005;65:1471-8; Jayson GC et al. "Molecular imaging and biological evaluation of HuMV833 anti-VEGF antibody: implications for trial design of antiangiogenic antibodies" J Natl Cancer Inst 2002;94:1484-93; Divgi CR, et al. "Preoperative characterisation of clear-cell renal carcinoma using iodine-124-labelled antibody chimeric G250 (124I-cG250) and PET in patients with renal masses: a phase I trial" Lancet Oncol 2007;8:304-10). Despite the relatively simple radioiodination techniques available for coupling ¹²⁴I onto mAbs, important limitations slow a widespread pre-clinical use of this radionuclide. Notably, the complex decay scheme involves energetic positrons (β⁺ max. 1.5 and 2.1 MeV) which negatively affects the resolution of small animal microPET. Additionally, internalized iodinated proteins undergo enzymatic deiodination with free iodide rapidly cleared from the target cells providing PET images not reflective of the actual mAb uptake "Perera RM et al. "Internalization, intracellular trafficking, and biodistribution of monoclonal antibody 806: a novel anti-epidermal growth factor receptor antibody" Neoplasia (New York, N.Y 2007;9:1099-110). The use of ⁸⁹Zr overcomes these drawbacks as the positrons emitted in ⁸⁹Zr decay (β⁺ max. 897 keV) provide microPET resolution comparable to ¹⁸F and ¹¹C (around 1 mm). Also, the metabolites of internalized ⁸⁹Zr-mAbs are intracellularly trapped in lysosomes, providing better correlation of actual mAb uptake with PET imaging (van Dongen GA, et al. "Immuno-PET: a navigator in monoclonal antibody development and applications" Oncologist 2007;12:1379-89).

Conventional means of attaching, i.e. linking through covalent bonds, a label, such as a radioisotope, fluorescent dye, or drug moiety, to an antibody generally leads to a heterogeneous mixture of molecules where the label moieties are attached at a number of sites on the antibody. For example, cytotoxic drugs have typically been conjugated to antibodies through the often-numerous lysine residues of an antibody, generating a heterogeneous antibody-drug conjugate mixture. Depending on reaction conditions, the heterogeneous mixture typically contains a distribution of antibodies with from 0 to about 8, or more, attached drug moieties. In addition, within each subgroup of conjugates with a particular integer ratio of drug moieties to antibody, is a potentially heterogeneous mixture where the drug moiety is attached at various sites on the antibody. Analytical and preparative methods are inadequate to separate and characterize the antibody-drug conjugate species molecules within the heterogeneous mixture resulting from a conjugation reaction. Antibodies are large, complex and structurally diverse biomolecules, often with many reactive functional groups. Their reactivities with linker reagents and drug-linker intermediates are dependent on factors such as pH, concentration, salt concentration, and cosolvents. Furthermore, the multistep conjugation process may be nonreproducible due to difficulties in controlling the reaction conditions and characterizing reactants and intermediates.

Cysteine thiols are reactive at neutral pH, unlike most amines which are protonated and less nucleophilic near pH 7. Since free thiol (RSH, sulfhydryl) groups are relatively reactive, proteins with cysteine residues often exist in their oxidized form as disulfide-linked oligomers or have internally bridged disulfide groups. Extracellular proteins generally do not have free thiols (Garman, 1997, Non-Radioactive Labelling: A Practical Approach, Academic Press, London , at page 55). The amount of free thiol in a protein may be estimated by the standard Ellman's assay. Immunoglobulin M is an example of a disulfide-linked pentamer, while immunoglobulin G is an example of a protein with internal disulfide bridges bonding the subunits together. In proteins such as this, reduction of the disulfide bonds with a reagent such as dithiothreitol (DTT) or selenol (Singh et al (2002) Anal. Biochem. 304:147-156) is required to generate the reactive free thiol. This approach may result in loss of antibody tertiary structure and antigen binding specificity.

Antibody cysteine thiol groups are generally more reactive, i.e. more nucleophilic, towards electrophilic conjugation reagents than antibody amine or hydroxyl groups. Cysteine residues have been introduced into proteins by genetic engineering techniques to form covalent attachments to ligands or to form new intramolecular disulfide bonds (Better et al (1994) J. Biol. Chem. 269(13):9644-9650; Bernhard et al (1994) Bioconjugate Chem. 5:126-132; Greenwood et al (1994) Therapeutic Immunology 1:247-255; Tu et al (1999) Proc. Natl. Acad. Sci USA 96:4862-4867; Kanno et al (2000) J. of Biotechnology, 76:207-214; Chmura et al (2001) Proc. Nat. Acad. Sci. USA 98(15):8480-8484; US 6248564). However, designing in cysteine thiol groups by the mutation of various amino acid residues of a protein to cysteine amino acids is potentially problematic, particularly in the case of unpaired (free Cys) residues or those which are relatively accessible for reaction or oxidation. In concentrated solutions of the protein, whether in the periplasm of E. coli, culture supernatants, or partially or completely purified protein, unpaired Cys residues on the surface of the protein can pair and oxidize to form intermolecular disulfides, and hence protein dimers or multimers. Disulfide dimer formation renders the new Cys unreactive for conjugation to a drug, ligand, or other label. Furthermore, if the protein oxidatively forms an intramolecular disulfide bond between the newly engineered Cys and an existing Cys residue, both Cys groups are unavailable for active site participation and interactions. Furthermore, the protein may be rendered inactive or non-specific, by misfolding or loss of tertiary structure (Zhang et al (2002) Anal. Biochem. 311:1-9).

Site-specific conjugation is preferred over random amino modification as it enables chemical modification of a site away from the binding site, promoting complete retention of biological activity and allowing control over the possible number of prosthetic groups added. Cysteine-engineered antibodies have been designed as FAB antibody fragments (thioFab) and expressed as full-length, IgG monoclonal (thioMab) antibodies. See: US 7521541; Junutula JR et al. "Rapid identification of reactive cysteine residues for site-specific labeling of antibody-Fabs" J Immunol Methods 2008;332:41-52; Junutula JR et al. "Site-specific conjugation of a cytotoxic drug to an antibody improves the therapeutic index" (2008) Nat Biotechnol. 26:925-32. ThioFab and ThioMab antibodies have been conjugated through linkers at the newly introduced cysteine thiols with thiol-reactive linker reagents and drug-linker reagents to prepare cysteine-engineered antibody drug conjugates (Thio ADC) with anti-cancer properties, including anti-MUC16 (US 2008/0311134), anti-CD22 (US 2008/0050310), anti-ROBO4 (US 2008/0247951), anti-TENB2 (US 2009/0117100), anti-CD79B (US 2009/0028856; US 2009/0068202) Thio ADC.

Dijkers EC et al. "Development and Characterization of Clinical-Grade 89Zr-Trastuzumab for HER2/neu ImmunoPET Imaging" (2009) J Nucl Med 50(6):974-981 discloses ⁸⁹Zr-trastuzumab for PET imaging. In D1, trastuzumab is linked to a chelating linker, TFP-N-SucDf-Fe, to chelate ⁸⁹Zr. TFP-N-SucDf-Fe is conjugated with lysine ε-amino groups of antibody.

WO2006/034488 discloses a cysteine engineered antibody with a free cysteine amino acid which can be used for site-specifically labelling an antibody with radionuclides.

### SUMMARY

The compounds of the disclosure include cysteine engineered antibodies where one or more amino acids of a parent antibody are replaced with a free cysteine amino acid. A cysteine engineered antibody comprises one or more free cysteine amino acids having a thiol reactivity value in the range of 0.6 to 1.0. A free cysteine amino acid is a cysteine residue which has been engineered into the parent antibody and is not part of a disulfide bridge.

Cysteine engineered antibodies may be useful in the diagnosis and treatment of cancer and include antibodies specific for cell surface and transmembrane receptors, and tumor-associated antigens (TAA). Such antibodies may be used as naked antibodies (unconjugated to a drug or label moiety) or as antibody-zirconium conjugates (AZC).

Embodiments of the methods for preparing and screening a cysteine engineered antibody include where the parent antibody is an antibody fragment, such as hu4D5Fabv8. The parent antibody may also be a fusion protein comprising an albumin-binding peptide sequence (ABP). The parent antibody may also be a humanized antibody selected from huMAb4D5-1, huMAb4D5-2, huMAb4D5-3, huMAb4D5-4, huMAb4D5-5, huMAb4D5-6, huMAb4D5-7 and huMAb4D5-8 (trastuzumab).

An aspect of the invention is a method of making a zirconium-labelled, cysteine-engineered antibody comprising a cysteine engineered antibody (Ab) conjugated through a free cysteine amino acid to a linker (L) and a zirconium complex (Z), having Formula I:

Ab-(L-Z)ₚ I

where p is 1 to 2;
the method comprising a first method of making a desferrioxamine-labelled, cysteine-engineered antibody comprising a cysteine engineered antibody (Ab) conjugated through a free cysteine amino acid to a linker (L) and a desferrioxamine moiety (Df), having Formula II:

Ab-(L-Df)ₚ II

wherein L-Df is selected from: 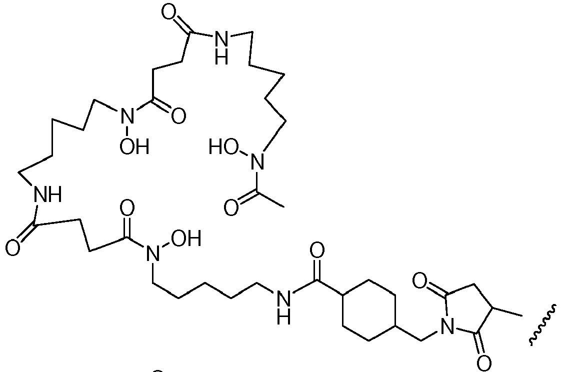 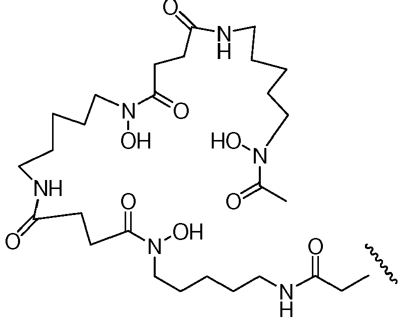 where the wavy line indicates the attachment to the antibody (Ab); and
p is 1 to 2;
the first method comprising reacting a composition selected from the structures: 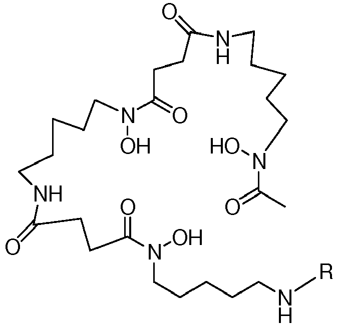 wherein R is selected from: 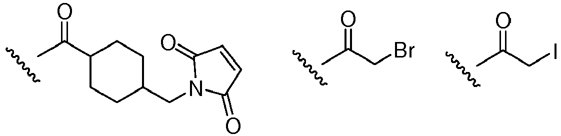 with a cysteine-engineered antibody having one or more free cysteine amino acids,
whereby the desferrioxamine-labelled, cysteine-engineered antibody is formed;
the method further comprising complexing a zirconium reagent with a desferrioxamine-labelled, cysteine-engineered antibody comprising a cysteine engineered antibody (Ab) conjugated through a free cysteine amino acid to a linker (L) and a desferrioxamine moiety (Df), having Formula II:

Ab-(L-Df)ₚ II

wherein L-Df is selected from: 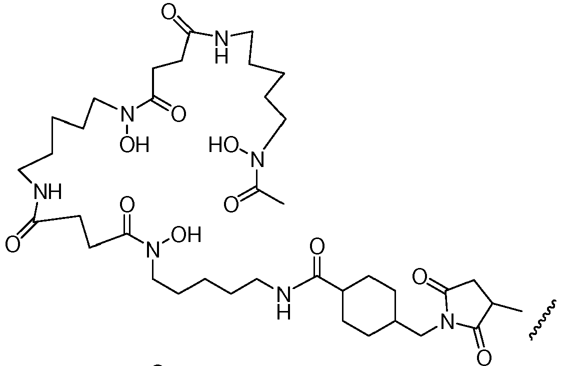 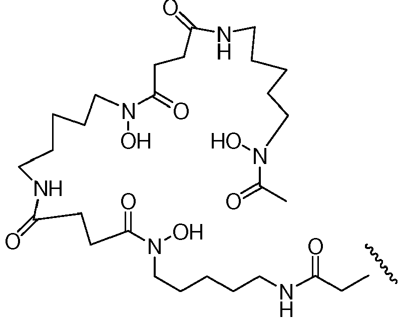 where the wavy line indicates the attachment to the antibody (Ab); and
p is 1 to 2;
whereby the zirconium-labelled, cysteine-engineered antibody is formed;
wherein the cysteine engineered antibody is prepared by a process comprising replacing one or more amino acid residues of a parent antibody with the one or more free cysteine amino acids, where the parent antibody selectively binds to an antigen and the cysteine engineered antibody selectively binds to the same antigen as the parent antibody,
wherein the cysteine engineered antibody comprises a sequence in the heavy chain selected from SEQ ID NO: 13:
LVTVSSCSTKGPS SEQ ID NO:13
where the cysteine in SEQ ID NO: 13 is the free cysteine amino acid.

### BRIEF DESCRIPTION OF THE DRAWINGS

- Figure 1A: shows a three-dimensional representation of the hu4D5Fabv7 antibody fragment derived by X-ray crystal coordinates. The structure positions of the exemplary engineered Cys residues of the heavy and light chains are numbered (according to a sequential numbering system).
- Figure 1B: shows a sequential numbering scheme (top row), starting at the N-terminus in comparison with the Kabat numbering scheme (bottom row) for 4D5v7fabH. Kabat numbering insertions are noted by a,b,c.
- Figures 2A and 2B: show binding measurements with detection of absorbance at 450nm of hu4D5Fabv8 and hu4D5Fabv8 Cys mutant (ThioFab) phage variants: (A) non-biotinylated phage-hu4D5Fabv8 and (B) biotinylated phage-hu4D5Fabv8 (B) by the PHESELECTOR assay for interactions with BSA (open bar), HER2 (striped bar) or streptavidin (solid bar).
- Figures 3A and 3B: show binding measurements with detection of absorbance at 450nm of hu4D5Fabv8 (left) and hu4D5Fabv8 Cys mutant (ThioFab) variants: (A) non-biotinylated phage-hu4D5Fabv8 and (B) biotinylated phage-hu4D5Fabv8 by the PHESELECTOR assay for interactions with: BSA (open bar), HER2 (striped bar) and streptavidin (solid bar). Light chain variants are on the left side and heavy chain variants are on the right side. Thiol reactivity = OD_{450 nm} for streptavidin binding ÷ OD_{450 nm} for HER2 (antibody) binding
- Figure 4A: shows Fractional Surface Accessibility values of residues on wild type hu4D5Fabv8. Light chain sites are on the left side and heavy chain sites are on the right side.
- Figure 4B: shows binding measurements with detection of absorbance at 450nm of biotinylated hu4D5Fabv8 (left) and hu4D5Fabv8 Cys mutant (ThioFab) variants for interactions with HER2 (day 2), streptavidin (SA) (day 2), HER2 (day 4), and SA (day 4). Phage-hu4D5Fabv8 Cys variants were isolated and stored at 4 °C. Biotin conjugation was carried out either at day 2 or day 4 followed by PHESELECTOR analyses to monitor their interaction with Her2 and streptavidin as described in Example 2, and probe the stability of reactive thiol groups on engineered ThioFab variants.
- Figure 5: shows binding measurements with detection of absorbance at 450nm of biotin-maleimide conjugated-hu4D5Fabv8 (A121C) and non-biotinylated wild type hu4D5Fabv8 for binding to streptavidin and HER2. Each Fab was tested at 2 ng and 20 ng.
- Figure 6: shows ELISA analysis with detection of absorbance at 450nm of biotinylated ABP-hu4D5Fabv8 wild type (wt), and ABP-hu4D5Fabv8 cysteine mutants V110C and A121C for binding with rabbit albumin, streptavidin (SA), and HER2.
- Figure 7: shows ELISA analysis with detection of absorbance at 450nm of biotinylated ABP-hu4D5Fabv8 cysteine mutants (ThioFab variants): (left to right) single Cys variants ABP-V110C, ABP-A121C, and double Cys variants ABP-V110C-A88C and ABP-V110C-A121C for binding with rabbit albumin, HER2 and streptavidin (SA), and probing with Fab-HRP or SA-HRP.
- Figure 8: shows binding of biotinylated ThioFab phage and an anti-phage HRP antibody to HER2 (top) and Streptavidin (bottom).
- Figure 13A: shows a cartoon depiction of biotinylated antibody binding to immobilized HER2 with binding of HRP labeled secondary antibody for absorbance detection.
- Figure 13B: shows binding measurements with detection of absorbance at 450nm of biotin-maleimide conjugated thio-trastuzumab variants and non-biotinylated wild type trastuzumab in binding to immobilized HER2. From left to right: V110C (single cys), A121C (single cys), V110C/A121C (double cys), and trastuzumab. Each thio IgG variant and trastuzumab was tested at 1, 10, and 100 ng.
- Figure 14A: shows a cartoon depiction of biotinylated antibody binding to immobilized HER2 with binding of biotin to anti-IgG-HRP for absorbance detection.
- Figure 14B: shows binding measurements with detection of absorbance at 450nm of biotin-maleimide conjugated-thio trastuzumab variants and non-biotinylated wild type trastuzumab in binding to immobilized streptavidin. From left to right: V110C (single cys), A121C (single cys), V110C/A121C (double cys), and trastuzumab. Each thio IgG variant and trastuzumab was tested at 1, 10, and 100 ng.
- Figure 15: shows the general process to prepare a cysteine engineered antibody (ThioMab) expressed from cell culture for conjugation.
- Figure 16: shows non-reducing (top) and reducing (bottom) denaturing polyacrylamide gel electrophoresis analysis of 2H9 ThioMab Fc variants (left to right, lanes 1-9): A339C; S337C; S324C; A287C; V284C; V282C; V279C; V273C, and 2H9 wild type after purification on immobilized Protein A. The lane on the right is a size marker ladder, indicating the intact proteins are about 150 kDa, heavy chain fragments about 50 kDa, and light chain fragments about 25 kDa.
- Figure 17A: shows non-reducing (left) and reducing (+DTT) (right) denaturing polyacrylamide gel electrophoresis analysis of 2H9 ThioMab variants (left to right, lanes 1-4): L-V15C; S179C; S375C; S400C, after purification on immobilized Protein A.
- Figure 17B: shows non-reducing (left) and reducing (+DTT) (right) denaturing polyacrylamide gel electrophoresis analysis of 2H9 and 3A5 ThioMab variants after purification on immobilized Protein A.
- Figure 18: shows western blot analysis of biotinylated Thio-IgG variants. 2H9 and 3A5 ThioMab variants were analyzed on reduced denaturing polyacrylamide gel electrophoresis, the proteins were transferred to nitrocellulose membrane. The presence of antibody and conjugated biotin were probed with anti-IgG-HRP (top) and streptavidin-HRP (bottom), respectively. Lane 1: 3A5 H-A121C. Lane 2: 3A5 L-V110C. Lane 3: 2H9 H-A121C. Lane 4: 2H9 L-V110C. Lane 5: 2H9 wild type.
- Figure 19: shows ELISA analysis for the binding of biotinylated 2H9 variants to streptavidin by probing with anti-IgG-HRP and measuring the absorbance at 450 nm of (top bar diagram). Bottom schematic diagram depicts the experimental design used in the ELISA analysis.
- Figure 20: shows bifunctional reagents for coupling chelator of ⁸⁹Zr desferrioxamine B (Df, top) with proteins using amino reactive linkers, TFP-N-SucDf and Df-Bz-NCS (center) and thiol reactive linkers, Df-Chx-Mal, Df-Bac, and Df-lac (bottom).
- Figure 21: shows the preparation of Df-Chx-Mal, Df-Bac, Df-Iac and conjugation to thiotrastuzumab via Cys residues incorporated into the heavy chain of Fab. Reaction conditions: i. DIEA, DMF/H₂O (10:1), RT, 0.5-1 h; ii. DIEA, DMF, 0°C, 4 h; iii. pH 7.5, RT, 1 h; iv. pH 9, RT, 5 h; v. pH 9, RT, 2 h.
- Figure 22: shows chelation of zirconium-89 oxalate with a desferrioxamine-labelled, cysteine-engineered antibody, such as variants of Df-linker-trastuzumab containing four linkers: N-Suc, Bz-SCN, Chx-maleimide (CHx-Mal), or acetyl (Ac).
- Figure 23: shows mass spectrometry analysis of reduced antibodies showing separate signals from light and heavy chains. A: thio-trasuzumab, B: Df-Ac-thio-trastuzumab (using Df-Bac), and C: Df-Ac-thio-trastuzumab (using Df-Iac), and D: Df-Chx-Mal-thiotratsuzumab.
- Figure 24: shows stability of ⁸⁹Zr-Chx-Mal-thio-trastuzumab (open circle) and ⁸⁹Zr-Df-Acthio-trastuzumab (full circle) in mouse serum at 37 °C (n = 3).
- Figure 25: shows representative full-body images (maximum intensity projection) acquired 96 hours after the tail vein bolus injection of 100 µCi of ⁸⁹Zr-Trasuzumab prepared using four different linkers (Bz-SCN, N-Suc, Chx-Mal, and Ac).
- Figure 26: shows *In vivo* uptake in selected tissues at 24, 96 and 144 h post injection as measured by PET.

### DETAILED DESCRIPTION OF EXEMPLARY EMBODIMENTS

Reference will now be made in detail to certain embodiments of the invention, examples of which are illustrated in the accompanying structures and formulas. While the invention will be described in conjunction with the enumerated embodiments, it will be understood that they are not intended to limit the invention to those embodiments. On the contrary, the invention is intended to cover all alternatives, modifications, and equivalents, which may be included within the scope of the present invention as defined by the claims.

One skilled in the art will recognize many methods and materials similar or equivalent to those described herein, which could be used in the practice of the present invention. The present invention is in no way limited to the methods and materials described.

Unless defined otherwise, technical and scientific terms used herein have the same meaning as commonly understood by one of ordinary skill in the art to which this invention belongs, and are consistent with: Singleton et al (1994) Dictionary of Microbiology and Molecular Biology, 2nd Ed., J. Wiley & Sons, New York, NY; and Janeway, C., Travers, P., Walport, M., Shlomchik (2001) Immunobiology, 5th Ed., Garland Publishing, New York.

### DEFINITIONS

Unless stated otherwise, the following terms and phrases as used herein are intended to have the following meanings:
When trade names are used herein, applicants intend to independently include the trade name product formulation, the generic drug, and the active pharmaceutical ingredient(s) of the trade name product.

The term "antibody" herein is used in the broadest sense and specifically covers monoclonal antibodies, polyclonal antibodies, dimers, multimers, multispecific antibodies (e.g., bispecific antibodies), and antibody fragments, so long as they exhibit the desired biological activity (Miller et al (2003) Jour. of Immunology 170:4854-4861). Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce autoimmune antibodies associated with an autoimmune disease. The immunoglobulin disclosed herein can be of any type (e.g., IgG, IgE, IgM, IgD, and IgA), class (e.g., IgG1, IgG2, IgG3, IgG4, IgA1 and IgA2) or subclass of immunoglobulin molecule. The immunoglobulins can be derived from any species. In one aspect, however, the immunoglobulin is of human, murine, or rabbit origin.

"Antibody fragments" comprise a portion of a full length antibody, generally the antigen binding or variable region thereof. Examples of antibody fragments include Fab, Fab', F(ab')₂, and Fv fragments; diabodies; linear antibodies; minibodies (Olafsen et al (2004) Protein Eng. Design & Sel. 17(4):315-323), fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al (1975) Nature 256:495, or may be made by recombinant DNA methods (see for example: US 4816567; US 5807715). The monoclonal antibodies may also be isolated from phage antibody libraries using the techniques described in Clackson et al (1991) Nature, 352:624-628; Marks et al (1991) J. Mol. Biol., 222:581-597; for example.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4816567; and Morrison et al (1984) Proc. Natl. Acad. Sci. USA, 81:6851-6855). Chimeric antibodies of interest herein include "primatized" antibodies comprising variable domain antigen-binding sequences derived from a non-human primate (*e.g.,* Old World Monkey, Ape etc) and human constant region sequences.

An "intact antibody" herein is one comprising a VL and VH domains, as well as a light chain constant domain (CL) and heavy chain constant domains, CH1, CH2 and CH3. The constant domains may be native sequence constant domains (e.g., human native sequence constant domains) or amino acid sequence variant thereof. The intact antibody may have one or more "effector functions" which refer to those biological activities attributable to the Fc constant region (a native sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; and down regulation of cell surface receptors such as B cell receptor and BCR.

Depending on the amino acid sequence of the constant domain of their heavy chains, intact antibodies can be assigned to different "classes." There are five major classes of intact immunoglobulin antibodies: IgA, IgD, IgE, IgG, and IgM, and several of these may be further divided into "subclasses" (isotypes), *e.g.,* IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known. Ig forms include hinge-modifications or hingeless forms (Roux et al (1998) J. Immunol. 161:4083-4090; Lund et al (2000) Eur. J. Biochem. 267:7246-7256; US 2005/0048572; US 2004/0229310).

An "ErbB receptor" is a receptor protein tyrosine kinase which belongs to the ErbB receptor family whose members are important mediators of cell growth, differentiation and survival. The ErbB receptor family includes four distinct members including epidermal growth factor receptor (EGFR, ErbB1, HER1), HER2 (ErbB2 or p185neu), HER3 (ErbB3) and HER4 (ErbB4 or tyro2). A panel of anti-ErbB2 antibodies has been characterized using the human breast tumor cell line SKBR3 (Hudziak et al (1989) Mol. Cell. Biol. 9(3): 1165-1172. Maximum inhibition was obtained with the antibody called 4D5 which inhibited cellular proliferation by 56%. Other antibodies in the panel reduced cellular proliferation to a lesser extent in this assay. The antibody 4D5 was further found to sensitize ErbB2-overexpressing breast tumor cell lines to the cytotoxic effects of TNF-α (US 5677171). The anti-ErbB2 antibodies discussed in Hudziak et al. are further characterized in Fendly et al (1990) Cancer Research 50:1550-1558; Kotts et al. (1990) In Vitro 26(3):59A; Sarup et al. (1991) Growth Regulation 1:72-82; Shepard et al. J. (1991) Clin. Immunol. 11(3):117-127; Kumar et al. (1991) Mol. Cell. Biol. 11(2):979-986; Lewis et al. (1993) Cancer Immunol. Immunother. 37:255-263; Pietras et al. (1994) Oncogene 9:1829-1838; Vitetta et al. (1994) Cancer Research 54:5301-5309; Sliwkowski et al. (1994) J. Biol. Chem. 269(20):14661-14665; Scott et al. (1991) J. Biol. Chem. 266:14300-5; D'souza et al. Proc. Natl. Acad. Sci. (1994) 91:7202-7206; Lewis et al. (1996) Cancer Research 56:1457-1465; and Schaefer et al. (1997) Oncogene 15:1385-1394.

The ErbB receptor will generally comprise an extracellular domain, which may bind an ErbB ligand; a lipophilic transmembrane domain; a conserved intracellular tyrosine kinase domain; and a carboxyl-terminal signaling domain harboring several tyrosine residues which can be phosphorylated. The ErbB receptor may be a "native sequence" ErbB receptor or an "amino acid sequence variant" thereof. Preferably, the ErbB receptor is native sequence human ErbB receptor. Accordingly, a "member of the ErbB receptor family" is EGFR (ErbB1), ErbB2, ErbB3, ErbB4 or any other ErbB receptor currently known or to be identified in the future.

The terms "ErbB1", "epidermal growth factor receptor", "EGFR" and "HER1" are used interchangeably herein and refer to EGFR as disclosed, for example, in Carpenter et al (1987) Ann. Rev. Biochem., 56:881-914, including naturally occurring mutant forms thereof *(e.g.,* a deletion mutant EGFR as in Humphrey et al (1990) Proc. Nat. Acad. Sci. (USA) 87:4207-4211). The term erbB1 refers to the gene encoding the EGFR protein product. Antibodies against HER1 are described, for example, in Murthy et al (1987) Arch. Biochem. Biophys., 252:549-560 and in WO 95/25167.

The term "ERRP", "EGF-Receptor Related Protein", "EGFR Related Protein" and "epidermal growth factor receptor related protein" are used interchangeably herein and refer to ERRP as disclosed, for example in US 6399743 and US Publication No. 2003/0096373.

The expressions "ErbB2" and "HER2" are used interchangeably herein and refer to human HER2 protein described, for example, in Semba et al (1985) Proc. Nat. Acad. Sci. (USA) 82:6497-6501 and Yamamoto et al (1986) Nature, 319:230-234 (Genebank accession number X03363). The term "erbB2" refers to the gene encoding human ErbB2 and "neu" refers to the gene encoding rat p185neu. Preferred ErbB2 is native sequence human ErbB2.

"ErbB3" and "HER3" refer to the receptor polypeptide as disclosed, for example, in U.S. Patent Nos. 5183884 and 5480968 as well as Kraus et al (1989) Proc. Nat. Acad. Sci. (USA) 86:9193-9197. Antibodies against ErbB3 are known in the art and are described, for example, in U.S. Patent Nos. 5183884, 5480968 and in WO 97/35885.

The terms "ErbB4" and "HER4" herein refer to the receptor polypeptide as disclosed, for example, in EP Pat Application No 599,274; Plowman et al (1993) Proc. Natl. Acad. Sci. USA 90:1746-1750; and Plowman et al (1993) Nature 366:473-475, including isoforms thereof, e.g., as disclosed in WO 99/19488. Antibodies against HER4 are described, for example, in WO 02/18444.

Antibodies to ErbB receptors are available commercially from a number of sources, including, for example, Santa Cruz Biotechnology, Inc., California, USA.

The term "amino acid sequence variant" refers to polypeptides having amino acid sequences that differ to some extent from a native sequence polypeptide. Ordinarily, amino acid sequence variants will possess at least about 70% sequence identity with at least one receptor binding domain of a native ErbB ligand or with at least one ligand binding domain of a native ErbB receptor, and preferably, they will be at least about 80%, more preferably, at least about 90% homologous by sequence with such receptor or ligand binding domains. The amino acid sequence variants possess substitutions, deletions, and/or insertions at certain positions within the amino acid sequence of the native amino acid sequence. Amino acids are designated by the conventional names, one-letter and three-letter codes.

"Sequence identity" is defined as the percentage of residues in the amino acid sequence variant that are identical after aligning the sequences and introducing gaps, if necessary, to achieve the maximum percent sequence identity. Methods and computer programs for the alignment are well known in the art. One such computer program is "Align 2," authored by Genentech, Inc., which was filed with user documentation in the United States Copyright Office, Washington, DC 20559, on December 10, 1991.

"Antibody-dependent cell-mediated cytotoxicity" and "ADCC" refer to a cell-mediated reaction in which nonspecific cytotoxic cells that express Fc receptors (FcRs) (e.g., Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcyRI, FcyRII and FcγRIII. FcR expression on hematopoietic cells in summarized is Table 3 on page 464 of Ravetch and Kinet, (1991) "Annu. Rev. Immunol." 9:457-92. To assess ADCC activity of a molecule of interest, an *in vitro* ADCC assay, such as that described in US 5500362 and US 5821337 may be performed. Useful effector cells for such assays include peripheral blood mononuclear cells (PBMC) and Natural Killer (NK) cells. Alternatively, or additionally, ADCC activity of the molecule of interest may be assessed *in vivo,* e.g., in a animal model such as that disclosed in Clynes et al (1998) PROC. NAT. ACAD. SCI. (USA) (USA) 95:652-656.

"Human effector cells" are leukocytes which express one or more constant region receptors (FcRs) and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source thereof, e.g., from blood or PBMCs as described herein.

The terms "Fc receptor" or "FcR" are used to describe a receptor that binds to the Fc constant region of an antibody. The preferred FcR is a native sequence human FcR. Moreover, a preferred FcR is one which binds an IgG antibody (a gamma receptor) and includes receptors of the FcyRI, FcyRII, and Fcγ RIII subclasses, including allelic variants and alternatively spliced forms of these receptors. FcyRII receptors include FcyRIIA (an "activating receptor") and FcyRIIB (an "inhibiting receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibiting receptor FcyRIIB contains an immunoreceptor tyrosine-based inhibition motif (ITIM) in its cytoplasmic domain. (See review M. in Daëron, "Annu. Rev. Immunol." 15:203-234 (1997)). FcRs are reviewed in Ravetch and Kinet, "Annu. Rev. Immunol"., 9:457-92 (1991); Capel et al (1994) Immunomethods 4:25-34; and de Haas et al (1995) J. Lab. Clin. Med. 126:330-41. Other FcRs, including those to be identified in the future, are encompassed by the term "FcR" herein. The term also includes the neonatal receptor, FcRn, which is responsible for the transfer of maternal IgGs to the fetus (Guyer et al (1976) J. Immunol., 117:587 and Kim et al (1994) J. Immunol. 24:249).

"Complement dependent cytotoxicity" or "CDC" refers to the ability of a molecule to lyse a target in the presence of complement. The complement activation pathway is initiated by the binding of the first component of the complement system (C1q) to a molecule *(e.g.,* an antibody) complexed with a cognate antigen. To assess complement activation, a CDC assay, *e.g.,* as described in Gazzano-Santoro et al J. Immunol. Methods, 202:163 (1996), may be performed.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end. The constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al (1991) Sequences of Proteins of Immunological Interest, 5th Ed. Public Health Service, National Institutes of Health, Bethesda, MD). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody which are responsible for antigen-binding. The hypervariable region generally comprises amino acid residues from a "complementarity determining region" or "CDR" (*e.g.,* residues 24-34 (L1), 50-56 (L2) and 89-97 (L3) in the light chain variable domain and 31-35 (H1), 50-65 (H2) and 95-102 (H3) in the heavy chain variable domain; Kabat et al *supra*) and/or those residues from a "hypervariable loop" (*e.g.,* residues 26-32 (L1), 50-52 (L2) and 91-96 (L3) in the light chain variable domain and 26-32 (H1), 53-55 (H2) and 96-101 (H3) in the heavy chain variable domain; Chothia and Lesk (1987) J. Mol. Biol., 196:901-917). "Framework Region" or "FR" residues are those variable domain residues other than the hypervariable region residues as herein defined.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')2 antibody fragments originally were produced as pairs of Fab' fragments which have hinge cysteines between them. Other chemical couplings of antibody fragments are also known.

The "light chains" of antibodies from any vertebrate species can be assigned to one of two clearly distinct types, called kappa (κ) and lambda (λ), based on the amino acid sequences of their constant domains.

"Single-chain Fv" or "scFv" antibody fragments comprise the V_{H} and V_{L} domains of antibody, wherein these domains are present in a single polypeptide chain. Preferably, the Fv polypeptide further comprises a polypeptide linker between the VH and VL domains which enables the scFv to form the desired structure for antigen binding. For a review of scFv, see Plückthun in The Pharmacology of Monoclonal Antibodies, vol. 113, Rosenburg and Moore eds., Springer-Verlag, New York, pp. 269-315 (1994). Anti-ErbB2 antibody scFv fragments are described in WO 93/16185; US Patent Nos. 5571894; and 5587458.

The term "diabodies" refers to small antibody fragments with two antigen-binding sites, which fragments comprise a variable heavy domain (VH) connected to a variable light domain (VL) in the same polypeptide chain (VH - VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al (1993) Proc. Natl. Acad. Sci. USA 90:6444-6448.

"Humanized" forms of non-human (e.g., rodent) antibodies are chimeric antibodies that contain minimal sequence derived from non-human immunoglobulin. Humanization is a method to transfer the murine antigen binding information to a non-immunogenic human antibody acceptor, and has resulted in many therapeutically useful drugs. The method of humanization generally begins by transferring all six murine complementarity determining regions (CDRs) onto a human antibody framework (Jones et al, (1986) Nature 321:522-525). These CDR-grafted antibodies generally do not retain their original affinity for antigen binding, and in fact, affinity is often severely impaired. Besides the CDRs, select non-human antibody framework residues must also be incorporated to maintain proper CDR conformation (Chothia et al (1989) Nature 342:877). The transfer of key mouse framework residues to the human acceptor in order to support the structural conformation of the grafted CDRs has been shown to restore antigen binding and affinity (Riechmann et al (1992) J. Mol. Biol. 224, 487-499; Foote and Winter, (1992) J. Mol. Biol. 224:487-499; Presta et al (1993) J. Immunol. 151, 2623-2632; Werther et al (1996) J. Immunol. Methods 157:4986-4995; and Presta et al (2001) Thromb. Haemost. 85:379-389). For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see US 6407213; Jones et al (1986) Nature, 321:522-525; Riechmann et al (1988) Nature 332:323-329; and Presta, (1992) Curr. Op. Struct. Biol., 2:593-596.

A "free cysteine amino acid" refers to a cysteine amino acid residue which has been engineered into a parent antibody, has a thiol functional group (-SH), and is not paired as an intramolecular or intermolecular disulfide bridge.

The term "thiol reactivity value" is a quantitative characterization of the reactivity of free cysteine amino acids. The thiol reactivity value is the percentage of a free cysteine amino acid in a cysteine engineered antibody which reacts with a thiol-reactive reagent, and converted to a maximum value of 1. For example, a free cysteine amino acid on a cysteine engineered antibody which reacts in 100% yield with a thiol-reactive reagent, such as a biotin-maleimide reagent, to form a biotin-labelled antibody has a thiol reactivity value of 1.0. Another cysteine amino acid engineered into the same or different parent antibody which reacts in 80% yield with a thiol-reactive reagent has a thiol reactivity value of 0.8. Another cysteine amino acid engineered into the same or different parent antibody which fails totally to react with a thiol-reactive reagent has a thiol reactivity value of 0. Determination of the thiol reactivity value of a particular cysteine may be conducted by ELISA assay, mass spectroscopy, liquid chromatography, autoradiography, or other quantitative analytical tests.

A "parent antibody" is an antibody comprising an amino acid sequence from which one or more amino acid residues are replaced by one or more cysteine residues. The parent antibody may comprise a native or wild type sequence. The parent antibody may have preexisting amino acid sequence modifications (such as additions, deletions and/or substitutions) relative to other native, wild type, or modified forms of an antibody. A parent antibody may be directed against a target antigen of interest, e.g. a biologically important polypeptide. Antibodies directed against nonpolypeptide antigens (such as tumor-associated glycolipid antigens; see US 5091178) are also contemplated.

Exemplary parent antibodies include antibodies having affinity and selectivity for cell surface and transmembrane receptors and tumor-associated antigens (TAA).

Other exemplary parent antibodies include those selected from, and without limitation, anti-estrogen receptor antibody, anti-progesterone receptor antibody, anti-p53 antibody, anti-HER-2/neu antibody, anti-EGFR antibody, anti-cathepsin D antibody, anti-Bcl-2 antibody, anti-E-cadherin antibody, anti-CA125 antibody, anti-CA15-3 antibody, anti-CA19-9 antibody, anti-c-erbB-2 antibody, anti-P-glycoprotein antibody, anti-CEA antibody, anti-retinoblastoma protein antibody, anti-ras oncoprotein antibody, anti-Lewis X antibody, anti-Ki-67 antibody, anti-PCNA antibody, anti-CD3 antibody, anti-CD4 antibody, anti-CD5 antibody, anti-CD7 antibody, anti-CD8 antibody, anti-CD9/p24 antibody, anti-CD10 antibody, anti-CD11c antibody, anti-CD13 antibody, anti-CD14 antibody, anti-CD15 antibody, anti-CD19 antibody, anti-CD20 antibody, anti-CD22 antibody, anti-CD23 antibody, anti-CD30 antibody, anti-CD31 antibody, anti-CD33 antibody, anti-CD34 antibody, anti-CD35 antibody, anti-CD38 antibody, anti-CD41 antibody, anti-LCA/CD45 antibody, anti-CD45RO antibody, anti-CD45RA antibody, anti-CD39 antibody, anti-CD100 antibody, anti-CD95/Fas antibody, anti-CD99 antibody, anti-CD106 antibody, anti-ubiquitin antibody, anti-CD71 antibody, anti-c-myc antibody, anti-cytokeratins antibody, anti-vimentins antibody, anti-HPV proteins antibody, anti-kappa light chains antibody, anti-lambda light chains antibody, anti-melanosomes antibody, anti-prostate specific antigen antibody, anti-S-100 antibody, anti-tau antigen antibody, anti-fibrin antibody, anti-keratins antibody and anti-Tn-antigen antibody.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody "which binds" a molecular target or an antigen of interest, e.g., ErbB2 antigen, is one capable of binding that antigen with sufficient affinity such that the antibody is useful in targeting a cell expressing the antigen. Where the antibody is one which binds ErbB2, it will usually preferentially bind ErbB2 as opposed to other ErbB receptors, and may be one which does not significantly cross-react with other proteins such as EGFR, ErbB3 or ErbB4. In such embodiments, the extent of binding of the antibody to these non-ErbB2 proteins *(e.g.,* cell surface binding to endogenous receptor) will be less than 10% as determined by fluorescence activated cell sorting (FACS) analysis or radioimmunoprecipitation (RIA). Sometimes, the anti-ErbB2 antibody will not significantly cross-react with the rat neu protein, *e.g.,* as described in Schecter et al. (1984) Nature 312:513 and Drebin et al (1984) Nature 312:545-548.

Molecular targets for antibodies encompassed by the present disclosure include CD proteins and their ligands, such as, but not limited to: (i) CD3, CD4, CD8, CD19, CD20, CD22, CD34, CD40, CD79α, (CD79a), and CD79β (CD79b); (ii) members of the ErbB receptor family such as the EGF receptor, HER2, HER3 or HER4 receptor; (iii) cell adhesion molecules such as LFA-1, Mac1, p150,95, VLA-4, ICAM-1, VCAM and ∀v/∃3 integrin, including either alpha or beta subunits thereof (*e*.*g*. anti-CD11a, anti-CD18 or anti-CD11b antibodies); (iv) growth factors such as VEGF; IgE; blood group antigens; flk2/flt3 receptor; obesity (OB) receptor; *mpl* receptor; CTLA-4; protein C, BR3, c-met, tissue factor, ∃7 etc; and (v) cell surface and transmembrane tumor-associated antigens (TAA).

Unless indicated otherwise, the term "monoclonal antibody 4D5" refers to an antibody that has antigen binding residues of, or derived from, the murine 4D5 antibody (ATCC CRL 10463). For example, the monoclonal antibody 4D5 may be murine monoclonal antibody 4D5 or a variant thereof, such as a humanized 4D5. Exemplary humanized 4D5 antibodies include huMAb4D5-1, huMAb4D5-2, huMAb4D5-3, huMAb4D5-4, huMAb4D5-5, huMAb4D5-6, huMAb4D5-7 and huMAb4D5-8 (trastuzumab, HERCEPTIN®) as in US Patent No. 5821337.

"Phage display" is a technique by which variant polypeptides are displayed as fusion proteins to a coat protein on the surface of phage, e.g., filamentous phage, particles. One utility of phage display lies in the fact that large libraries of randomized protein variants can be rapidly and efficiently sorted for those sequences that bind to a target molecule with high affinity. Display of peptide and protein libraries on phage has been used for screening millions of polypeptides for ones with specific binding properties. Polyvalent phage display methods have been used for displaying small random peptides and small proteins, typically through fusions to either pIII or pVIII of filamentous phage (Wells and Lowman, (1992) Curr. Opin. Struct. Biol., 3:355-362, and references cited therein). In monovalent phage display, a protein or peptide library is fused to a phage coat protein or a portion thereof, and expressed at low levels in the presence of wild type protein. Avidity effects are reduced relative to polyvalent phage so that sorting is on the basis of intrinsic ligand affinity, and phagemid vectors are used, which simplify DNA manipulations. Lowman and Wells, Methods: A companion to Methods in Enzymology, 3:205-0216 (1991). Phage display includes techniques for producing antibody-like molecules (Janeway, C., Travers, P., Walport, M., Shlomchik (2001) Immunobiology, 5th Ed., Garland Publishing, New York, p627-628; Lee et al).

A "phagemid" is a plasmid vector having a bacterial origin of replication, *e*.*g*., Co1E1, and a copy of an intergenic region of a bacteriophage. The phagemid may be used on any known bacteriophage, including filamentous bacteriophage and lambdoid bacteriophage. The plasmid will also generally contain a selectable marker for antibiotic resistance. Segments of DNA cloned into these vectors can be propagated as plasmids. When cells harboring these vectors are provided with all genes necessary for the production of phage particles, the mode of replication of the plasmid changes to rolling circle replication to generate copies of one strand of the plasmid DNA and package phage particles. The phagemid may form infectious or non-infectious phage particles. This term includes phagemids which contain a phage coat protein gene or fragment thereof linked to a heterologous polypeptide gene as a gene fusion such that the heterologous polypeptide is displayed on the surface of the phage particle.

"Linker", "Linker Unit", or "link" means a chemical moiety comprising a covalent bond or a chain of atoms that covalently attaches an antibody to a drug moiety. In various embodiments, a linker is specified as L. Linkers include a divalent radical such as an alkyldiyl, an arylene, a heteroarylene, moieties such as: -(CR₂)ₙO(CR₂)ₙ-, repeating units of alkyloxy (e.g. polyethylenoxy, PEG, polymethyleneoxy) and alkylamino (e.g. polyethyleneamino, Jeffamine™); and diacid ester and amides including succinate, succinamide, diglycolate, malonate, and caproamide.

The term "label" means any moiety which can be covalently attached to an antibody and that functions to: (i) provide a detectable signal; (ii) interact with a second label to modify the detectable signal provided by the first or second label, e.g. FRET (fluorescence resonance energy transfer); (iii) stabilize interactions or increase affinity of binding, with antigen or ligand; (iv) affect mobility, e.g. electrophoretic mobility, or cell-permeability, by charge, hydrophobicity, shape, or other physical parameters, or (v) provide a capture moiety, to modulate ligand affinity, antibody/antigen binding, or ionic complexation.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., Stereochemistry of Organic Compounds (1994) John Wiley & Sons, Inc., New York. Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The phrase "pharmaceutically acceptable salt," as used herein, refers to pharmaceutically acceptable organic or inorganic salts of an AZC. Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, *p*-toluenesulfonate, and pamoate (*i.e.,* 1,1'-methylene-bis -(2-hydroxy-3- naphthoate)) salts. A pharmaceutically acceptable salt may involve the inclusion of another molecule such as an acetate ion, a succinate ion or other counterion. The counterion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counterion.

"Pharmaceutically acceptable solvate" refers to an association of one or more solvent molecules and an AZC. Examples of solvents that form pharmaceutically acceptable solvates include, but are not limited to, water, isopropanol, ethanol, methanol, DMSO, ethyl acetate, acetic acid, and ethanolamine.

The following abbreviations are used herein and have the indicated definitions: BME is beta-mercaptoethanol, Boc is *N*-(*t*-butoxycarbonyl), cit is citrulline (2-amino-5-ureido pentanoic acid), dap is dolaproine, DCC is 1,3-dicyclohexylcarbodiimide, DCM is dichloromethane, DEA is diethylamine, DEAD is diethylazodicarboxylate, DEPC is diethylphosphorylcyanidate, DIAD is diisopropylazodicarboxylate, DIEA is *N,N-*diisopropylethylamine, dil is dolaisoleucine, DMA is dimethylacetamide, DMAP is 4-dimethylaminopyridine, DME is ethyleneglycol dimethyl ether (or 1,2-dimethoxyethane), DMF is *N*,*N*-dimethylformamide, DMSO is dimethylsulfoxide, doe is dolaphenine, dov is N,N-dimethylvaline, DTNB is 5,5'-dithiobis(2-nitrobenzoic acid), DTPA is diethylenetriaminepentaacetic acid, DTT is dithiothreitol, EDCI is 1-(3-dimethylaminopropyl)-3-ethylcarbodiimide hydrochloride, EEDQ is 2-ethoxy-1-ethoxycarbonyl-1,2-dihydroquinoline, ES-MS is electrospray mass spectrometry, EtOAc is ethyl acetate, Fmoc is *N*-(9-fluorenylmethoxycarbonyl), gly is glycine, HATU is O-(7-azabenzotriazol-1-yl)-*N*,*N*,*N'*,*N'*-tetramethyluronium hexafluorophosphate, HOBt is 1-hydroxybenzotriazole, HPLC is high pressure liquid chromatography, ile is isoleucine, lys is lysine, MeCN (CH₃CN) is acetonitrile, MeOH is methanol, Mtr is 4-anisyldiphenylmethyl (or 4-methoxytrityl),nor is (*1S*, *2R*)-(+)-norephedrine, PAB is p-aminobenzylcarbamoyl, PBS is phosphate-buffered saline (pH 7), PEG is polyethylene glycol, Ph is phenyl, Pnp is p-nitrophenyl, MC is 6-maleimidocaproyl, phe is L-phenylalanine, PyBrop is bromo *tris-*pyrrolidino phosphonium hexafluorophosphate, SEC is size-exclusion chromatography, Su is succinimide, TFA is trifluoroacetic acid, TLC is thin layer chromatography, UV is ultraviolet, and val is valine.

### CYSTEINE ENGINEERED ANTIBODIES

The compounds of the disclosure include cysteine engineered antibodies where one or more amino acids of a wild-type or parent antibody are replaced with a cysteine amino acid. Any form of antibody may be so engineered, i.e. mutated. For example, a parent Fab antibody fragment may be engineered to form a cysteine engineered Fab, referred to herein as "ThioFab." Similarly, a parent monoclonal antibody may be engineered to form a "ThioMab." It should be noted that a single site mutation yields a single engineered cysteine residue in a ThioFab, while a single site mutation yields two engineered cysteine residues in a ThioMab, due to the dimeric nature of the IgG antibody. Mutants with replaced ("engineered") cysteine (Cys) residues are evaluated for the reactivity of the newly introduced, engineered cysteine thiol groups. The thiol reactivity value is a relative, numerical term in the range of 0 to 1.0 and can be measured for any cysteine engineered antibody. Thiol reactivity values of cysteine engineered antibodies of the disclosure are in the ranges of 0.6 to 1.0; 0.7 to 1.0; or 0.8 to 1.0.

The design, selection, and preparation methods of the disclosure enable cysteine engineered antibodies which are reactive with electrophilic functionality. These methods further enable antibody conjugate compounds such as antibody-zirconium conjugate (AZC) compounds with zirconium atoms at designated, designed, selective sites. Reactive cysteine residues on an antibody surface allow specifically conjugating a zirconium moiety through a thiol reactive group such as maleimide or haloacetyl. The nucleophilic reactivity of the thiol functionality of a Cys residue to a maleimide group is about 1000 times higher compared to any other amino acid functionality in a protein, such as amino group of lysine residues or the N-terminal amino group. Thiol specific functionality in iodoacetyl and maleimide reagents may react with amine groups, but higher pH (>9.0) and longer reaction times are required (Garman, 1997, Non-Radioactive Labelling: A Practical Approach, Academic Press, London).

Cysteine engineered antibodies of the disclosure preferably retain the antigen binding capability of their wild type, parent antibody counterparts. Thus, cysteine engineered antibodies are capable of binding, preferably specifically, to antigens. Such antigens include, for example, tumor-associated antigens (TAA), cell surface receptor proteins and other cell surface molecules, transmembrane proteins, signalling proteins, cell survival regulatory factors, cell proliferation regulatory factors, molecules associated with (for e.g., known or suspected to contribute functionally to) tissue development or differentiation, lymphokines, cytokines, molecules involved in cell cycle regulation, molecules involved in vasculogenesis and molecules associated with (for e.g., known or suspected to contribute functionally to) angiogenesis. The tumor-associated antigen may be a cluster differentiation factor (i.e., a CD protein). An antigen to which a cysteine engineered antibody is capable of binding may be a member of a subset of one of the above-mentioned categories, wherein the other subset(s) of said category comprise other molecules/antigens that have a distinct characteristic (with respect to the antigen of interest).

The parent antibody may also be a humanized antibody selected from huMAb4D5-1, huMAb4D5-2, huMAb4D5-3, huMAb4D5-4, huMAb4D5-5, huMAb4D5-6, huMAb4D5-7 and huMAb4D5-8 (Trastuzumab, HERCEPTIN®) as described in Table 3 of US 5821337; humanized 520C9 (WO 93/21319) and humanized 2C4 antibodies as described herein.

Cysteine engineered antibodies of the disclosure may be site-specifically and efficiently coupled with a thiol-reactive reagent. The thiol-reactive reagent may be a multifunctional linker reagent, a capture, i.e. affinity, label reagent (e.g. a biotin-linker reagent), a detection label (e.g. a fluorophore reagent), a solid phase immobilization reagent (e.g. SEPHAROSE™, polystyrene, or glass), or a zirconium-linker intermediate. One example of a thiol-reactive reagent is N-ethyl maleimide (NEM). In an exemplary embodiment, reaction of a ThioFab with a biotin-linker reagent provides a biotinylated ThioFab by which the presence and reactivity of the engineered cysteine residue may be detected and measured. Reaction of a ThioFab with a multifunctional linker reagent provides a ThioFab with a functionalized linker which may be further reacted with a zirconium moiety reagent or other label. Reaction of a ThioFab with a zirconium-linker intermediate provides a ThioFab zirconium conjugate.

The exemplary methods described here may be applied generally to the identification and production of antibodies, and more generally, to other proteins through application of the design and screening steps described herein.

Such an approach may be applied to the conjugation of other thiol-reactive agents in which the reactive group is, for example, a maleimide, an iodoacetamide, a pyridyl disulfide, or other thiol-reactive conjugation partner (Haugland, 2003, Molecular Probes Handbook of Fluorescent Probes and Research Chemicals, Molecular Probes, Inc.; Brinkley, 1992, Bioconjugate Chem. 3:2; Garman, 1997, Non-Radioactive Labelling: A Practical Approach, Academic Press, London; Means (1990) Bioconjugate Chem. 1:2; Hermanson, G. in Bioconjugate Techniques (1996) Academic Press, San Diego, pp. 40-55, 643-671). The partner may be a cytotoxic agent (e.g. a toxin such as doxorubicin or pertussis toxin), a fluorophore such as a fluorescent dye like fluorescein or rhodamine, a chelating agent for an imaging or radiotherapeutic metal, a peptidyl or non-peptidyl label or detection tag, or a clearance-modifying agent such as various isomers of polyethylene glycol, a peptide that binds to a third component, or another carbohydrate or lipophilic agent.

The sites identified on the exemplary antibody fragment, hu4D5Fabv8, herein are primarily in the constant domain of an antibody which is well conserved across all species of antibodies. These sites should be broadly applicable to other antibodies, without further need of structural design or knowledge of specific antibody structures, and without interference in the antigen binding properties inherent to the variable domains of the antibody.

Cysteine engineered antibodies which may be useful in the treatment of cancer include, but are not limited to, antibodies against cell surface receptors and tumor-associated antigens (TAA). Such antibodies may be used as naked antibodies (unconjugated to a label moiety) or as Formula I antibody-zirconium conjugates (AZC). Tumor-associated antigens are known in the art, and can prepared for use in generating antibodies using methods and information which are well known in the art. In attempts to discover effective cellular targets for cancer diagnosis and therapy, researchers have sought to identify transmembrane or otherwise tumor-associated polypeptides that are specifically expressed on the surface of one or more particular type(s) of cancer cell as compared to on one or more normal non-cancerous cell(s). Often, such tumor-associated polypeptides are more abundantly expressed on the surface of the cancer cells as compared to on the surface of the non-cancerous cells. The identification of such tumor-associated cell surface antigen polypeptides has given rise to the ability to specifically target cancer cells for destruction via antibody-based therapies.

Examples of TAA include, but are not limited to, TAA (1)-(36) listed below. For convenience, information relating to these antigens, all of which are known in the art, is listed below and includes names, alternative names, Genbank accession numbers and primary reference(s), following nucleic acid and protein sequence identification conventions of the National Center for Biotechnology Information (NCBI). Nucleic acid and protein sequences corresponding to TAA (1)-(36) are available in public databases such as GenBank. Tumor-associated antigens targeted by antibodies include all amino acid sequence variants and isoforms possessing at least about 70%, 80%, 85%, 90%, or 95% sequence identity relative to the sequences identified in the cited references, or which exhibit substantially the same biological properties or characteristics as a TAA having a sequence found in the cited references. For example, a TAA having a variant sequence generally is able to bind specifically to an antibody that binds specifically to the TAA with the corresponding sequence listed.

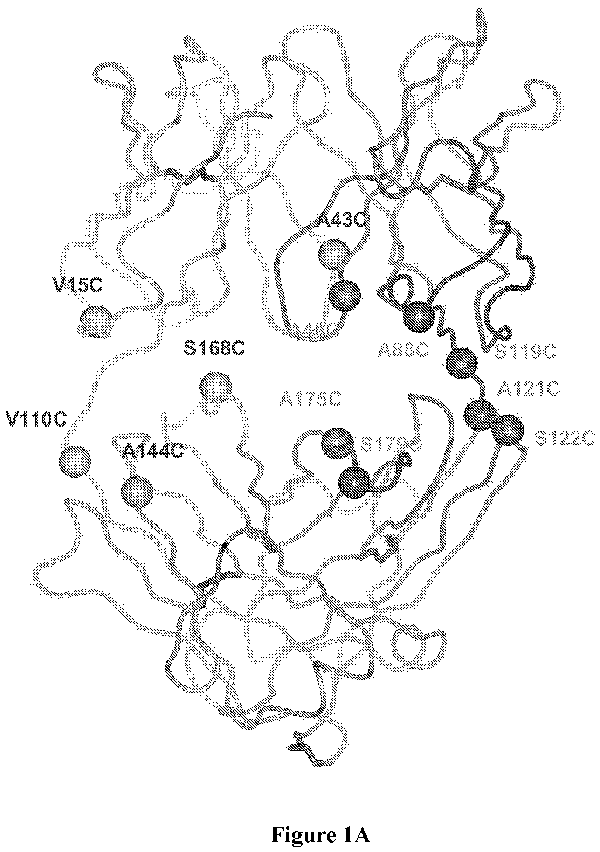

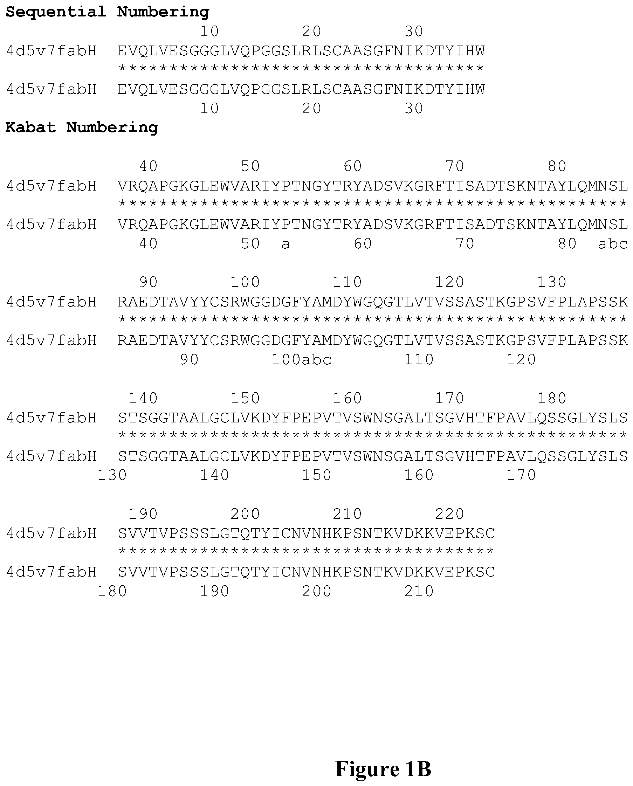

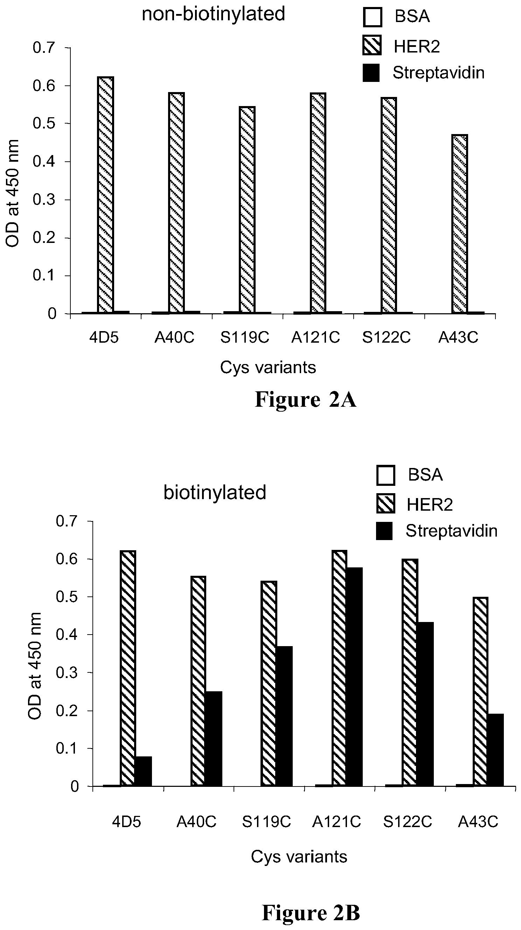

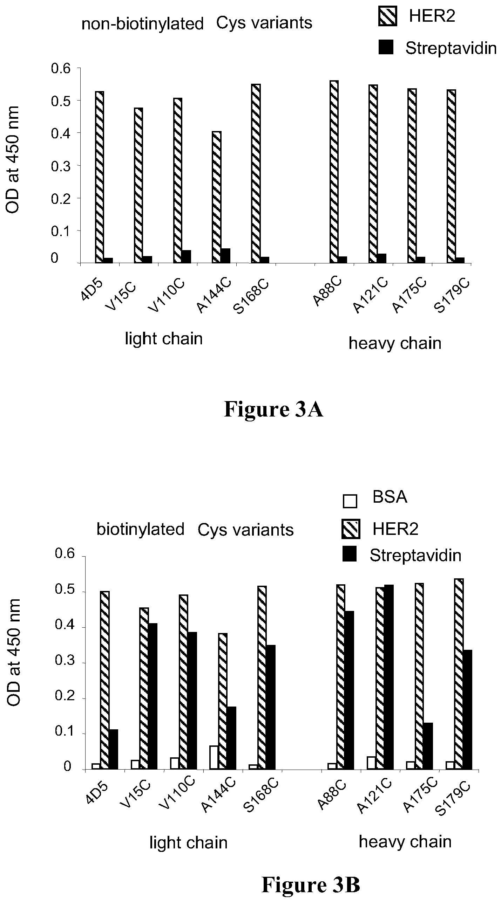

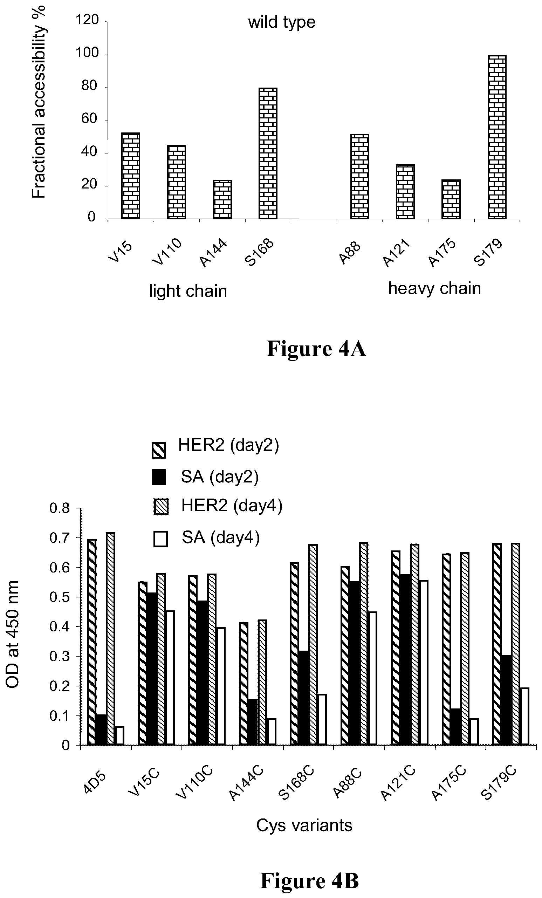

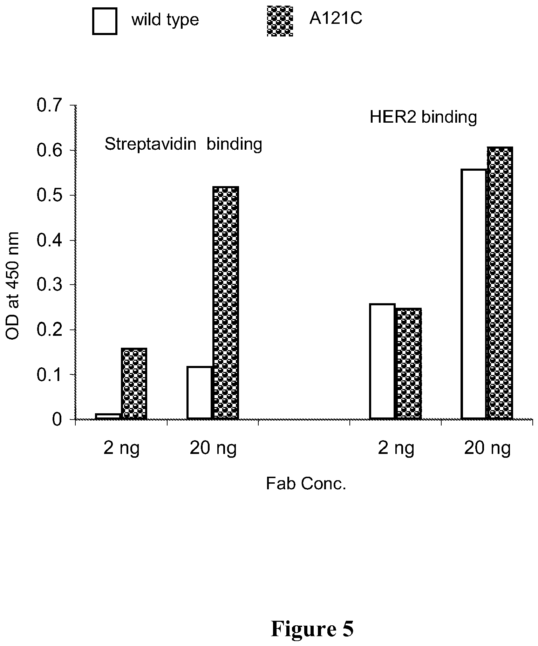

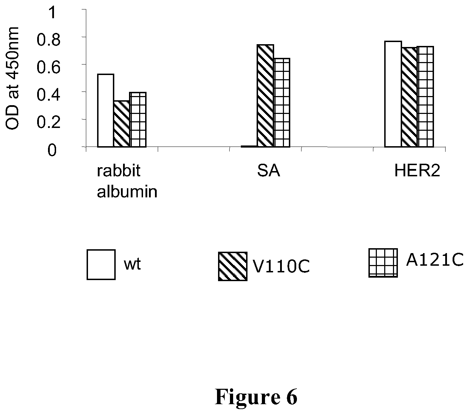

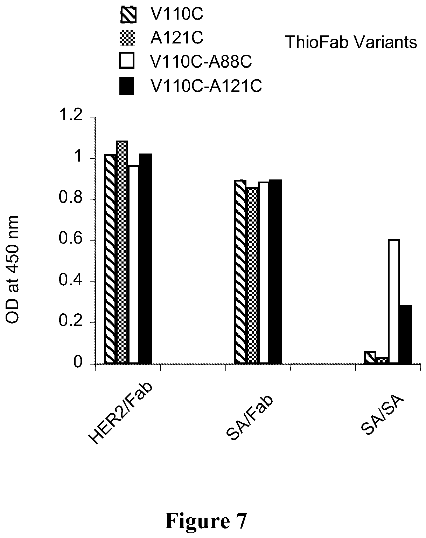

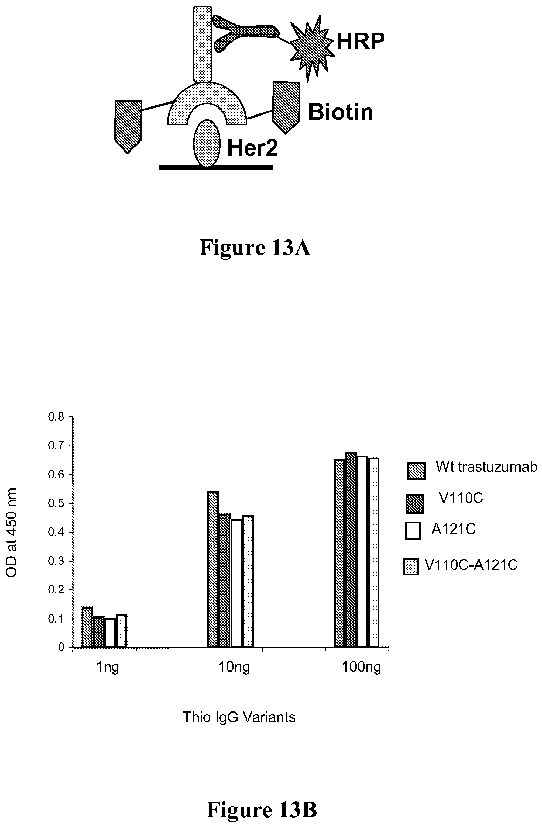

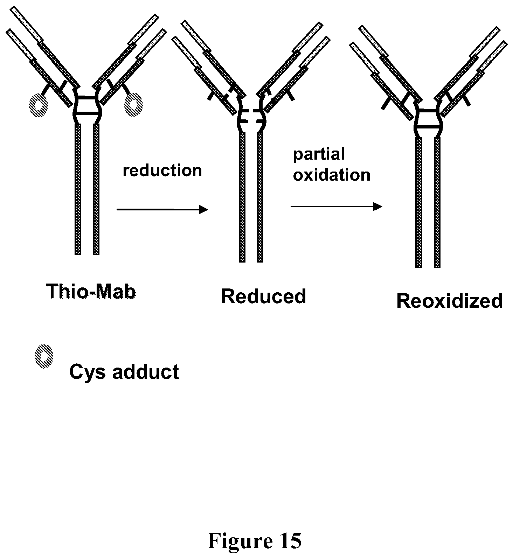

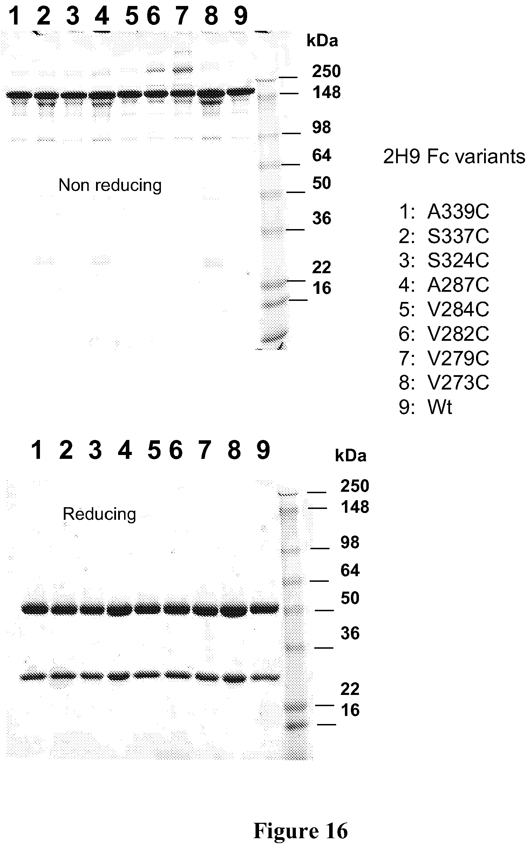

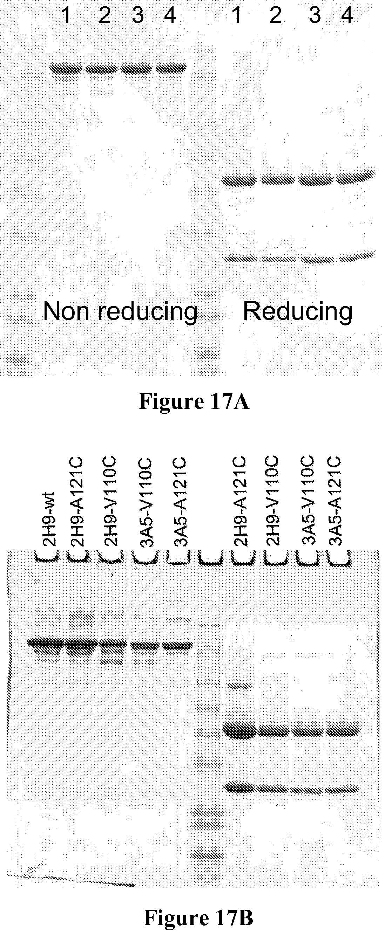

TUMOR-ASSOCIATED ANTIGENS (1)-(36):
(1) BMPR1B (bone morphogenetic protein receptor-type IB, Genbank accession no. NM_001203)
   ten Dijke,P., et al Science 264 (5155):101-104 (1994), Oncogene 14 (11):1377-1382 (1997)); WO2004063362 (Claim 2); WO2003042661 (Claim 12); US2003134790-A1 (Page 38-39); WO2002102235 (Claim 13; Page 296); WO2003055443 (Page 91-92); WO200299122 (Example 2; Page 528-530); WO2003029421 (Claim 6); WO2003024392 (Claim 2; Fig 112); WO200298358 (Claim 1; Page 183); WO200254940 (Page 100-101); WO200259377(Page 349-350); WO200230268 (Claim 27; Page 376); WO200148204 (Example; Fig 4) NP_001194 bone morphogenetic protein receptor, type IB /pid=NP_001194.1 -
   Cross-references: MIM:603248; NP_001194.1; AY065994
(2) E16 (LAT1, SLC7A5, Genbank accession no. NM_003486) Biochem. Biophys. Res. Commun. 255 (2), 283-288 (1999), Nature 395 (6699):288-291 (1998), Gaugitsch, H.W., et al (1992) J. Biol. Chem. 267 (16):11267-11273); WO2004048938 (Example 2); WO2004032842 (Example IV); WO2003042661 (Claim 12); WO2003016475 (Claim 1); WO200278524 (Example 2); WO200299074 (Claim 19; Page 127-129); WO200286443 (Claim 27; Pages 222, 393); WO2003003906 (Claim 10; Page 293); WO200264798 (Claim 33; Page 93-95); WO200014228 (Claim 5; Page 133-136); US2003224454 (Fig 3); WO2003025138 (Claim 12; Page 150); NP_003477 solute carrier family 7 (cationic amino acid transporter, y+ system), member 5 /pid=NP_003477.3 - Homo sapiens
   Cross-references: MIM:600182; NP_003477.3; NM_015923; NM_003486_1
(3) STEAP1 (six transmembrane epithelial antigen of prostate, Genbank accession no. NM_012449)
   Cancer Res. 61 (15), 5857-5860 (2001), Hubert, R.S., et al (1999) Proc. Natl. Acad. Sci. U.S.A. 96 (25):14523-14528); WO2004065577 (Claim 6); WO2004027049 (Fig 1L); EP1394274 (Example 11); WO2004016225 (Claim 2); WO2003042661 (Claim 12); US2003157089 (Example 5); US2003185830 (Example 5); US2003064397 (Fig 2); WO200289747 (Example 5; Page 618-619); WO2003022995 (Example 9; Fig 13A, Example 53; Page 173, Example 2; Fig 2A);
   NP_036581 six transmembrane epithelial antigen of the prostate
   Cross-references: MIM:604415; NP_036581.1; NM_012449_1
(4) 0772P (CA125, MUC16, Genbank accession no. AF361486)
   J. Biol. Chem. 276 (29):27371-27375 (2001)); WO2004045553 (Claim 14); WO200292836 (Claim 6; Fig 12); WO200283866 (Claim 15; Page 116-121); US2003124140 (Example 16); Cross-references: GI:34501467; AAK74120.3; AF361486_1
(5) MPF (MPF, MSLN, SMR, megakaryocyte potentiating factor, mesothelin,
   Genbank accession no. NM_005823) Yamaguchi, N., et al Biol. Chem. 269 (2), 805-808 (1994), Proc. Natl. Acad. Sci. U.S.A. 96 (20):11531-11536 (1999), Proc. Natl. Acad. Sci. U.S.A. 93 (1):136-140 (1996), J. Biol. Chem. 270 (37):21984-21990 (1995)); WO2003101283 (Claim 14); (WO2002102235 (Claim 13; Page 287-288); WO2002101075 (Claim 4; Page 308-309); WO200271928 (Page 320-321); WO9410312 (Page 52-57); Cross-references: MIM:601051; NP_005814.2; NM_005823_1
(6) Napi3b (NAPI-3B, NPTIIb, SLC34A2, solute carrier family 34 (sodium phosphate), member 2, type II sodium-dependent phosphate transporter 3b,Genbank accession no. NM_006424)
   J. Biol. Chem. 277 (22):19665-19672 (2002), Genomics 62 (2):281-284 (1999), Feild, J.A., et al (1999) Biochem. Biophys. Res. Commun. 258 (3):578-582); WO2004022778 (Claim 2); EP1394274 (Example 11); WO2002102235 (Claim 13; Page 326); EP875569 (Claim 1; Page 17-19); WO200157188 (Claim 20; Page 329); WO2004032842 (Example IV); WO200175177 (Claim 24; Page 139-140);
   Cross-references: MIM:604217; NP_006415.1; NM_006424_1
(7) Sema 5b (FLJ10372, KIAA1445, Mm.42015, SEMA5B, SEMAG, Semaphorin 5b Hlog, sema domain, seven thrombospondin repeats (type 1 and type 1-like), transmembrane domain (TM) and short cytoplasmic domain, (semaphorin) 5B, Genbank accession no. AB040878)
   Nagase T., et al (2000) DNA Res. 7 (2):143-150); WO2004000997 (Claim 1); WO2003003984 (Claim 1); WO200206339 (Claim 1; Page 50); WO200188133 (Claim 1; Page 41-43, 48-58); WO2003054152 (Claim 20); WO2003101400 (Claim 11);
   Accession: Q9P283; EMBL; AB040878; BAA95969.1. Genew; HGNC:10737;
(8) PSCA hlg (2700050C12Rik, C530008O16Rik, RIKEN cDNA 2700050C12, RIKEN cDNA 2700050C12 gene, Genbank accession no. AY358628); Ross et al (2002) Cancer Res. 62:2546-2553; US2003129192 (Claim 2); US2004044180 (Claim 12); US2004044179 (Claim 11); US2003096961 (Claim 11); US2003232056 (Example 5); WO2003105758 (Claim 12); US2003206918 (Example 5); EP1347046 (Claim 1); WO2003025148 (Claim 20);
   Cross-references: GI:37182378; AAQ88991.1; AY358628_1
(9) ETBR (Endothelin type B receptor, Genbank accession no. AY275463);
   Nakamuta M., et al Biochem. Biophys. Res. Commun. 177, 34-39, 1991; Ogawa Y., et al Biochem. Biophys. Res. Commun. 178, 248-255, 1991; Arai H., et al Jpn. Circ. J. 56, 1303-1307, 1992; Arai H., et al J. Biol. Chem. 268, 3463-3470, 1993; Sakamoto A., Yanagisawa M., et al Biochem. Biophys. Res. Commun. 178, 656-663, 1991; Elshourbagy N.A., et al J. Biol. Chem. 268, 3873-3879, 1993; Haendler B., et al J. Cardiovasc. Pharmacol. 20, s1-S4, 1992; Tsutsumi M., et al Gene 228, 43-49, 1999; Strausberg R.L., et al Proc. Natl. Acad. Sci. U.S.A. 99, 16899-16903, 2002; Bourgeois C., et al J. Clin. Endocrinol. Metab. 82, 3116-3123, 1997; Okamoto Y., et al Biol. Chem. 272, 21589-21596, 1997; Verheij J.B., et al Am. J. Med. Genet. 108, 223-225, 2002; Hofstra R.M.W., et al Eur. J. Hum. Genet. 5, 180-185, 1997; Puffenberger E.G., et al Cell 79, 1257-1266, 1994; Attie T., et al, Hum. Mol. Genet. 4, 2407-2409, 1995; Auricchio A., et al Hum. Mol. Genet. 5:351-354, 1996; Amiel J., et al Hum. Mol. Genet. 5, 355-357, 1996; Hofstra R.M.W., et al Nat. Genet. 12, 445-447, 1996; Svensson P.J., et al Hum. Genet. 103, 145-148, 1998; Fuchs S., et al Mol. Med. 7, 115-124, 2001; Pingault V., et al (2002) Hum. Genet. 111, 198-206; WO2004045516 (Claim 1); WO2004048938 (Example 2); WO2004040000 (Claim 151); WO2003087768 (Claim 1); WO2003016475 (Claim 1); WO2003016475 (Claim 1); WO200261087 (Fig 1); WO2003016494 (Fig 6); WO2003025138 (Claim 12; Page 144); WO200198351 (Claim 1; Page 124-125); EP522868 (Claim 8; Fig 2); WO200177172 (Claim 1; Page 297-299); US2003109676; US6518404 (Fig 3); US5773223 (Claim 1a; Col 31-34); WO2004001004;
(10) MSG783 (RNF124, hypothetical protein FLJ20315, Genbank accession no. NM_017763);
   WO2003104275 (Claim 1); WO2004046342 (Example 2); WO2003042661 (Claim 12); WO2003083074 (Claim 14; Page 61); WO2003018621 (Claim 1); WO2003024392 (Claim 2; Fig 93); WO200166689 (Example 6);
   Cross-references: LocusID:54894; NP_060233.2; NM_017763_1
(11) STEAP2 (HGNC_8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein, Genbank accession no. AF455138)
   Lab. Invest. 82 (11):1573-1582 (2002)); WO2003087306; US2003064397 (Claim 1; Fig 1); WO200272596 (Claim 13; Page 54-55); WO200172962 (Claim 1; Fig 4B); WO2003104270 (Claim 11); WO2003104270 (Claim 16); US2004005598 (Claim 22); WO2003042661 (Claim 12); US2003060612 (Claim 12; Fig 10); WO200226822 (Claim 23; Fig 2); WO200216429 (Claim 12; Fig 10);
   Cross-references: GI:22655488; AAN04080.1; AF455138_1
(12) TrpM4 (BR22450, FLJ20041, TRPM4, TRPM4B, transient receptor potential cation channel, subfamily M, member 4, Genbank accession no. NM_017636)
   Xu, X.Z., et al Proc. Natl. Acad. Sci. U.S.A. 98 (19):10692-10697 (2001), Cell 109 (3):397-407 (2002), J. Biol. Chem. 278 (33):30813-30820 (2003)); US2003143557 (Claim 4); WO200040614 (Claim 14; Page 100-103); WO200210382 (Claim 1; Fig 9A); WO2003042661 (Claim 12); WO200230268 (Claim 27; Page 391); US2003219806 (Claim 4); WO200162794 (Claim 14; Fig 1A-D);
   Cross-references: MIM:606936; NP_060106.2; NM_017636_1
(13) CRIPTO (CR, CR1, CRGF, CRIPTO, TDGF1, teratocarcinoma-derived growth factor, Genbank accession no. NP_003203 or NM_003212)
   Ciccodicola, A., et al EMBO J. 8 (7):1987-1991 (1989), Am. J. Hum. Genet. 49 (3):555-565 (1991)); US2003224411 (Claim 1); WO2003083041 (Example 1); WO2003034984 (Claim 12); WO200288170 (Claim 2; Page 52-53); WO2003024392 (Claim 2; Fig 58); WO200216413 (Claim 1; Page 94-95, 105); WO200222808 (Claim 2; Fig 1); US5854399 (Example 2; Col 17-18); US5792616 (Fig 2);
   Cross-references: MIM:187395; NP_003203.1; NM_003212_1
(14) CD21 (CR2 (Complement receptor 2) or C3DR (C3d/Epstein Barr virus receptor) or Hs.73792 Genbank accession no. M26004)
   Fujisaku et al (1989) J. Biol. Chem. 264 (4):2118-2125); Weis J.J., et al J. Exp. Med. 167, 1047-1066, 1988; Moore M., et al Proc. Natl. Acad. Sci. U.S.A. 84, 9194-9198, 1987; Barel M., et al Mol. Immunol. 35, 1025-1031, 1998; Weis J.J., et al Proc. Natl. Acad. Sci. U.S.A. 83, 5639-5643, 1986; Sinha S.K., et al (1993) J. Immunol. 150, 5311-5320; WO2004045520 (Example 4); US2004005538 (Example 1); WO2003062401 (Claim 9); WO2004045520 (Example 4); WO9102536 (Fig 9.1-9.9); WO2004020595 (Claim 1);
   Accession: P20023; Q13866; Q14212; EMBL; M26004; AAA35786.1.
(15) CD79b (CD79B, CD79β, IGb (immunoglobulin-associated beta), B29, Genbank accession no. NM_000626 or 11038674)
   Proc. Natl. Acad. Sci. U.S.A. (2003) 100 (7):4126-4131, Blood (2002) 100 (9):3068-3076, Muller et al (1992) Eur. J. Immunol. 22 (6):1621-1625); WO2004016225 (claim 2, Fig 140); WO2003087768, US2004101874 (claim 1, page 102); WO2003062401 (claim 9); WO200278524 (Example 2); US2002150573 (claim 5, page 15); US5644033; WO2003048202 (claim 1, pages 306 and 309); WO 99/558658, US6534482 (claim 13, Fig 17A/B); WO200055351 (claim 11, pages 1145-1146);
   Cross-references: MIM:147245; NP_000617.1; NM_000626_1
(16) FcRH2 (IFGP4, IRTA4, SPAP1A (SH2 domain containing phosphatase anchor protein 1a), SPAP1B, SPAP1C, Genbank accession no. NM_030764, AY358130)
   Genome Res. 13 (10):2265-2270 (2003), Immunogenetics 54 (2):87-95 (2002), Blood 99 (8):2662-2669 (2002), Proc. Natl. Acad. Sci. U.S.A. 98 (17):9772-9777 (2001), Xu, M.J., et al (2001) Biochem. Biophys. Res. Commun. 280 (3):768-775; WO2004016225 (Claim 2); WO2003077836; WO200138490 (Claim 5; Fig 18D-1-18D-2); WO2003097803 (Claim 12); WO2003089624 (Claim 25);
   Cross-references: MIM:606509; NP_110391.2; NM_030764_1
(17) HER2 (ErbB2, Genbank accession no. M11730)
   Coussens L., et al Science (1985) 230(4730):1132-1139); Yamamoto T., et al Nature 319, 230-234, 1986; Semba K., et al Proc. Natl. Acad. Sci. U.S.A. 82, 6497-6501, 1985; Swiercz J.M., et al J. Cell Biol. 165, 869-880, 2004; Kuhns J.J., et al J. Biol. Chem. 274, 36422-36427, 1999; Cho H.-S., et al Nature 421, 756-760, 2003; Ehsani A., et al (1993) Genomics 15, 426-429; WO2004048938 (Example 2); WO2004027049 (Fig 1I); WO2004009622; WO2003081210; WO2003089904 (Claim 9); WO2003016475 (Claim 1); US2003118592; WO2003008537 (Claim 1); WO2003055439 (Claim 29; Fig 1A-B); WO2003025228 (Claim 37; Fig 5C); WO200222636 (Example 13; Page 95-107); WO200212341 (Claim 68; Fig 7); WO200213847 (Page 71-74); WO200214503 (Page 114-117); WO200153463 (Claim 2; Page 41-46); WO200141787 (Page 15); WO200044899 (Claim 52; Fig 7); WO200020579 (Claim 3; Fig 2); US5869445 (Claim 3; Col 31-38); WO9630514 (Claim 2; Page 56-61); EP1439393 (Claim 7); WO2004043361 (Claim 7); WO2004022709; WO200100244 (Example 3; Fig 4);
   Accession: P04626; EMBL; M11767; AAA35808.1. EMBL; M11761; AAA35808.1.
(18) NCA (CEACAM6, Genbank accession no. M18728);
   Barnett T., et al Genomics 3, 59-66, 1988; Tawaragi Y., et al Biochem. Biophys. Res. Commun. 150, 89-96, 1988; Strausberg R.L., et al Proc. Natl. Acad. Sci. U.S.A. 99:16899-16903, 2002; WO2004063709; EP1439393 (Claim 7); WO2004044178 (Example 4); WO2004031238; WO2003042661 (Claim 12); WO200278524 (Example 2); WO200286443 (Claim 27; Page 427); WO200260317 (Claim 2);
   Accession: P40199; Q14920; EMBL; M29541; AAA59915.1. EMBL; M18728;
(19) MDP (DPEP1, Genbank accession no. BC017023)
   Proc. Natl. Acad. Sci. U.S.A. 99 (26):16899-16903 (2002)); WO2003016475 (Claim 1); WO200264798 (Claim 33; Page 85-87); JP05003790 (Fig 6-8); WO9946284 (Fig 9);
   Cross-references: MIM:179780; AAH17023.1; BC017023_1
(20) IL20Rα (IL20Ra, ZCYTOR7, Genbank accession no. AF184971);
   Clark H.F., et al Genome Res. 13, 2265-2270, 2003; Mungall A.J., et al Nature 425, 805-811, 2003; Blumberg H., et al Cell 104, 9-19, 2001; Dumoutier L., et al J. Immunol. 167, 3545-3549, 2001; Parrish-Novak J., et al J. Biol. Chem. 277, 47517-47523, 2002; Pletnev S., et al (2003) Biochemistry 42:12617-12624; Sheikh F., et al (2004) J. Immunol. 172, 2006-2010; EP1394274 (Example 11); US2004005320 (Example 5); WO2003029262 (Page 74-75); WO2003002717 (Claim 2; Page 63); WO200222153 (Page 45-47); US2002042366 (Page 20-21); WO200146261 (Page 57-59); WO200146232 (Page 63-65); WO9837193 (Claim 1; Page 55-59);
   Accession: Q9UHF4; Q6UWA9; Q96SH8; EMBL; AF184971; AAF01320.1.
(21) Brevican (BCAN, BEHAB, Genbank accession no. AF229053)
   Gary S.C., et al Gene 256, 139-147, 2000; Clark H.F., et al Genome Res. 13, 2265-2270, 2003; Strausberg R.L., et al Proc. Natl. Acad. Sci. U.S.A. 99, 16899-16903, 2002; US2003186372 (Claim 11); US2003186373 (Claim 11); US2003119131 (Claim 1; Fig 52); US2003119122 (Claim 1; Fig 52); US2003119126 (Claim 1); US2003119121 (Claim 1; Fig 52); US2003119129 (Claim 1); US2003119130 (Claim 1); US2003119128 (Claim 1; Fig 52); US2003119125 (Claim 1); WO2003016475 (Claim 1); WO200202634 (Claim 1);
(22) EphB2R (DRT, ERK, Hek5, EPHT3, Tyro5, Genbank accession no. NM_004442) Chan,J. and Watt, V.M., Oncogene 6 (6), 1057-1061 (1991) Oncogene 10 (5):897-905 (1995), Annu. Rev. Neurosci. 21:309-345 (1998), Int. Rev. Cytol. 196:177-244 (2000)); WO2003042661 (Claim 12); WO200053216 (Claim 1; Page 41); WO2004065576 (Claim 1); WO2004020583 (Claim 9); WO2003004529 (Page 128-132); WO200053216 (Claim 1; Page 42); Cross-references: MIM:600997; NP_004433.2; NM_004442_1
(23) ASLG659 (B7h, Genbank accession no. AX092328)
   US20040101899 (Claim 2); WO2003104399 (Claim 11); WO2004000221 (Fig 3); US2003165504 (Claim 1); US2003124140 (Example 2); US2003065143 (Fig 60); WO2002102235 (Claim 13; Page 299); US2003091580 (Example 2); WO200210187 (Claim 6; Fig 10); WO200194641 (Claim 12; Fig 7b); WO200202624 (Claim 13; Fig 1A-1B); US2002034749 (Claim 54; Page 45-46); WO200206317 (Example 2; Page 320-321, Claim 34; Page 321-322); WO200271928 (Page 468-469); WO200202587 (Example 1; Fig 1); WO200140269 (Example 3; Pages 190-192); WO200036107 (Example 2; Page 205-207); WO2004053079 (Claim 12); WO2003004989 (Claim 1); WO200271928 (Page 233-234, 452-453); WO 0116318;
(24) PSCA (Prostate stem cell antigen precursor, Genbank accession no. AJ297436) Reiter R.E., et al Proc. Natl. Acad. Sci. U.S.A. 95, 1735-1740, 1998; Gu Z., et al Oncogene 19, 1288-1296, 2000; Biochem. Biophys. Res. Commun. (2000) 275(3):783-788; WO2004022709; EP1394274 (Example 11); US2004018553 (Claim 17); WO2003008537 (Claim 1); WO200281646 (Claim 1; Page 164); WO2003003906 (Claim 10; Page 288); WO200140309 (Example 1; Fig 17); US2001055751 (Example 1; Fig 1b); WO200032752 (Claim 18; Fig 1); WO9851805 (Claim 17; Page 97); WO9851824 (Claim 10; Page 94); WO9840403 (Claim 2; Fig 1B);
   Accession: O43653; EMBL; AF043498; AAC39607.1.
(25) GEDA (Genbank accession No. AY260763);
   AAP14954 lipoma HMGIC fusion-partner-like protein /pid=AAP14954.1 - Homo sapiens Species: Homo sapiens (human)
   WO2003054152 (Claim 20); WO2003000842 (Claim 1); WO2003023013 (Example 3, Claim 20); US2003194704 (Claim 45);
   Cross-references: GI:30102449; AAP14954.1; AY260763_1
(26) BAFF-R (B cell -activating factor receptor, BLyS receptor 3, BR3, Genbank accession No. AF116456); BAFF receptor /pid=NP_443177.1 - Homo sapiens Thompson, J.S., et al Science 293 (5537), 2108-2111 (2001); WO2004058309; WO2004011611; WO2003045422 (Example; Page 32-33); WO2003014294 (Claim 35; Fig 6B); WO2003035846 (Claim 70; Page 615-616); WO200294852 (Col 136-137); WO200238766 (Claim 3; Page 133); WO200224909 (Example 3; Fig 3);
   Cross-references: MIM:606269; NP_443177.1; NM_052945_1; AF132600
(27) CD22 (B-cell receptor CD22-B isoform, BL-CAM, Lyb-8, Lyb8, SIGLEC-2, FLJ22814, Genbank accession No. AK026467);
   Wilson et al (1991) J. Exp. Med. 173:137-146; WO2003072036 (Claim 1; Fig 1); Cross-references: MIM: 107266; NP_001762.1; NM_001771_1
(28) CD79a (CD79A, CD79α, immunoglobulin-associated alpha, a B cell-specific protein that covalently interacts with Ig beta (CD79B) and forms a complex on the surface with Ig M molecules, transduces a signal involved in B-cell differentiation), pI: 4.84, MW: 25028 TM: 2 [P] Gene Chromosome: 19q13.2, Genbank accession No. NP_001774.10) WO2003088808, US20030228319; WO2003062401 (claim 9); US2002150573 (claim 4, pages 13-14); WO9958658 (claim 13, Fig 16); WO9207574 (Fig 1); US5644033; Ha et al (1992) J. Immunol. 148(5):1526-1531; Mueller et al (1992) Eur. J. Biochem. 22:1621-1625; Hashimoto et al (1994) Immunogenetics 40(4):287-295; Preud'homme et al (1992) Clin. Exp. Immunol. 90(1):141-146; Yu et al (1992) J. Immunol. 148(2) 633-637; Sakaguchi et al (1988) EMBO J. 7(11):3457-3464;
(29) CXCR5 (Burkitt's lymphoma receptor 1, a G protein-coupled receptor that is activated by the CXCL13 chemokine, functions in lymphocyte migration and humoral defense, plays a role in HIV-2 infection and perhaps development of AIDS, lymphoma, myeloma, and leukemia); 372 aa, pI: 8.54 MW: 41959 TM: 7 [P] Gene Chromosome: 11q23.3, Genbank accession No. NP_001707.1)
   WO2004040000; WO2004015426; US2003105292 (Example 2); US6555339 (Example 2); WO200261087 (Fig 1); WO200157188 (Claim 20, page 269); WO200172830 (pages 12-13); WO200022129 (Example 1, pages 152-153, Example 2, pages 254-256); WO9928468 (claim 1, page 38); US5440021 (Example 2, col 49-52); WO9428931 (pages 56-58); WO9217497 (claim 7, Fig 5); Dobner et al (1992) Eur. J. Immunol. 22:2795-2799; Barella et al (1995) Biochem. J. 309:773-779;
(30) HLA-DOB (Beta subunit of MHC class II molecule (Ia antigen) that binds peptides and presents them to CD4+ T lymphocytes); 273 aa, pI: 6.56 MW: 30820 TM: 1 [P] Gene Chromosome: 6p21.3, Genbank accession No. NP_002111.1)
   Tonnelle et al (1985) EMBO J. 4(11):2839-2847; Jonsson et al (1989) Immunogenetics 29(6):411-413; Beck et al (1992) J. Mol. Biol. 228:433-441; Strausberg et al (2002) Proc. Natl. Acad. Sci USA 99:16899-16903; Servenius et al (1987) J. Biol. Chem. 262:8759-8766; Beck et al (1996) J. Mol. Biol. 255:1-13; Naruse et al (2002) Tissue Antigens 59:512-519; WO9958658 (claim 13, Fig 15); US6153408 (Col 35-38); US5976551 (col 168-170); US6011146 (col 145-146); Kasahara et al (1989) Immunogenetics 30(1):66-68; Larhammar et al (1985) J. Biol. Chem. 260(26):14111-14119;
(31) P2X5 (Purinergic receptor P2X ligand-gated ion channel 5, an ion channel gated by extracellular ATP, may be involved in synaptic transmission and neurogenesis, deficiency may contribute to the pathophysiology of idiopathic detrusor instability); 422 aa), pI: 7.63, MW: 47206 TM: 1 [P] Gene Chromosome: 17p13.3, Genbank accession No. NP_002552.2) Le et al (1997) FEBS Lett. 418(1-2):195-199; WO2004047749; WO2003072035 (claim 10); Touchman et al (2000) Genome Res. 10:165-173; WO200222660 (claim 20);
   WO2003093444 (claim 1); WO2003087768 (claim 1); WO2003029277 (page 82);
(32) CD72 (B-cell differentiation antigen CD72, Lyb-2) PROTEIN SEQUENCE Full maeaity...tafrfpd (1..359; 359 aa), pI: 8.66, MW: 40225 TM: 1 [P] Gene Chromosome: 9p13.3, Genbank accession No. NP_001773.1)
   WO2004042346 (claim 65); WO2003026493 (pages 51-52, 57-58); WO200075655 (pages 105-106); Von Hoegen et al (1990) J. Immunol. 144(12):4870-4877; Strausberg et al (2002) Proc. Natl. Acad. Sci USA 99:16899-16903;
(33) LY64 (Lymphocyte antigen 64 (RP105), type I membrane protein of the leucine rich repeat (LRR) family, regulates B-cell activation and apoptosis, loss of function is associated with increased disease activity in patients with systemic lupus erythematosis); 661 aa, pI: 6.20, MW: 74147 TM: 1 [P] Gene Chromosome: 5q12, Genbank accession No. NP_005573.1)
   US2002193567; WO9707198 (claim 11, pages 39-42); Miura et al (1996) Genomics 38(3):299-304; Miura et al (1998) Blood 92:2815-2822; WO2003083047; WO9744452 (claim 8, pages 57-61); WO200012130 (pages 24-26);
(34) FcRH1 (Fc receptor-like protein 1, a putative receptor for the immunoglobulin Fc domain that contains C2 type Ig-like and ITAM domains, may have a role in B-lymphocyte differentiation); 429 aa, pI: 5.28, MW: 46925 TM: 1 [P] Gene Chromosome: 1q21-1q22, Genbank accession No. NP_443170.1)
   WO2003077836; WO200138490 (claim 6, Fig 18E-1-18-E-2); Davis et al (2001) Proc. Natl. Acad. Sci USA 98(17):9772-9777; WO2003089624 (claim 8); EP1347046 (claim 1); WO2003089624 (claim 7);
(35) IRTA2 (Immunoglobulin superfamily receptor translocation associated 2, a putative immunoreceptor with possible roles in B cell development and lymphomagenesis; deregulation of the gene by translocation occurs in some B cell malignancies); 977 aa, pI: 6.88 MW: 106468 TM: 1 [P] Gene Chromosome: 1q21, Genbank accession No. Human:AF343662, AF343663, AF343664, AF343665, AF369794, AF397453, AK090423, AK090475, AL834187, AY358085; Mouse:AK089756, AY158090, AY506558; NP_112571.1
   WO2003024392 (claim 2, Fig 97); Nakayama et al (2000) Biochem. Biophys. Res. Commun. 277(1):124-127; WO2003077836; WO200138490 (claim 3, Fig 18B-1-18B-2);
(36) TENB2 (TMEFF2, tomoregulin, TPEF, HPP1, TR, putative transmembrane proteoglycan, related to the EGF/heregulin family of growth factors and follistatin); 374 aa, NCBI Accession: AAD55776, AAF91397, AAG49451, NCBI RefSeq: NP_057276; NCBI Gene: 23671; OMIM: 605734; SwissProt Q9UIK5; Genbank accession No. AF179274; AY358907, CAF85723, CQ782436
   WO2004074320 (SEQ ID NO 810); JP2004113151 (SEQ ID NOS 2, 4, 8); WO2003042661 (SEQ ID NO 580); WO2003009814 (SEQ ID NO 411); EP1295944 (pages 69-70); WO200230268 (page 329); WO200190304 (SEQ ID NO 2706); US2004249130; US2004022727; WO2004063355; US2004197325; US2003232350; US2004005563;
   US2003124579; Horie et al (2000) Genomics 67:146-152; Uchida et al (1999) Biochem. Biophys. Res. Commun. 266:593-602; Liang et al (2000) Cancer Res. 60:4907-12; Glynne-Jones et al (2001) Int J Cancer. Oct 15;94(2):178-84;
(37) PMEL17 (silver homolog; SILV; D12S53E; PMEL17; (SI); (SIL); ME20; gp100) BC001414; BT007202; M32295; M77348; NM_006928; McGlinchey,R.P. et al (2009) Proc. Natl. Acad. Sci. U.S.A. 106 (33), 13731-13736; Kummer,M.P. et al (2009) J. Biol. Chem. 284 (4), 2296-2306;
(38) TMEFF1 (transmembrane protein with EGF-like and two follistatin-like domains 1; Tomoregulin-1; H7365; C9orf2; C9ORF2; U19878; X83961) NM_080655; NM_003692; Harms, P.W. (2003) Genes Dev. 17 (21), 2624-2629; Gery, S. et al (2003) Oncogene 22 (18):2723-2727;
(39) GDNF-Ra1 (GDNF family receptor alpha 1; GFRA1; GDNFR; GDNFRA; RETL1; TRNR1; RET1L; GDNFR-alpha1; GFR-ALPHA-1; U95847; BC014962; NM_145793) NM_005264; Kim,M.H. et al (2009) Mol. Cell. Biol. 29 (8), 2264-2277; Treanor, J.J. et al (1996) Nature 382 (6586):80-83;
(40) Ly6E (lymphocyte antigen 6 complex, locus E; Ly67,RIG-E,SCA-2,TSA-1) NP_002337.1; NM_002346.2; de Nooij-van Dalen,A.G. et al (2003) Int. J. Cancer 103 (6), 768-774; Zammit,D.J. et al (2002) Mol. Cell. Biol. 22 (3):946-952;
(41) TMEM46 (shisa homolog 2 (Xenopus laevis); SHISA2) NP_001007539.1; NM_001007538.1; Furushima,K. et al (2007) Dev. Biol. 306 (2), 480-492; Clark,H.F. et al (2003) Genome Res. 13 (10):2265-2270;
(42) Ly6G6D (lymphocyte antigen 6 complex, locus G6D; Ly6-D, MEGT1) NP_067079.2; NM_021246.2; Mallya, M. et al (2002) Genomics 80 (1):113-123; Ribas,G. et al (1999) J. Immunol. 163 (1):278-287;
(43) LGR5 (leucine-rich repeat-containing G protein-coupled receptor 5; GPR49, GPR67) NP_003658.1; NM_003667.2; Salanti,G. et al (2009) Am. J. Epidemiol. 170 (5):537-545; Yamamoto,Y. et al (2003) Hepatology 37 (3):528-533;
(44) RET (ret proto-oncogene; MEN2A; HSCR1; MEN2B; MTC1; (PTC); CDHF12; Hs.168114; RET51; RET-ELE1) NP_066124.1; NM_020975.4; Tsukamoto,H. et al (2009) Cancer Sci. 100 (10):1895-1901; Narita,N. et al (2009) Oncogene 28 (34):3058-3068;
(45) LY6K (lymphocyte antigen 6 complex, locus K; LY6K; HSJ001348; FLJ35226) NP_059997.3; NM_017527.3; Ishikawa,N. et al (2007) Cancer Res. 67 (24):11601-11611; de Nooij-van Dalen,A.G. et al (2003) Int. J. Cancer 103 (6):768-774;
(46) GPR19 (G protein-coupled receptor 19; Mm.4787) NP_006134.1; NM_006143.2; Montpetit, A. and Sinnett,D. (1999) Hum. Genet. 105 (1-2):162-164; O'Dowd, B.F. et al (1996) FEBS Lett. 394 (3):325-329;
(47) GPR54 (KISS1 receptor; KISS1R; GPR54; HOT7T175; AXOR12) NP_115940.2; NM_032551.4; Navenot, J.M. et al (2009) Mol. Pharmacol. 75 (6):1300-1306; Hata, K. et al (2009) Anticancer Res. 29 (2):617-623;
(48) ASPHD1 (aspartate beta-hydroxylase domain containing 1; LOC253982) NP_859069.2; NM_181718.3; Gerhard, D.S. et al (2004) Genome Res. 14 (10B):2121-2127;
(49) Tyrosinase (TYR; OCAIA; OCA1A; tyrosinase; SHEP3) NP_000363.1; NM_000372.4; Bishop,D.T. et al (2009) Nat. Genet. 41 (8):920-925; Nan, H. et al (2009) Int. J. Cancer 125 (4):909-917;
(50) TMEM118 (ring finger protein, transmembrane 2; RNFT2; FLJ14627) NP_001103373.1; NM_001109903.1; Clark, H.F. et al (2003) Genome Res. 13 (10):2265-2270; Scherer,S.E. et al (2006) Nature 440 (7082):346-351
(51) GPR172A (G protein-coupled receptor 172A; GPCR41; FLJ11856; D15Ertd747e) NP_078807.1; NM_024531.3; Ericsson, T.A. et al (2003) Proc. Natl. Acad. Sci. U.S.A. 100 (11):6759-6764; Takeda, S. et al (2002) FEBS Lett. 520 (1-3):97-101.

The parent antibody may also be a fusion protein comprising an albumin-binding peptide (ABP) sequence (Dennis et al. (2002) "Albumin Binding As A General Strategy For Improving The Pharmacokinetics Of Proteins" J Biol Chem. 277:35035-35043; WO 01/45746). Antibodies of the disclosure include fusion proteins with ABP sequences taught by: (i) Dennis et al (2002) J Biol Chem. 277:35035-35043 at Tables III and IV, page 35038; (ii) US 20040001827 at [0076] SEQ ID NOS: 9-22; and (iii) WO 01/45746 at pages 12-13, SEQ ID NOS: z1-z14.

### MUTAGENESIS

DNA encoding an amino acid sequence variant of the starting polypeptide is prepared by a variety of methods known in the art. These methods include, but are not limited to, preparation by site-directed (or oligonucleotide-mediated) mutagenesis, PCR mutagenesis, and cassette mutagenesis of an earlier prepared DNA encoding the polypeptide. Variants of recombinant antibodies may be constructed also by restriction fragment manipulation or by overlap extension PCR with synthetic oligonucleotides. Mutagenic primers encode the cysteine codon replacement(s). Standard mutagenesis techniques can be employed to generate DNA encoding such mutant cysteine engineered antibodies. General guidance can be found in Sambrook et al Molecular Cloning, A Laboratory Manual, Cold Spring Harbor Laboratory Press, Cold Spring Harbor, N.Y., 1989; and Ausubel et al Current Protocols in Molecular Biology, Greene Publishing and Wiley-Interscience, New York, N.Y., 1993.

Site-directed mutagenesis is one method for preparing substitution variants, i.e. mutant proteins. This technique is well known in the art (see for example, Carter (1985) et al Nucleic Acids Res. 13:4431-4443; Ho et al (1989) Gene (Amst.) 77:51-59; and Kunkel et al (1987) Proc. Natl. Acad. Sci. USA 82:488). Briefly, in carrying out site-directed mutagenesis of DNA, the starting DNA is altered by first hybridizing an oligonucleotide encoding the desired mutation to a single strand of such starting DNA. After hybridization, a DNA polymerase is used to synthesize an entire second strand, using the hybridized oligonucleotide as a primer, and using the single strand of the starting DNA as a template. Thus, the oligonucleotide encoding the desired mutation is incorporated in the resulting double-stranded DNA. Site-directed mutagenesis may be carried out within the gene expressing the protein to be mutagenized in an expression plasmid and the resulting plasmid may be sequenced to confirm the introduction of the desired cysteine replacement mutations (Liu et al (1998) J. Biol. Chem. 273:20252-20260). Site-directed of protocols and formats, including those commercially available, e.g. QuikChange® Multi Site-Directed Mutagenesis Kit (Stratagene, La Jolla, CA).

PCR mutagenesis is also suitable for making amino acid sequence variants of the starting polypeptide. See Higuchi, (1990) in PCR Protocols, pp.177-183, Academic Press; Ito et al (1991) Gene 102:67-70; Bernhard et al (1994) Bioconjugate Chem. 5:126-132; and Vallette et al (1989) Nuc. Acids Res. 17:723-733. Briefly, when small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template.

Another method for preparing variants, cassette mutagenesis, is based on the technique described by Wells et al (1985) Gene 34:315-323. The starting material is the plasmid (or other vector) comprising the starting polypeptide DNA to be mutated. The codon(s) in the starting DNA to be mutated are identified. There must be a unique restriction endonuclease site on each side of the identified mutation site(s). If no such restriction sites exist, they may be generated using the above described oligonucleotide-mediated mutagenesis method to introduce them at appropriate locations in the starting polypeptide DNA. The plasmid DNA is cut at these sites to linearize it. A double-stranded oligonucleotide encoding the sequence of the DNA between the restriction sites but containing the desired mutation(s) is synthesized using standard procedures, wherein the two strands of the oligonucleotide are synthesized separately and then hybridized together using standard techniques. This double-stranded oligonucleotide is referred to as the cassette. This cassette is designed to have 5' and 3' ends that are compatible with the ends of the linearized plasmid, such that it can be directly ligated to the plasmid. This plasmid now contains the mutated DNA sequence. Mutant DNA containing the encoded cysteine replacements can be confirmed by DNA sequencing.

Single mutations are also generated by oligonucleotide directed mutagenesis using double stranded plasmid DNA as template by PCR based mutagenesis (Sambrook and Russel, (2001) Molecular Cloning: A Laboratory Manual, 3rd edition; Zoller et al (1983) Methods Enzymol. 100:468-500; Zoller, M.J. and Smith, M. (1982) Nucl. Acids Res. 10:6487-6500).

In the present invention, hu4D5Fabv8 displayed on M13 phage (Gerstner et al (2002) "Sequence Plasticity In The Antigen-Binding Site Of A Therapeutic Anti-HER2 Antibody", J Mol Biol. 321:851-62) was used for experiments as a model system. Cysteine mutations were introduced in hu4D5Fabv8-phage, hu4D5Fabv8, and ABP-hu4D5Fabv8 constructs. The hu4D5-ThioFab-Phage preps were carried out using the polyethylene glycol (PEG) precipitation method as described earlier (Lowman, Henry B. (1998) Methods in Molecular Biology (Totowa, New Jersey) 87 (Combinatorial Peptide Library Protocols) 249-264).

Oligonucleotides are prepared by the phosphoramidite synthesis method (US 4415732; US 4458066; Beaucage, S. and Iyer, R. (1992) "Advances in the synthesis of oligonucleotides by the phosphoramidite approach", Tetrahedron 48:2223-2311). The phosphoramidite method entails cyclical addition of nucleotide monomer units with a reactive 3' phosphoramidite moiety to an oligonucleotide chain growing on a solid-support comprised of controlled-pore glass or highly crosslinked polystyrene, and most commonly in the 3' to 5' direction in which the 3' terminus nucleoside is attached to the solid-support at the beginning of synthesis (US 5047524; US 5262530). The method is usually practiced using automated, commercially available synthesizers (Applied Biosystems, Foster City, CA). Oligonucleotides can be chemically labelled with non-isotopic moieties for detection, capture, stabilization, or other purposes (Andrus, A. "Chemical methods for 5' non-isotopic labelling of PCR probes and primers" (1995) in PCR 2: A Practical Approach, Oxford University Press, Oxford, pp. 39-54; Hermanson, G. in Bioconjugate Techniques (1996) Academic Press, San Diego, pp. 40-55, 643-671; Keller, G. and Manak, M. in DNA Probes Second Edition (1993), Stockton Press, New York, pp. 121-23).

### PHESELECTOR ASSAY

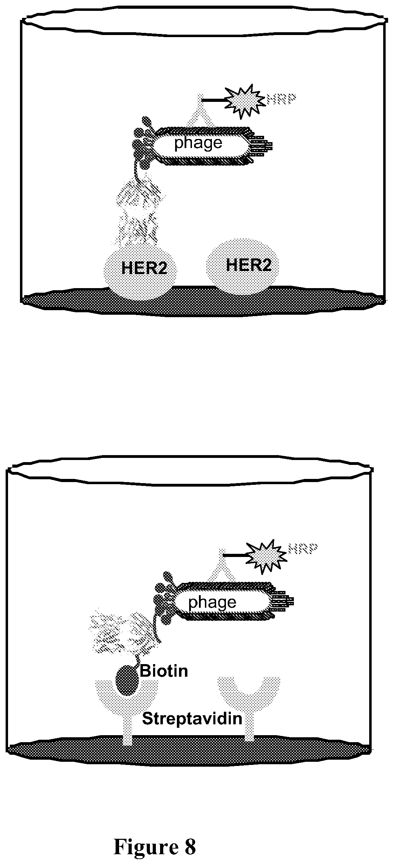

The PHESELECTOR (Phage ELISA for Selection of Reactive Thiols) assay allows for detection of reactive cysteine groups in antibodies in an ELISA phage format (US 7521541; Junutula JR et al. "Rapid identification of reactive cysteine residues for site-specific labeling of antibody-Fabs" J Immunol Methods 2008;332:41-52). The process of coating the protein (e.g. antibody) of interest on well surfaces, followed incubation with phage particles and then HRP labeled secondary antibody with absorbance detection is detailed in Example 2. Mutant proteins displayed on phage may be screened in a rapid, robust, and high-throughput manner. Libraries of cysteine engineered antibodies can be produced and subjected to binding selection using the same approach to identify appropriately reactive sites of free Cys incorporation from random protein-phage libraries of antibodies or other proteins. This technique includes reacting cysteine mutant proteins displayed on phage with an affinity reagent or reporter group which is also thiol-reactive. Figure 8 illustrates the PHESELECTOR Assay by a schematic representation depicting the binding of Fab or ThioFab to HER2 (top) and biotinylated ThioFab to streptavidin (bottom).

### PROTEIN EXPRESSION AND PURIFICATION

DNA encoding the cysteine engineered antibodies is readily isolated and sequenced using conventional procedures (*e.g*., by using oligonucleotide probes that are capable of binding specifically to genes encoding the heavy and light chains of murine antibodies). The hybridoma cells serve as a source of such DNA. Once isolated, the DNA may be placed into expression vectors, which are then transfected into host cells such as *E. coli* cells, simian COS cells, Chinese Hamster Ovary (CHO) cells, or other mammalian host cells, such as myeloma cells (US 5807715; US 2005/0048572; US 2004/0229310) that do not otherwise produce the antibody protein, to obtain the synthesis of monoclonal antibodies in the recombinant host cells. The yields of hu4D5Fabv8 cysteine engineered antibodies were similar to wild type hu4D5Fabv8. Review articles on recombinant expression in bacteria of DNA encoding the antibody include Skerra et al (1993) Curr. Opinion in Immunol. 5:256-262 and Plückthun (1992) Immunol. Revs. 130:151-188.

After design and selection, cysteine engineered antibodies, e.g. ThioFabs, with highly reactive unpaired Cys residues, may be produced by: (i) expression in a bacterial, e.g. *E. coli,* system or a mammalian cell culture system (WO 01/00245), e.g. Chinese Hamster Ovary cells (CHO); and (ii) purification using common protein purification techniques (Lowman et al (1991) J. Biol. Chem. 266(17):10982-10988).

ThioFabs were expressed upon induction in 34B8, a non-suppressor E. *coli* strain (Baca et al (1997) Journal Biological Chemistry 272(16): 10678-84). See Example 3a. The harvested cell pellet was resuspended in PBS (phosphate buffered saline), total cell lysis was performed by passing through a microfluidizer and the ThioFabs were purified by affinity chromatography with protein G SEPHAROSE™ (Amersham). ThioFabs were conjugated with biotin-PEO-maleimide as described above and the biotinylated-ThioFabs were further purified by Superdex-200™ (Amersham) gel filtration chromatography, which eliminated the free biotin-PEO-maleimide and the oligomeric fraction of ThioFabs.

### MASS SPECTROSCOPY ANALYSIS

Liquid chromatography electrospray ionization mass spectrometric (LC-ESI-MS) analysis was employed for the accurate molecular weight determination of biotin conjugated Fab (Cole, R.B. Electro Spray Ionization Mass Spectrometry: Fundamentals, Instrumentation And Applications. (1997) Wiley, New York). The amino acid sequence of biotinylated hu4D5Fabv8 (A121C) peptide was determined by tryptic digestion followed by LC-ESI-Tandem MS analysis (Table 4, Example 3b).

The antibody Fab fragment hu4D5Fabv8 contains about 445 amino acid residues, including 10 Cys residues (five on the light and five on the heavy chain). The high-resolution structure of the humanized 4D5 variable fragment (Fv4D5) has been established, see: Eigenbrot et al "X-Ray Structures Of The Antigen-Binding Domains From Three Variants Of Humanized Anti-P185her2 Antibody 4D5 And Comparison With Molecular Modeling" (1993) J Mol Biol. 229:969-995). All the Cys residues are present in the form of disulfide bonds, therefore these residues do not have any reactive thiol groups to conjugate with zirconium-maleimide (unless treated with a reducing agent). Hence, the newly engineered Cys residue, can remain unpaired, and able to react with, i.e. conjugate to, an electrophilic linker reagent or zirconium-linker intermediate, such as a zirconium-maleimide. Figure 1A shows a three-dimensional representation of the hu4D5Fabv8 antibody fragment derived by X-ray crystal coordinates. The structure positions of the engineered Cys residues of the heavy and light chains are numbered according to a sequential numbering system. This sequential numbering system is correlated to the Kabat numbering system (Kabat et al., (1991) Sequences of Proteins of Immunological Interest, 5th Ed. Public Health Service, National Institutes of Health, Bethesda, MD) for the 4d5v7fabH variant of trastuzumab according to Figure 1B which shows the sequential numbering scheme (top row), starting at the N-terminus, differs from the Kabat numbering scheme (bottom row) by insertions noted by a,b,c. Using the Kabat numbering system, the actual linear amino acid sequence may contain fewer or additional amino acids corresponding to a shortening of, or insertion into, a FR or CDR of the variable domain. The cysteine engineered heavy chain variant sites are identified by the sequential numbering and Kabat numbering schemes in the following chart:

| 4D5Fab Heavy chain variants | Sequential Numbering | Kabat Numbering |
|---|---|---|
| A40C | Ala-40 | Ala-40 |
| A88C | Ala-88 | Ala-84 |
| S119C | Ser-119 | Ser-112 |
| S120C | Ser-120 | Ser-113 |
| A121C | Ala-121 | Ala-114 |
| S122C | Ser-122 | Ser-115 |
| A175C | Ala-175 | Ala-168 |

M13 phagemid-Cys mutant Fabs (Figures 3A and 3B) can be rapidly screened compared to Fab proteins. Phagemid-ThioFab binding to antigen and to streptavidin can be tested by coating HER2 and streptavidin, respectively, onto ELISA plates followed by probing with anti-Fab-HRP (Horse radish peroxidase) as described in Example 2 and depicted in Figure 8. This method allowed simultaneous monitoring of the effect on the antigen binding and the reactivity of the thiol group by the engineered Cys residue/conjugated biotin molecule. Also, the method can be applied to screen the reactive thiol groups for any protein displayed on M13 phage. Conjugated or unconjugated phagemid-ThioFabs are purified by simple PEG precipitation.

The antigen-binding fragment of humanized 4D5 (hu4D5Fab) is well expressed in E. *Coli* and has been displayed on bacteriophage (Garrard et al (1993) Gene 128:103-109). The antibody Fab fragment hu4D5Fabv8 was displayed on M13 phage as a model system in the ELISA based assay to probe thiol reactivity. Figure 8 is a graphical representation of the PHESELECTOR assay, depicting binding of a biotinylated ThioFab phage and an anti-phage HRP antibody to HER2 (top) and Streptavidin (bottom). Five amino acid residues (L-Ala43, H-Ala40, H-Ser119, H-Ala121 and H-Ser122) were initially selected from crystal structure information as remote from the antigen binding surface (Eigenbrot et al. (1993) J Mol Biol. 229:969-995). The Protein Database X-ray crystal structure was designated as 1FVC. Cys residues were engineered at these positions by site directed mutagenesis. ThioFab-phage preparations were isolated and reacted with the biotinylation reagent.

Biotin conjugated and unconjugated variants were tested for HER2 and streptavidin binding using an ELISA based PHESELECTOR assay (Figure 8, Example 2) with an HRP (horseradish peroxidase)-conjugated anti-phage antibody. The interaction of non-biotinylated phage-hu4D5Fabv8 (Figure 2A) and biotinylated phage-hu4D5Fabv8 (Figure 2B) with BSA (open box), HER2 (grey box) or streptavidin (solid box) were monitored through anti-M13-horseradish peroxidase (HRP) antibody by developing a standard HRP reaction and measuring absorbance at 450 nm. The absorbance produced by turnover of a colorimetric substrate was measured at 450 nm. The reactivity of ThioFab with HER2 measures antigen binding. The reactivity of ThioFab with streptavidin measures the extent of biotinylation. The reactivity of ThioFab with BSA is a negative control for nonspecific interaction. As seen in Figure 2A, all the ThioFab-phage variants have similar binding to HER2 compared to that of wild type hu4D5Fabv8-phage. Furthermore, conjugation with biotin did not interfere in the ThioFab binding to HER2 (Figure 2B).

Surprisingly and unexpectedly, the ThioFabs-phage samples showed varying levels of streptavidin binding activity. From all the tested phage-ThioFabs, the A121C cysteine engineered antibody exhibited maximal thiol reactivity. Even though wild type hu4D5Fabv8-phage was incubated with the same amounts of biotin-maleimide, these phage had little streptavidin binding indicating that preexisting cysteine residues (involved in disulfide bond formation) from the hu4D5Fabv8 and M13 phage coat proteins did not interfere with the site-specific conjugation of biotin-maleimide. These results demonstrate that the phage ELISA assay can be used successfully to screen reactive thiol groups on the Fab surface.

The PHESELECTOR assay allows screening of reactive thiol groups in antibodies. Identification of the A121C variant by this method is exemplary. The entire Fab molecule may be effectively searched to identify more ThioFab variants with reactive thiol groups. A parameter, fractional surface accessibility, was employed to identify and quantitate the accessibility of solvent to the amino acid residues in a polypeptide. The surface accessibility can be expressed as the surface area (Å²) that can be contacted by a solvent molecule, e.g. water. The occupied space of water is approximated as a 1.4 Å radius sphere. Software is freely available or licensable (Secretary to CCP4, Daresbury Laboratory, Warrington, WA4 4AD, United Kingdom, Fax: (+44) 1925 603825, or by internet:
www.ccp4.ac.uk/dist/html/INDEX.html) as the CCP4 Suite of crystallography programs which employ algorithms to calculate the surface accessibility of each amino acid of a protein with known x-ray crystallography derived coordinates ("The CCP4 Suite: Programs for Protein Crystallography" (1994) Acta. Cryst. D50:760-763). Two exemplary software modules that perform surface accessibility calculations are "AREAIMOL" and "SURFACE", based on the algorithms of B.Lee and F.M.Richards (1971) J.Mol.Biol. 55:379-400. AREAIMOL defines the solvent accessible surface of a protein as the locus of the centre of a probe sphere (representing a solvent molecule) as it rolls over the Van der Waals surface of the protein. AREAIMOL calculates the solvent accessible surface area by generating surface points on an extended sphere about each atom (at a distance from the atom centre equal to the sum of the atom and probe radii), and eliminating those that lie within equivalent spheres associated with neighboring atoms. AREAIMOL finds the solvent accessible area of atoms in a PDB coordinate file, and summarizes the accessible area by residue, by chain and for the whole molecule. Accessible areas (or area differences) for individual atoms can be written to a pseudo-PDB output file. AREAIMOL assumes a single radius for each element, and only recognizes a limited number of different elements. Unknown atom types (i.e. those not in AREAIMOL's internal database) will be assigned the default radius of 1.8 Å. The list of recognized atoms is:

| Atom | Atomic no. | Van der Waals rad. (Å) |
|---|---|---|
| C | 6 | 1.80 |
| N | 7 | 1.65 |
| O | 8 | 1.60 |
| Mg | 12 | 1.60 |
| S | 16 | 1.85 |
| P | 15 | 1.90 |
| Cl | 17 | 1.80 |
| Co | 27 | 1.80 |

AREAIMOL and SURFACE report absolute accessibilities, i.e. the number of square Angstroms (Å). Fractional surface accessibility is calculated by reference to a standard state relevant for an amino acid within a polypeptide. The reference state is tripeptide Gly-X-Gly, where X is the amino acid of interest, and the reference state should be an 'extended' conformation, i.e. like those in beta-strands. The extended conformation maximizes the accessibility of X. A calculated accessible area is divided by the accessible area in a Gly-X-Gly tripeptide reference state and reports the quotient, which is the fractional accessibility. Percent accessibility is fractional accessibility multiplied by 100.

Another exemplary algorithm for calculating surface accessibility is based on the SOLV module of the program xsae (Broger, C., F. Hoffman-LaRoche, Basel) which calculates fractional accessibility of an amino acid residue to a water sphere based on the X-ray coordinates of the polypeptide.

The fractional surface accessibility for every amino acid in hu4D5Fabv7 was calculated using the crystal structure information (Eigenbrot et al. (1993) J Mol Biol. 229:969-995). The fractional surface accessibility values for the amino acids of the light chain and heavy chain of hu4D5Fabv7 are shown in descending order in Table 1.

**Table 1.**

| **hu4D5Fabv7-light chain** | | | | | | | | | | | |
|---|---|---|---|---|---|---|---|---|---|---|---|
| | | | | | | | | | | | |
| SER | A | 202 | frac | acc= | 101.236 | ASP | A | 151 | frac | acc= | 41.586 |
| GLY | A | 41 | frac | acc= | 90.775 | SER | A | 12 | frac | acc= | 40.633 |
| GLY | A | 157 | frac | acc= | 88.186 | ASN | A | 210 | frac | acc= | 40.158 |
| ASP | A | 1 | frac | acc= | 87.743 | SER | A | 63 | frac | acc= | 39.872 |
| SER | A | 156 | frac | acc= | 83.742 | ARG | A | 66 | frac | acc= | 39.669 |
| GLY | A | 57 | frac | acc= | 81.611 | PRO | A | 8 | frac | acc= | 39.297 |
| SER | A | 168 | frac | acc= | 79.680 | SER | A | 65 | frac | acc= | 39.219 |
| SER | A | 56 | frac | acc= | 79.181 | SER | A | 77 | frac | acc= | 38.820 |
| LYS | A | 169 | frac | acc= | 77.591 | THR | A | 180 | frac | acc= | 38.296 |
| SER | A | 60 | frac | acc= | 75.291 | ASP | A | 185 | frac | acc= | 38.234 |
| THR | A | 109 | frac | acc= | 74.603 | THR | A | 31 | frac | acc= | 38.106 |
| CYS | A | 214 | frac | acc= | 72.021 | THR | A | 94 | frac | acc= | 37.452 |
| LYS | A | 126 | frac | acc= | 71.002 | THR | A | 93 | frac | acc= | 37.213 |
| SER | A | 67 | frac | acc= | 66.694 | THR | A | 197 | frac | acc= | 36.709 |
| ARG | A | 18 | frac | acc= | 66.126 | SER | A | 182 | frac | acc= | 36.424 |
| ASN | A | 152 | frac | acc= | 65.415 | GLY | A | 128 | frac | acc= | 35.779 |
| SER | A | 127 | frac | acc= | 65.345 | LYS | A | 207 | frac | acc= | 35.638 |
| LYS | A | 190 | frac | acc= | 65.189 | ASP | A | 17 | frac | acc= | 35.413 |
| LYS | A | 145 | frac | acc= | 63.342 | GLY | A | 200 | frac | acc= | 35.274 |
| GLN | A | 199 | frac | acc= | 62.470 | GLU | A | 165 | frac | acc= | 35.067 |
| GLU | A | 143 | frac | acc= | 61.681 | ALA | A | 112 | frac | acc= | 34.912 |
| GLN | A | 3 | frac | acc= | 59.976 | GLN | A | 79 | frac | acc= | 34.601 |
| LYS | A | 188 | frac | acc= | 59.680 | VAL | A | 191 | frac | acc= | 33.935 |
| ARG | A | 24 | frac | acc= | 59.458 | SER | A | 208 | frac | acc= | 33.525 |
| PHE | A | 53 | frac | acc= | 58.705 | LYS | A | 39 | frac | acc= | 33.446 |
| SER | A | 9 | frac | acc= | 58.446 | GLU | A | 123 | frac | acc= | 32.486 |
| GLN | A | 27 | frac | acc= | 57.247 | THR | A | 69 | frac | acc= | 32.276 |
| ALA | A | 153 | frac | acc= | 56.538 | SER | A | 76 | frac | acc= | 32.108 |
| SER | A | 203 | frac | acc= | 55.864 | HIS | A | 189 | frac | acc= | 31.984 |
| LYS | A | 42 | frac | acc= | 54.730 | ARG | A | 108 | frac | acc= | 31.915 |
| GLY | A | 16 | frac | acc= | 54.612 | ASN | A | 158 | frac | acc= | 31.447 |
| LYS | A | 45 | frac | acc= | 54.464 | VAL | A | 205 | frac | acc= | 31.305 |
| PRO | A | 204 | frac | acc= | 53.172 | SER | A | 14 | frac | acc= | 31.094 |
| GLU | A | 213 | frac | acc= | 53.084 | GLN | A | 155 | frac | acc= | 30.630 |
| ALA | A | 184 | frac | acc= | 52.556 | GLU | A | 187 | frac | acc= | 30.328 |
| VAL | A | 15 | frac | acc= | 52.460 | ARG | A | 211 | frac | acc= | 30.027 |
| SER | A | 7 | frac | acc= | 51.936 | LYS | A | 183 | frac | acc= | 29.751 |
| LEU | A | 154 | frac | acc= | 51.525 | ASN | A | 138 | frac | acc= | 29.306 |
| GLN | A | 100 | frac | acc= | 51.195 | ASP | A | 170 | frac | acc= | 29.041 |
| SER | A | 10 | frac | acc= | 49.907 | SER | A | 159 | frac | acc= | 27.705 |
| THR | A | 5 | frac | acc= | 48.879 | GLN | A | 147 | frac | acc= | 27.485 |
| THR | A | 206 | frac | acc= | 48.853 | THR | A | 22 | frac | acc= | 27.121 |
| ASP | A | 28 | frac | acc= | 48.758 | ALA | A | 43 | frac | acc= | 26.801 |
| GLY | A | 68 | frac | acc= | 48.690 | ARG | A | 142 | frac | acc= | 26.447 |
| THR | A | 20 | frac | acc= | 48.675 | LEU | A | 54 | frac | acc= | 25.882 |
| ASP | A | 122 | frac | acc= | 47.359 | ASP | A | 167 | frac | acc= | 25.785 |
| PRO | A | 80 | frac | acc= | 46.984 | THR | A | 129 | frac | acc= | 23.880 |
| SER | A | 52 | frac | acc= | 46.917 | ALA | A | 144 | frac | acc= | 23.652 |
| SER | A | 26 | frac | acc= | 46.712 | VAL | A | 163 | frac | acc= | 22.261 |
| TYR | A | 92 | frac | acc= | 46.218 | PRO | A | 95 | frac | acc= | 20.607 |
| LYS | A | 107 | frac | acc= | 45.912 | ALA | A | 111 | frac | acc= | 19.942 |
| GLU | A | 161 | frac | acc= | 45.100 | LYS | A | 103 | frac | acc= | 18.647 |
| VAL | A | 110 | frac | acc= | 44.844 | LEU | A | 181 | frac | acc= | 18.312 |
| GLU | A | 81 | frac | acc= | 44.578 | THR | A | 72 | frac | acc= | 18.226 |
| PRO | A | 59 | frac | acc= | 44.290 | GLU | A | 195 | frac | acc= | 18.006 |
| ASN | A | 30 | frac | acc= | 42.721 | THR | A | 178 | frac | acc= | 17.499 |
| GLN | A | 160 | frac | acc= | 42.692 | THR | A | 85 | frac | acc= | 17.343 |
| SER | A | 114 | frac | acc= | 42.374 | ASP | A | 70 | frac | acc= | 17.194 |
| PRO | A | 40 | frac | acc= | 41.928 | LEU | A | 11 | frac | acc= | 16.568 |
| PHE | A | 116 | frac | acc= | 16.406 | LEU | A | 125 | frac | acc= | 2.398 |
| THR | A | 97 | frac | acc= | 16.204 | PRO | A | 96 | frac | acc= | 2.387 |
| ARG | A | 61 | frac | acc= | 16.192 | LEU | A | 47 | frac | acc= | 2.180 |
| TYR | A | 49 | frac | acc= | 16.076 | ALA | A | 51 | frac | acc= | 1.837 |
| SER | A | 50 | frac | acc= | 15.746 | PHE | A | 118 | frac | acc= | 1.779 |
| LYS | A | 149 | frac | acc= | 15.510 | PHE | A | 62 | frac | acc= | 1.581 |
| GLU | A | 55 | frac | acc= | 14.927 | ALA | A | 25 | frac | acc= | 1.538 |
| LEU | A | 201 | frac | acc= | 14.012 | VAL | A | 133 | frac | acc= | 1.315 |
| GLY | A | 64 | frac | acc= | 13.735 | ASP | A | 82 | frac | acc= | 1.141 |
| GLY | A | 212 | frac | acc= | 13.396 | LEU | A | 179 | frac | acc= | 0.872 |
| PHE | A | 98 | frac | acc= | 12.852 | GLN | A | 124 | frac | acc= | 0.787 |
| THR | A | 74 | frac | acc= | 12.169 | MET | A | 4 | frac | acc= | 0.778 |
| SER | A | 171 | frac | acc= | 11.536 | SER | A | 177 | frac | acc= | 0.693 |
| PRO | A | 141 | frac | acc= | 11.073 | SER | A | 131 | frac | acc= | 0.693 |
| PHE | A | 83 | frac | acc= | 10.871 | LEU | A | 135 | frac | acc= | 0.654 |
| THR | A | 164 | frac | acc= | 10.325 | PHE | A | 71 | frac | acc= | 0.593 |
| ALA | A | 32 | frac | acc= | 9.971 | TRP | A | 35 | frac | acc= | 0.448 |
| HIS | A | 198 | frac | acc= | 9.958 | PHE | A | 209 | frac | acc= | 0.395 |
| VAL | A | 146 | frac | acc= | 9.861 | TYR | A | 186 | frac | acc= | 0.259 |
| SER | A | 121 | frac | acc= | 9.833 | LEU | A | 78 | frac | acc= | 0.157 |
| ALA | A | 13 | frac | acc= | 9.615 | VAL | A | 196 | frac | acc= | 0.000 |
| GLU | A | 105 | frac | acc= | 9.416 | VAL | A | 132 | frac | acc= | 0.000 |
| SER | A | 162 | frac | acc= | 9.304 | VAL | A | 104 | frac | acc= | 0.000 |
| ILE | A | 117 | frac | acc= | 8.780 | VAL | A | 33 | frac | acc= | 0.000 |
| HIS | A | 91 | frac | acc= | 8.557 | VAL | A | 29 | frac | acc= | 0.000 |
| ALA | A | 193 | frac | acc= | 8.547 | TYR | A | 192 | frac | acc= | 0.000 |
| GLN | A | 37 | frac | acc= | 8.442 | TYR | A | 86 | frac | acc= | 0.000 |
| VAL | A | 58 | frac | acc= | 8.281 | TYR | A | 36 | frac | acc= | 0.000 |
| PRO | A | 120 | frac | acc= | 8.095 | THR | A | 102 | frac | acc= | 0.000 |
| GLN | A | 38 | frac | acc= | 6.643 | SER | A | 174 | frac | acc= | 0.000 |
| PRO | A | 113 | frac | acc= | 6.594 | PHE | A | 139 | frac | acc= | 0.000 |
| GLY | A | 101 | frac | acc= | 6.558 | LEU | A | 136 | frac | acc= | 0.000 |
| TYR | A | 140 | frac | acc= | 5.894 | LEU | A | 73 | frac | acc= | 0.000 |
| VAL | A | 115 | frac | acc= | 5.712 | ILE | A | 75 | frac | acc= | 0.000 |
| TYR | A | 87 | frac | acc= | 4.539 | ILE | A | 48 | frac | acc= | 0.000 |
| SER | A | 176 | frac | acc= | 4.106 | ILE | A | 21 | frac | acc= | 0.000 |
| ILE | A | 2 | frac | acc= | 4.080 | GLN | A | 90 | frac | acc= | 0.000 |
| ASN | A | 137 | frac | acc= | 3.906 | GLN | A | 89 | frac | acc= | 0.000 |
| TRP | A | 148 | frac | acc= | 3.676 | CYS | A | 194 | frac | acc= | 0.000 |
| GLY | A | 99 | frac | acc= | 3.550 | CYS | A | 134 | frac | acc= | 0.000 |
| PRO | A | 44 | frac | acc= | 3.543 | CYS | A | 88 | frac | acc= | 0.000 |
| LEU | A | 175 | frac | acc= | 3.488 | CYS | A | 23 | frac | acc= | 0.000 |
| VAL | A | 19 | frac | acc= | 3.420 | ALA | A | 130 | frac | acc= | 0.000 |
| ILE | A | 106 | frac | acc= | 3.337 | ALA | A | 84 | frac | acc= | 0.000 |
| PRO | A | 119 | frac | acc= | 2.953 | ALA | A | 34 | frac | acc= | 0.000 |
| LEU | A | 46 | frac | acc= | 2.887 | | | | | | |
| GLN | A | 6 | frac | acc= | 2.860 | | | | | | |
| TYR | A | 173 | frac | acc= | 2.825 | | | | | | |
| VAL | A | 150 | frac | acc= | 2.525 | | | | | | |
| GLN | A | 166 | frac | acc= | 2.525 | | | | | | |
| THR | A | 172 | frac | acc= | 2.436 | | | | | | |
| | | | | | | | | | | | |

| **hu4D5Fabv7-heavy chain** | | | | | | | | | | | |
|---|---|---|---|---|---|---|---|---|---|---|---|
| | | | | | | | | | | | |
| SER | | 179 | frac | acc= | 99.479 | PRO | B | 14 | frac | acc= | 45.729 |
| GLY | B | 42 | frac | acc= | 95.850 | THR | B | 54 | frac | acc= | 45.503 |
| GLU | B | 1 | frac | acc= | 87.276 | THR | B | 200 | frac | acc= | 45.369 |
| GLY | B | 66 | frac | acc= | 84.541 | LEU | B | 177 | frac | acc= | 45.337 |
| ASP | B | 102 | frac | acc= | 83.794 | GLY | B | 8 | frac | acc= | 44.898 |
| SER | B | 75 | frac | acc= | 80.567 | SER | B | 7 | frac | acc= | 43.530 |
| GLY | B | 140 | frac | acc= | 80.344 | THR | B | 69 | frac | acc= | 43.503 |
| ASN | B | 211 | frac | acc= | 79.588 | PRO | B | 220 | frac | acc= | 43.378 |
| GLY | B | 197 | frac | acc= | 78.676 | LYS | B | 208 | frac | acc= | 43.138 |
| ASP | B | 62 | frac | acc= | 77.716 | LYS | B | 30 | frac | acc= | 42.380 |
| GLY | B | 103 | frac | acc= | 77.176 | ALA | B | 23 | frac | acc= | 41.952 |
| SER | | 163 | frac | acc= | 76.664 | GLU | B | 46 | frac | acc= | 41.430 |
| SER | | 139 | frac | acc= | 74.946 | SER | B | 25 | frac | acc= | 41.323 |
| LYS | B | 213 | frac | acc= | 74.442 | ARG | B | 87 | frac | acc= | 41.282 |
| ALA | B | 165 | frac | acc= | 74.339 | LYS | B | 124 | frac | acc= | 40.888 |
| THR | B | 167 | frac | acc= | 73.934 | ASN | B | 28 | frac | acc= | 40.529 |
| SER | B | 122 | frac | acc= | 72.870 | GLN | B | | frac | acc= | 39.824 |
| SER | B | 194 | frac | acc= | 71.959 | THR | B | 123 | frac | acc= | 39.306 |
| PRO | B | 41 | frac | acc= | 71.540 | SER | B | 63 | frac | acc= | 38.867 |
| THR | B | 198 | frac | acc= | 68.668 | GLY | B | 56 | frac | acc= | 38.582 |
| SER | B | 222 | frac | acc= | 68.128 | GLY | B | 169 | frac | acc= | 38.469 |
| LYS | B | 43 | frac | acc= | 67.782 | THR | B | 172 | frac | acc= | 38.421 |
| GLY | B | 26 | frac | acc= | 67.782 | PRO | B | 209 | frac | acc= | 38.309 |
| THR | B | 138 | frac | acc= | 65.826 | GLY | B | 101 | frac | acc= | 38.040 |
| ASP | B | 31 | frac | acc= | 64.222 | TYR | B | 109 | frac | acc= | 36.829 |
| GLY | B | 15 | frac | acc= | 64.172 | LYS | B | 221 | frac | acc= | 36.520 |
| SER | B | 168 | frac | acc= | 62.100 | GLY | B | 44 | frac | acc= | 35.147 |
| SER | B | 120 | frac | acc= | 61.332 | GLY | B | 181 | frac | acc= | 34.735 |
| LYS | B | 76 | frac | acc= | 61.092 | THR | B | 58 | frac | acc= | 34.457 |
| GLY | B | 141 | frac | acc= | 59.419 | GLY | B | 9 | frac | acc= | 34.254 |
| SER | B | 137 | frac | acc= | 59.179 | VAL | B | 5 | frac | acc= | 34.198 |
| TYR | B | 57 | frac | acc= | 58.916 | ALA | B | 121 | frac | acc= | 33.049 |
| GLU | B | 89 | frac | acc= | 58.483 | SER | B | 127 | frac | acc= | 32.390 |
| SER | B | 180 | frac | acc= | 56.289 | GLY | B | 10 | frac | acc= | 32.230 |
| LYS | B | 65 | frac | acc= | 55.044 | SER | B | 71 | frac | acc= | 30.659 |
| ASP | B | 215 | frac | acc= | 54.656 | ASP | B | 73 | frac | acc= | 30.245 |
| GLN | B | 13 | frac | acc= | 53.719 | LEU | B | 115 | frac | acc= | 29.867 |
| GLN | B | 112 | frac | acc= | 53.215 | LEU | B | 11 | frac | acc= | 29.825 |
| TYR | B | 105 | frac | acc= | 51.940 | ASN | B | 84 | frac | acc= | 29.765 |
| ALA | B | 88 | frac | acc= | 51.602 | SER | B | 210 | frac | acc= | 28.656 |
| GLY | B | 164 | frac | acc= | 50.259 | GLU | B | 155 | frac | acc= | 28.162 |
| PRO | B | 192 | frac | acc= | 49.826 | SER | B | 160 | frac | acc= | 26.526 |
| THR | B | 158 | frac | acc= | 49.694 | CYS | B | 223 | frac | acc= | 26.270 |
| THR | B | 142 | frac | acc= | 48.896 | GLY | B | 16 | frac | acc= | 26.158 |
| ASN | B | 55 | frac | acc= | 48.344 | ILE | B | 202 | frac | acc= | 26.068 |
| LYS | B | 136 | frac | acc= | 48.312 | GLN | B | 82 | frac | acc= | 25.836 |
| ARG | B | 19 | frac | acc= | 48.082 | SER | B | 193 | frac | acc= | 25.550 |
| PRO | B | 156 | frac | acc= | 47.366 | ASN | B | 77 | frac | acc= | 25.418 |
| PRO | B | 174 | frac | acc= | 47.157 | ARG | B | 59 | frac | acc= | 25.301 |
| LYS | B | 217 | frac | acc= | 47.102 | VAL | B | 93 | frac | acc= | 25.254 |
| GLN | B | 199 | frac | acc= | 46.650 | THR | B | 74 | frac | acc= | 24.902 |
| SER | B | 17 | frac | acc= | 45.980 | GLU | B | 219 | frac | acc= | 24.778 |
| SER | B | 85 | frac | acc= | 45.824 | ASN | B | 206 | frac | acc= | 24.647 |
| | | | | | | | | | | | |
| VAL | B | 170 | frac | acc= | 24.549 | PRO | B | 154 | frac | acc= | 6.767 |
| TYR | B | 52 | frac | acc= | 24.298 | PRO | B | 133 | frac | acc= | 6.767 |
| ALA | B | 175 | frac | acc= | 23.804 | TRP | B | 99 | frac | acc= | 6.502 |
| LYS | B | 216 | frac | acc= | 23.277 | THR | B | 32 | frac | acc= | 6.291 |
| VAL | B | 214 | frac | acc= | 23.150 | LEU | B | 45 | frac | acc= | 4.649 |
| GLY | B | 125 | frac | acc= | 22.802 | VAL | B | 128 | frac | acc= | 4.515 |
| ASN | B | 162 | frac | acc= | 22.245 | ILE | B | 51 | frac | acc= | 4.307 |
| ALA | B | 72 | frac | acc= | 22.166 | SER | B | 186 | frac | acc= | 4.084 |
| ALA | B | 40 | frac | acc= | 21.974 | PHE | B | 173 | frac | acc= | 3.969 |
| LEU | B | 18 | frac | acc= | 20.273 | ARG | B | 38 | frac | acc= | 3.734 |
| THR | B | 212 | frac | acc= | 20.170 | TRP | B | 47 | frac | acc= | 3.561 |
| LEU | B | 182 | frac | acc= | 19.619 | VAL | B | 118 | frac | acc= | 3.409 |
| TYR | B | 33 | frac | acc= | 19.398 | ALA | B | 24 | frac | acc= | 3.376 |
| THR | B | 190 | frac | acc= | 19.365 | TYR | B | 95 | frac | acc= | 3.242 |
| VAL | B | 176 | frac | acc= | 18.941 | GLU | B | 6 | frac | acc= | 3.216 |
| SER | B | 21 | frac | acc= | 18.929 | ALA | B | 144 | frac | acc= | 3.167 |
| SER | B | 119 | frac | acc= | 18.877 | ILE | B | 70 | frac | acc= | 1.958 |
| THR | B | 91 | frac | acc= | 18.237 | GLY | B | 111 | frac | acc= | 1.868 |
| ASP | B | 151 | frac | acc= | 17.849 | LEU | B | 4 | frac | acc= | 1.808 |
| THR | B | 114 | frac | acc= | 17.601 | TYR | B | 201 | frac | acc= | 1.758 |
| SER | B | 134 | frac | acc= | 17.571 | LEU | B | 148 | frac | acc= | 1.744 |
| LEU | B | 196 | frac | acc= | 17.090 | PHE | B | 68 | frac | acc= | 1.708 |
| TYR | B | 60 | frac | acc= | 16.575 | VAL | B | 188 | frac | acc= | 1.315 |
| TYR | B | 183 | frac | acc= | 15.968 | CYS | B | 22 | frac | acc= | 0.935 |
| VAL | B | 2 | frac | acc= | 15.901 | TRP | B | 161 | frac | acc= | 0.876 |
| PRO | B | 130 | frac | acc= | 15.342 | LEU | B | 131 | frac | acc= | 0.654 |
| LEU | B | 166 | frac | acc= | 15.268 | VAL | B | 205 | frac | acc= | 0.495 |
| GLY | B | 100 | frac | acc= | 15.003 | ALA | B | 92 | frac | acc= | 0.356 |
| PHE | B | 27 | frac | acc= | 14.383 | ALA | B | 79 | frac | acc= | 0.356 |
| ASN | B | 204 | frac | acc= | 13.873 | VAL | B | 64 | frac | acc= | 0.263 |
| PHE | B | 104 | frac | acc= | 13.836 | ILE | B | 29 | frac | acc= | 0.227 |
| TYR | B | 80 | frac | acc= | 13.490 | VAL | B | 218 | frac | acc= | 0.000 |
| VAL | B | 159 | frac | acc= | 12.782 | VAL | B | 189 | frac | acc= | 0.000 |
| ARG | B | 67 | frac | acc= | 12.362 | VAL | B | 149 | frac | acc= | 0.000 |
| GLN | B | 178 | frac | acc= | 12.131 | VAL | B | 116 | frac | acc= | 0.000 |
| HIS | B | 171 | frac | acc= | 11.412 | VAL | B | 48 | frac | acc= | 0.000 |
| SER | B | 184 | frac | acc= | 11.255 | VAL | B | 37 | frac | acc= | 0.000 |
| ARG | B | 98 | frac | acc= | 11.115 | TYR | B | 152 | frac | acc= | 0.000 |
| PRO | B | 53 | frac | acc= | 11.071 | TYR | B | 94 | frac | acc= | 0.000 |
| GLN | B | 39 | frac | acc= | 11.037 | TRP | B | 36 | frac | acc= | 0.000 |
| SER | B | 195 | frac | acc= | 10.909 | SER | B | 187 | frac | acc= | 0.000 |
| ASP | B | 108 | frac | acc= | 10.525 | SER | B | 97 | frac | acc= | 0.000 |
| LEU | B | 185 | frac | acc= | 10.464 | MET | B | 107 | frac | acc= | 0.000 |
| GLY | B | 113 | frac | acc= | 10.406 | MET | B | 83 | frac | acc= | 0.000 |
| THR | B | 78 | frac | acc= | 10.213 | LEU | B | 145 | frac | acc= | 0.000 |
| THR | B | 117 | frac | acc= | 9.990 | LEU | B | 86 | frac | acc= | 0.000 |
| LYS | B | 150 | frac | acc= | 9.447 | LEU | B | 81 | frac | acc= | 0.000 |
| VAL | B | 157 | frac | acc= | 9.323 | LEU | B | 20 | frac | acc= | 0.000 |
| VAL | B | 12 | frac | acc= | 9.207 | ILE | B | 34 | frac | acc= | 0.000 |
| TRP | B | 110 | frac | acc= | 9.069 | HIS | B | 207 | frac | acc= | 0.000 |
| ALA | B | 143 | frac | acc= | 8.903 | HIS | B | 35 | frac | acc= | 0.000 |
| SER | B | 135 | frac | acc= | 8.897 | GLY | B | 146 | frac | acc= | 0.000 |
| PHE | B | 129 | frac | acc= | 8.895 | CYS | B | 203 | frac | acc= | 0.000 |
| ARG | B | 50 | frac | acc= | 8.639 | CYS | B | 147 | frac | acc= | 0.000 |
| ALA | B | 61 | frac | acc= | 8.547 | CYS | B | 96 | frac | acc= | 0.000 |
| ALA | B | 132 | frac | acc= | 7.882 | ASP | B | 90 | frac | acc= | 0.000 |
| VAL | B | 191 | frac | acc= | 7.366 | ALA | B | 106 | frac | acc= | 0.000 |
| PRO | B | 126 | frac | acc= | 7.258 | ALA | B | 49 | frac | acc= | 0.000 |
| PHE | B | 153 | frac | acc= | 6.918 | | | | | | |

The following two criteria were applied to identify the residues of hu4D5Fabv8 that can be engineered to replace with Cys residues:
1. Amino acid residues that are completely buried are eliminated, i.e. less than 10% fractional surface accessibility. Table 1 shows there are 134 (light chain) and 151 (heavy chain) residues of hu4D5Fabv8 that are more than 10% accessible (fractional surface accessibility). The top ten most accessible Ser, Ala and Val residues were selected due to their close structural similarity to Cys over other amino acids, introducing only minimal structural constraints in the antibody by newly engineered Cys. Other cysteine replacement sites can also be screened, and may be useful for conjugation.
2. Residues are sorted based on their role in functional and structural interactions of Fab. The residues which are not involved in antigen interactions and distant from the existing disulfide bonds were further selected. The newly engineered Cys residues should be distinct from, and not interfere with, antigen binding nor mispair with cysteines involved in disulfide bond formation.

The following residues of hu4D5Fabv8 possessed the above criteria and were selected to be replaced with Cys: L-V15, L-A43, L-V110, L-A144, L-S168, H-A88, H-A121, H-S122, H-A175 and H-S179 (shown in Figure 1).

Thiol reactivity may be generalized to any antibody where substitution of amino acids with reactive cysteine amino acids may be made within the ranges in the light chain selected from: L-10 to L-20; L-38 to L-48; L-105 to L-115; L-139 to L-149; L-163 to L-173; and within the ranges in the heavy chain selected from: H-35 to H-45; H-83 to H-93; H-114 to H-127; and H-170 to H-184, and in the Fc region within the ranges selected from H-268 to H-291; H-319 to H-344; H-370 to H-380; and H-395 to H-405.

Thiol reactivity may also be generalized to certain domains of an antibody, such as the light chain constant domain (CL) and heavy chain constant domains, CH1, CH2 and CH3. Cysteine replacements resulting in thiol reactivity values of 0.6 and higher may be made in the heavy chain constant domains α, δ, ε, γ, and µ of intact antibodies: IgA, IgD, IgE, IgG, and IgM, respectively, including the IgG subclasses: IgG1, IgG2, IgG3, IgG4, IgA, and IgA2.

It is evident from the crystal structure data that the selected 10 Cys mutants are far away from the antigen-combining site, such as the interface with HER2 in this case. These mutants can be tested experimentally for indirect effects on functional interactions. The thiol reactivities of all the Cys Fab variants were measured and calculated as described in Examples 1 and 2, and presented in Table 2. The residues L-V15C, L-V110C, H-A88C and H-A121C have reactive and stable thiol groups (Figures 3A and 3B). Mutants V15C, V110C, A144C, S168C are light chain Cys variants. Mutants A88C, A121C, A175C, S179C are heavy chain Cys variants. It was surprising and unexpected that the sites with high fractional surface accessibility did not have the highest thiol reactivity as calculated by the PHESELECTOR assay (Table 2). In other words, fractional surface accessibility (Tables 1, 2) did not correlate with thiol reactivity (Table 2). In fact, the Cys residues engineered at the sites with moderate surface accessibility of 20% to 80% (Figure 4A, Table 1), or partially exposed sites, like Ala or Val residues, exhibited better thiol reactivity, i.e. >0.6, (Figure 3B, Table 2) than the Cys introduced at Ser residues, thus necessitating the use of PHESELECTOR assay in the screening of thiol reactive sites since the crystal structure information alone is not sufficient to select these sites (Figure 3B and 4A).

Thiol reactivity data is shown in Figures 3A and 3B for amino acid residues of 4D5 ThioFab Cys mutants: (3A) non-biotinylated (control) and (3B) biotinylated phage-ThioFabs. Reactive thiol groups on antibody/Fab surface were identified by PHESELECTOR assay analyses for the interaction of non-biotinylated phage-hu4D5Fabv8 (3A) and biotinylated phage-hu4D5Fabv8 (3B) with BSA (open box), HER2 (grey box) or streptavidin (solid box). The assay was carried out as described in Example 2. Light chain variants are on the left side and heavy chain variants are on the right side. The binding of non-biotinylated 4D5 ThioFab Cys mutants is low as expected, but strong binding to HER2 is retained. The ratio of binding to streptavidin and to HER2 of the biotinylated 4D5 ThioFab Cys mutants gives the thiol reactivity values in Table 2. Background absorbance at 450 nm or small amounts of non-specific protein binding of the biotinylated 4D5 ThioFab Cys mutants to BSA is also evident in Figure 3B. Fractional Surface Accessibility values of the selected amino acid residues that were replaced with a Cys residue are shown in Figure 4A. Fractional surface accessibility was calculated from the available hu4D5Fabv7 structure and shown on Table 1 (Eigenbrot et al. (1993) J Mol Biol. 229:969-995). The conformational parameters of the hu4D5Fabv7 and hu4D5Fabv8 structures are highly consistent and allow for determination of any correlation between fractional surface accessibility calculations of hu4D5Fabv7 and thiol reactivity of hu4D5Fabv8 cysteine mutants. The measured thiol reactivity of phage ThioFab Cys residues introduced at partially exposed residues (Ala or Val) have better thiol reactivity compared to the ones introduced at Ser residues (Table 2). It can be seen from the ThioFab Cys mutants of Table 2 that there is little or no correlation between thio reactivity values and fractional surface accessibility.

Amino acids at positions L-15, L-43, L-110, L-144, L-168, H-40, H-88, H-119, H-121, H-122, H-175, and H-179 of an antibody may generally be mutated (replaced) with free cysteine amino acids. Ranges within about 5 amino acid residues on each side of these positions may also be replaced with free cysteine acids, i.e. L-10 to L-20; L-38 to L-48; L-105 to L-115; L-139 to L-149; L-163 to L-173; H-35 to H-45; H-83 to H-93; H-114 to H-127; and H-170 to H-184, as well as the ranges in the Fc region selected from H-268 to H-291; H-319 to H-344; H-370 to H-380; and H-395 to H-405, to yield the cysteine engineered antibodies of the disclosure.

**Table 2. Thiol reactivity of phage-ThioFabs**

| Phage-ThioFab construct | Thiol Reactivity* | Fractional Surface Accessibility (%) (from Table 1) |
|---|---|---|
| hu4D5Fabv8-wt | 0.125 | - |
| L-V15C | 0.934 | 52.46 |
| L-A43C | 0.385 | 26.80 |
| L-V110C | 0.850 | 44.84 |
| L-A144C | 0.373 | 23.65 |
| L-S168C | 0.514 | 79.68 |
| H-A40C | 0.450 | 21.97 |
| H-A88C | 0.914 | 51.60 |
| H-S119C | 0.680 | 18.88 |
| H-A121C | 0.925 | 33.05 |
| H-S122C | 0.720 | 72.87 |
| H-A175C | 0.19 | 23.80 |
| H-S179C | 0.446 | 99.48 |

| | | |
|---|---|---|
| L = light chain, H = heavy chain, A = alanine, S = serine, V = valine, C = cysteine * Thiol reactivity is measured as the ratio of OD_{450 nm} for streptavidin binding to OD_{450 nm} for HER2 (antibody) binding (Example 2). Thiol reactivity value of 1 indicates complete biotinylation of the cysteine thiol. | | |

Two Cys variants from light chain (L-V15C and L-V110C) and two from heavy chain (H-A88C and H-A121C) were selected for further analysis as these variants showed the highest thiol reactivity (Table 2).

Unlike phage purification, Fab preparation may require 2-3 days, depending on the scale of production. During this time, thiol groups may lose reactivity due to oxidation. To probe the stability of thiol groups on hu4D5Fabv8-phage, stability of the thiol reactivity of phage-thioFabs was measured (Figure 4B). After ThioFab-phage purification, on day 1, day 2 and day 4, all the samples were conjugated with biotin-PEO-maleimide and probed with phage ELISA assay (PHESELECTOR) to test HER2 and streptavidin binding. L-V15C, L-V110C, H-A88C and H-A121C retain significant amounts of thiol reactivity compared to other ThioFab variants (Figure 4B).

### METHODS TO PREPARE CYSTEINE ENGINEERED ANTIBODIES

The compounds of the disclosure include cysteine engineered antibodies where one or more amino acids of a parent antibody are replaced with a free cysteine amino acid. A cysteine engineered antibody comprises one or more free cysteine amino acids having a thiol reactivity value in the range of 0.6 to 1.0. A free cysteine amino acid is a cysteine residue which has been engineered into the parent antibody and is not part of a disulfide bridge.

In one aspect, the cysteine engineered antibody is prepared by a process comprising:
(a) replacing one or more amino acid residues of a parent antibody by cysteine; and
(b) determining the thiol reactivity of the cysteine engineered antibody by reacting the cysteine engineered antibody with a thiol-reactive reagent.

The cysteine engineered antibody may be more reactive than the parent antibody with the thiol-reactive reagent.

The free cysteine amino acid residues may be located in the heavy or light chains, or in the constant or variable domains. Antibody fragments, e.g. Fab, may also be engineered with one or more cysteine amino acids replacing amino acids of the antibody fragment, to form cysteine engineered antibody fragments.

Another aspect of the disclosure provides a method of preparing (making) a cysteine engineered antibody, comprising:
(a) introducing one or more cysteine amino acids into a parent antibody in order to generate the cysteine engineered antibody; and
(b) determining the thiol reactivity of the cysteine engineered antibody with a thiol-reactive reagent;
wherein the cysteine engineered antibody is more reactive than the parent antibody with the thiol-reactive reagent.

Step (a) of the method of preparing a cysteine engineered antibody may comprise:
(i) mutagenizing a nucleic acid sequence encoding the cysteine engineered antibody;
(ii) expressing the cysteine engineered antibody; and
(iii) isolating and purifying the cysteine engineered antibody.

Step (b) of the method of preparing a cysteine engineered antibody may comprise expressing the cysteine engineered antibody on a viral particle selected from a phage or a phagemid particle.

Step (b) of the method of preparing a cysteine engineered antibody may also comprise:
(i) reacting the cysteine engineered antibody with a thiol-reactive affinity reagent to generate an affinity labelled, cysteine engineered antibody; and
(ii) measuring the binding of the affinity labelled, cysteine engineered antibody to a capture media.

Another aspect of the disclosure is a method of screening cysteine engineered antibodies with highly reactive, unpaired cysteine amino acids for thiol reactivity comprising:
(a) introducing one or more cysteine amino acids into a parent antibody in order to generate a cysteine engineered antibody;
(b) reacting the cysteine engineered antibody with a thiol-reactive affinity reagent to generate an affinity labelled, cysteine engineered antibody; and
(c) measuring the binding of the affinity labelled, cysteine engineered antibody to a capture media; and
(d) determining the thiol reactivity of the cysteine engineered antibody with the thiol-reactive reagent.

Step (a) of the method of screening cysteine engineered antibodies may comprise:
(i) mutagenizing a nucleic acid sequence encoding the cysteine engineered antibody;
(ii) expressing the cysteine engineered antibody; and
(iii) isolating and purifying the cysteine engineered antibody.

Step (b) of the method of screening cysteine engineered antibodies may comprise expressing the cysteine engineered antibody on a viral particle selected from a phage or a phagemid particle.

Step (b) of the method of screening cysteine engineered antibodies may also comprise:
(i) reacting the cysteine engineered antibody with a thiol-reactive affinity reagent to generate an affinity labelled, cysteine engineered antibody; and
(ii) measuring the binding of the affinity labelled, cysteine engineered antibody to a capture media.

### LABELLED CYSTEINE ENGINEERED ANTIBODIES

The cysteine engineered antibodies of the disclosure may be conjugated with any label moiety which can be covalently attached to the antibody through a reactive cysteine thiol group (Singh et al (2002) Anal. Biochem. 304:147-15; Harlow E. and Lane, D. (1999) Using Antibodies: A Laboratory Manual, Cold Springs Harbor Laboratory Press, Cold Spring Harbor, NY; Lundblad R.L. (1991) Chemical Reagents for Protein Modification, 2nd ed. CRC Press, Boca Raton, FL). The attached label may function to: (i) provide a detectable signal; (ii) interact with a second label to modify the detectable signal provided by the first or second label, e.g. to give FRET (fluorescence resonance energy transfer); (iii) stabilize interactions or increase affinity of binding, with antigen or ligand; (iv) affect mobility, e.g. electrophoretic mobility or cell-permeability, by charge, hydrophobicity, shape, or other physical parameters, or (v) provide a capture moiety, to modulate ligand affinity, antibody/antigen binding, or ionic complexation.

Labelled cysteine engineered antibodies may be useful in diagnostic assays, e.g., for detecting expression of an antigen of interest in specific cells, tissues, or serum. For diagnostic applications, the antibody will typically be labeled with a detectable moiety. Numerous labels are available which can be generally grouped into the following categories:
(a) Radioisotopes (radionuclides), such as ³H, ¹¹C, ¹⁴C, ¹⁸F, ³²P, ³⁵S, ⁶⁴Cu, ⁶⁸Ga, ⁸⁶Y, ⁸⁹Zr_{,} ⁹⁹Tc, ¹¹¹In, ¹²³I, ¹²⁴I, ¹²⁵I, ¹³¹I, ¹³³Xe, ¹⁷⁷LU, ²¹¹At, or ²¹³Bi. Radioisotope labelled antibodies are useful in receptor targeted imaging experiments. The antibody can be labeled with ligand reagents that bind, chelate or otherwise complex a radioisotope metal where the reagent is reactive with the engineered cysteine thiol of the antibody, using the techniques described in Current Protocols in Immunology, Volumes 1 and 2, Coligen et al, Ed. Wiley-Interscience, New York, NY, Pubs. (1991). Chelating ligands which may complex a metal ion include DOTA, DOPA, DOTP, DOTMA, DTPA and TETA (Macrocyclics, Dallas, TX). Radionuclides can be targetted via complexation with cysteine-engineered antibodies as antibody-zirconium conjugates of the disclosure (Wu et al (2005) Nature Biotechnology 23(9):1137-1146).
   Metal-chelate complexes suitable as antibody labels for imaging experiments are disclosed: US 5342606; US 5428155; US 5316757; US 5480990; US 5462725; US 5428139; US 5385893; US 5739294; US 5750660; US 5834456; Hnatowich et al (1983) J. Immunol. Methods 65:147-157; Meares et al (1984) Anal. Biochem. 142:68-78; Mirzadeh et al (1990) Bioconjugate Chem. 1:59-65; Meares et al (1990) J. Cancer1990, Suppl. 10:21-26; Izard et al (1992) Bioconjugate Chem. 3:346-350; Nikula et al (1995) Nucl. Med. Biol. 22:387-90; Camera et al (1993) Nucl. Med. Biol. 20:955-62; Kukis et al (1998) J. Nucl. Med. 39:2105-2110; Verel et al (2003) J. Nucl. Med. 44:1663-1670; Camera et al (1994) J. Nucl. Med. 21:640-646; Ruegg et al (1990) Cancer Res. 50:4221-4226; Verel et al (2003) J. Nucl. Med. 44:1663-1670; Lee et al (2001) Cancer Res. 61:4474-4482; Mitchell, et al (2003) J. Nucl. Med. 44:1105-1112; Kobayashi et al (1999) Bioconjugate Chem. 10:103-111; Miederer et al (2004) J. Nucl. Med. 45:129-137; DeNardo et al (1998) Clinical Cancer Research 4:2483-90; Blend et al (2003) Cancer Biotherapy & Radiopharmaceuticals 18:355-363; Nikula et al (1999) J. Nucl. Med. 40:166-76; Kobayashi et al (1998) J. Nucl. Med. 39:829-36; Mardirossian et al (1993) Nucl. Med. Biol. 20:65-74; Roselli et al (1999) Cancer Biotherapy & Radiopharmaceuticals, 14:209-20.
(b) Fluorescent labels such as rare earth chelates (europium chelates), fluorescein types including FITC, 5-carboxyfluorescein, 6-carboxy fluorescein; rhodamine types including TAMRA; dansyl; Lissamine; cyanines; phycoerythrins; Texas Red; and analogs thereof. The fluorescent labels can be conjugated to antibodies using the techniques disclosed in *Current Protocols in Immunology, supra,* for example. Fluorescent dyes and fluorescent label reagents include those which are commercially available from Invitrogen/Molecular Probes (Eugene, OR) and Pierce Biotechnology, Inc. (Rockford, IL).
(c) Various enzyme-substrate labels are available or disclosed (US 4275149). The enzyme generally catalyzes a chemical alteration of a chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (*e.g*., firefly luciferase and bacterial luciferase; US 4737456), luciferin, 2,3-dihydrophthalazinediones, malate dehydrogenase, urease, peroxidase such as horseradish peroxidase (HRP), alkaline phosphatase (AP), β-galactosidase, glucoamylase, lysozyme, saccharide oxidases (*e.g*., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al (1981) "Methods for the Preparation of Enzyme-Antibody Conjugates for use in Enzyme Immunoassay", in Methods in Enzym. (ed J. Langone & H. Van Vunakis), Academic Press, New York, 73:147-166.

Examples of enzyme-substrate combinations include, for example:
(i) Horseradish peroxidase (HRP) with hydrogen peroxidase as a substrate, wherein the hydrogen peroxidase oxidizes a dye precursor (e.g., orthophenylene diamine (OPD) or 3,3',5,5'-tetramethylbenzidine hydrochloride (TMB));
(ii) alkaline phosphatase (AP) with para-nitrophenyl phosphate as chromogenic substrate; and
(iii) β-D-galactosidase (β-D-Gal) with a chromogenic substrate (*e.g*., p-nitrophenyl-β-D-galactosidase) or fluorogenic substrate 4-methylumbelliferyl-β-D-galactosidase.

Numerous other enzyme-substrate combinations are available to those skilled in the art. For a general review, see US 4275149 and US 4318980.

A label may be indirectly conjugated with a cysteine engineered antibody. For example, the antibody can be conjugated with biotin and any of the three broad categories of labels mentioned above can be conjugated with avidin or streptavidin, or vice versa. Biotin binds selectively to streptavidin and thus, the label can be conjugated with the antibody in this indirect manner. Alternatively, to achieve indirect conjugation of the label with the polypeptide variant, the polypeptide variant is conjugated with a small hapten (e.g., digoxin) and one of the different types of labels mentioned above is conjugated with an anti-hapten polypeptide variant (*e.g*., anti-digoxin antibody). Thus, indirect conjugation of the label with the polypeptide variant can be achieved (Hermanson, G. (1996) in Bioconjugate Techniques Academic Press, San Diego).

The polypeptide variant of the present disclosure may be employed in any known assay method, such as ELISA, competitive binding assays, direct and indirect sandwich assays, and immunoprecipitation assays (Zola, (1987) Monoclonal Antibodies: A Manual of Techniques, pp. 147-158, CRC Press, Inc.).

A detection label may be useful for localizing, visualizing, and quantitating a binding or recognition event. The labelled antibodies of the disclosure can detect cell-surface receptors. Another use for detectably labelled antibodies is a method of bead-based immunocapture comprising conjugating a bead with a fluorescent labelled antibody and detecting a fluorescence signal upon binding of a ligand. Similar binding detection methodologies utilize the surface plasmon resonance (SPR) effect to measure and detect antibody-antigen interactions.

Detection labels such as fluorescent dyes and chemiluminescent dyes (Briggs et al (1997) "Synthesis of Functionalised Fluorescent Dyes and Their Coupling to Amines and Amino Acids," J. Chem. Soc., Perkin-Trans. 1:1051-1058) provide a detectable signal and are generally applicable for labelling antibodies, preferably with the following properties: (i) the labelled antibody should produce a very high signal with low background so that small quantities of antibodies can be sensitively detected in both cell-free and cell-based assays; and (ii) the labelled antibody should be photostable so that the fluorescent signal may be observed, monitored and recorded without significant photo bleaching. For applications involving cell surface binding of labelled antibody to membranes or cell surfaces, especially live cells, the labels preferably (iii) have good water-solubility to achieve effective conjugate concentration and detection sensitivity and (iv) are non-toxic to living cells so as not to disrupt the normal metabolic processes of the cells or cause premature cell death.

Direct quantification of cellular fluorescence intensity and enumeration of fluorescently labelled events, e.g. cell surface binding of peptide-dye conjugates may be conducted on an system (FMAT® 8100 HTS System, Applied Biosystems, Foster City, Calif.) that automates mix-and-read, non-radioactive assays with live cells or beads (Miraglia, "Homogeneous cell- and bead-based assays for high throughput screening using fluorometric microvolume assay technology", (1999) J. of Biomolecular Screening 4:193-204). Uses of labelled antibodies also include cell surface receptor binding assays, inmmunocapture assays, fluorescence linked immunosorbent assays (FLISA), caspase-cleavage (Zheng, "Caspase-3 controls both cytoplasmic and nuclear events associated with Fas-mediated apoptosis in vivo", (1998) Proc. Natl. Acad. Sci. USA 95:618-23; US 6372907), apoptosis (Vermes, "A novel assay for apoptosis. Flow cytometric detection of phosphatidylserine expression on early apoptotic cells using fluorescein labelled Annexin V" (1995) J. Immunol. Methods 184:39-51) and cytotoxicity assays. Fluorometric microvolume assay technology can be used to identify the up or down regulation by a molecule that is targeted to the cell surface (Swartzman, "A homogeneous and multiplexed immunoassay for high-throughput screening using fluorometric microvolume assay technology", (1999) Anal. Biochem. 271:143-51).

Labelled cysteine engineered antibodies of the disclosure are useful as imaging biomarkers and probes by the various methods and techniques of biomedical and molecular imaging such as: (i) MRI (magnetic resonance imaging); (ii) MicroCT (computerized tomography); (iii) SPECT (single photon emission computed tomography); (iv) PET (positron emission tomography) Chen et al (2004) Bioconjugate Chem. 15:41-49; (v) bioluminescence; (vi) fluorescence; and (vii) ultrasound. Immunoscintigraphy is an imaging procedure in which antibodies labeled with radioactive substances are administered to an animal or human patient and a picture is taken of sites in the body where the antibody localizes (US 6528624). Imaging biomarkers may be objectively measured and evaluated as an indicator of normal biological processes, pathogenic processes, or pharmacological responses to a therapeutic intervention. Biomarkers may be of several types: Type 0 are natural history markers of a disease and correlate longitudinally with known clinical indices, e.g. MRI assessment of synovial inflammation in rheumatoid arthritis; Type I markers capture the effect of an intervention in accordance with a mechanism-of-action, even though the mechanism may not be associated with clinical outcome; Type II markers function as surrogate endpoints where the change in, or signal from, the biomarker predicts a clinical benefit to "validate" the targeted response, such as measured bone erosion in rheumatoid arthritis by CT. Imaging biomarkers thus can provide pharmacodynamic (PD) therapeutic information about: (i) expression of a target protein, (ii) binding of a therapeutic to the target protein, i.e. selectivity, and (iii) clearance and half-life pharmacokinetic data. Advantages of *in vivo* imaging biomarkers relative to lab-based biomarkers include: non-invasive treatment, quantifiable, whole body assessment, repetitive dosing and assessment, i.e. multiple time points, and potentially transferable effects from preclinical (small animal) to clinical (human) results. For some applications, bioimaging supplants or minimizes the number of animal experiments in preclinical studies.

Radionuclide imaging labels include radionuclides such as ³H, ¹¹C, ¹⁴C, ¹⁸F, ³²P, ³⁵S, ⁶⁴Cu, ⁶⁸Ga, ⁸⁶Y, ⁸⁹Zr, ⁹⁹Tc, ¹¹¹In, ¹²³I, ¹²⁴I, ¹²⁵I, ¹³¹I, ¹³³Xe, ¹⁷⁷Lu, ²¹¹At, or ²¹³Bi. The radionuclide metal ion can be complexed with a chelating linker such as DOTA. Linker reagents such as DOTA-maleimide (4-maleimidobutyramidobenzyl-DOTA) can be prepared by the reaction of aminobenzyl-DOTA with 4-maleimidobutyric acid (Fluka) activated with isopropylchloroformate (Aldrich), following the procedure of Axworthy et al (2000) Proc. Natl. Acad. Sci. USA 97(4):1802-1807). DOTA-maleimide reagents react with the free cysteine amino acids of the cysteine engineered antibodies and provide a metal complexing ligand on the antibody (Lewis et al (1998) Bioconj. Chem. 9:72-86). Chelating linker labelling reagents such as DOTA-NHS (1,4,7,10-tetraazacyclododecane-1,4,7,10-tetraacetic acid mono (N-hydroxysuccinimide ester) are commercially available (Macrocyclics, Dallas, TX). Receptor target imaging with radionuclide labelled antibodies can provide a marker of pathway activation by detection and quantitation of progressive accumulation of antibodies in tumor tissue (Albert et al (1998) Bioorg. Med. Chem. Lett. 8:1207-1210). The conjugated radio-metals may remain intracellular following lysosomal degradation.

Peptide labelling methods are well known. See Haugland, 2003, Molecular Probes Handbook of Fluorescent Probes and Research Chemicals, Molecular Probes, Inc.; Brinkley, 1992, Bioconjugate Chem. 3:2; Garman, (1997) Non-Radioactive Labelling: A Practical Approach, Academic Press, London; Means (1990) Bioconjugate Chem. 1:2; Glazer et al (1975) Chemical Modification of Proteins. Laboratory Techniques in Biochemistry and Molecular Biology (T. S. Work and E. Work, Eds.) American Elsevier Publishing Co., New York; Lundblad, R. L. and Noyes, C. M. (1984) Chemical Reagents for Protein Modification, Vols. I and II, CRC Press, New York; Pfleiderer, G. (1985) "Chemical Modification of Proteins", Modern Methods in Protein Chemistry, H. Tschesche, Ed., Walter DeGryter, Berlin and New York; and Wong (1991) Chemistry of Protein Conjugation and Crosslinking, CRC Press, Boca Raton, Fla.); De Leon-Rodriguez et al (2004) Chem.Eur. J. 10:1149-1155; Lewis et al (2001) Bioconjugate Chem. 12:320-324; Li et al (2002) Bioconjugate Chem. 13:110-115; Mier et al (2005) Bioconjugate Chem. 16:240-237.

Peptides and proteins labelled with two moieties, a fluorescent reporter and quencher in sufficient proximity undergo fluorescence resonance energy transfer (FRET). Reporter groups are typically fluorescent dyes that are excited by light at a certain wavelength and transfer energy to an acceptor, or quencher, group, with the appropriate Stokes shift for emission at maximal brightness. Fluorescent dyes include molecules with extended aromaticity, such as fluorescein and rhodamine, and their derivatives. The fluorescent reporter may be partially or significantly quenched by the quencher moiety in an intact peptide. Upon cleavage of the peptide by a peptidase or protease, a detectable increase in fluorescence may be measured (Knight, C. (1995) "Fluorimetric Assays of Proteolytic Enzymes", Methods in Enzymology, Academic Press, 248:18-34).

The labelled antibodies of the disclosure may also be used as an affinity purification agent. In this process, the labelled antibody is immobilized on a solid phase such a Sephadex resin or filter paper, using methods well known in the art. The immobilized antibody is contacted with a sample containing the antigen to be purified, and thereafter the support is washed with a suitable solvent that will remove substantially all the material in the sample except the antigen to be purified, which is bound to the immobilized polypeptide variant. Finally, the support is washed with another suitable solvent, such as glycine buffer, pH 5.0, that will release the antigen from the polypeptide variant.

Labelling reagents typically bear reactive functionality which may react (i) directly with a cysteine thiol of a cysteine engineered antibody to form the labelled antibody, (ii) with a linker reagent to form a linker-label intermediate, or (iii) with a linker antibody to form the labelled antibody. Reactive functionality of labelling reagents include: maleimide, haloacetyl, iodoacetamide succinimidyl ester (e.g. NHS, N-hydroxysuccinimide), isothiocyanate, sulfonyl chloride, 2,6-dichlorotriazinyl, pentafluorophenyl ester, and phosphoramidite, although other functional groups can also be used.

An exemplary reactive functional group is N-hydroxysuccinimidyl ester (NHS) of a carboxyl group substituent of a detectable label, e.g. biotin or a fluorescent dye. The NHS ester of the label may be preformed, isolated, purified, and/or characterized, or it may be formed in situ and reacted with a nucleophilic group of an antibody. Typically, the carboxyl form of the label is activated by reacting with some combination of a carbodiimide reagent, e.g. dicyclohexylcarbodiimide, diisopropylcarbodiimide, or a uronium reagent, e.g. TSTU (O-(N-Succinimidyl)-N,N,N',N'-tetramethyluronium tetrafluoroborate, HBTU (O-benzotriazol-1-yl)-N,N,N',N'-tetramethyluronium hexafluorophosphate), or HATU (O-(7-azabenzotriazol-1-yl)-N,N,N',N'-tetramethyluronium hexafluorophosphate), an activator, such as 1-hydroxybenzotriazole (HOBt), and N-hydroxysuccinimide to give the NHS ester of the label. In some cases, the label and the antibody may be coupled by *in situ* activation of the label and reaction with the antibody to form the label-antibody conjugate in one step. Other activating and coupling reagents include TBTU (2-(1H-benzotriazo-1-yl)-1-1-3,3-tetramethyluronium hexafluorophosphate), TFFH (N,N',N'',N'''-tetramethyluronium 2-fluoro-hexafluorophosphate), PyBOP (benzotriazole- 1-yl-oxy-tris-pyrrolidino-phosphonium hexafluorophosphate, EEDQ (2-ethoxy-1-ethoxycarbonyl-1,2-dihydro-quinoline), DCC (dicyclohexylcarbodiimide); DIPCDI (diisopropylcarbodiimide), MSNT (1-(mesitylene-2-sulfonyl)-3-nitro-1H-1,2,4-triazole, and aryl sulfonyl halides, e.g. triisopropylbenzenesulfonyl chloride.

### CONJUGATION OF BIOTIN-MALEIMIDE TO THIOFABS

The above-described ThioFab properties were established in the presence of phage because fusion of the Fab to the phage coat protein could potentially alter Cys thiol accessibility or reactivity. Therefore, the ThioFab constructs were cloned into an expression vector under alkaline phosphatase promoter (Chang et al (1987) Gene 55:189-196) and the ThioFab expression was induced by growing *E. coli* cells in the phosphate-free medium. ThioFabs were purified on a Protein G SEPHAROSE™ column and analyzed on reducing and non-reducing SDS-PAGE gels. These analyses allow assessment of whether ThioFabs retained their reactive thiol group or were rendered inactive by forming intramolecular or intermolecular disulfide bonds. ThioFabs L-V15C, L-V110C, H-A88C, and H-A121C were expressed and purified by Protein-G SEPHAROSE™ column chromatography (see methods sections for details). Purified proteins were analyzed on SDS-PAGE gel in reducing (with DTT) and non-reducing (without DTT) conditions. Other reducing agents such as BME (beta-mercaptoethanol) can used in the gel to cleave interchain disulfide groups. It is evident from SDS-PAGE gel analysis that the major (-90%) fraction of ThioFab is in the monomeric form, while wild type hu4D5Fabv8 is essentially in the monomeric form (47 kDa).

ThioFab (A121C) and wild type hu4D5Fabv8 were incubated with 100 fold excess of biotin-maleimide for 3 hours at room temperature and the biotinylated Fabs were loaded onto a Superdex-200™ gel filtration column. This purification step was useful in separating monomeric Fab from oligomeric Fab and also from excess free biotin-maleimide (or free zirconium reagent).

Figure 5 shows validation of the properties of ThioFab variants in the absence of the phage context. The proteins without phage fusion, hu4D5Fabv8 and hu4D5Fabv8-A121C (ThioFab-A121C), were expressed and purified using protein-G agarose beads followed by incubation with 100 fold molar excess of biotin-maleimide. Streptavidin and HER2 binding of a biotinylated cys engineered ThioFab and a non-biotinylated wild type Fab was compared. The extent of biotin conjugation (interaction with streptavidin) and their binding ability to HER2 were monitored by ELISA analyses. Each Fab was tested at 2ng and 20ng.

Biotinylated A121C ThioFab retained comparable HER2 binding to that of wild type hu4D5Fabv8 (Figure 5). Wild type Fab and A121C-ThioFab were purified by gel filtration column chromatography. The two samples were tested for HER2 and streptavidin binding by ELISA using goat anti-Fab-HRP as secondary antibody. Both wild type (open box) and ThioFab (dotted box) have similar binding to HER2 but only ThioFab retained streptavidin binding. Only a background level of interaction with streptavidin was observed with non-biotinylated wild type hu4D5Fabv8 (Figure 5). Mass spectral (LC-ESI-MS) analysis of biotinylated-ThioFab (A121C) resulted in a major peak with 48294.5 daltons compared to the wild type hu4D5Fabv8 (47737 daltons). The 537.5 daltons difference between the two molecules exactly corresponds to a single biotin-maleimide conjugated to the ThioFab. Mass spec protein sequencing (LC-ESI-Tandem mass spec analysis) results further confirmed that the conjugated biotin molecule was at the newly engineered Cys residue (Table 4, Example 3).

### SITE SPECIFIC CONJUGATION OF BIOTIN-MALEIMIDE TO ALBUMIN BINDING PEPTIDE (ABP)-THIOFABS

Plasma-protein binding can be an effective means of improving the pharmacokinetic properties of short lived molecules. Albumin is the most abundant protein in plasma. Serum albumin binding peptides (ABP) can alter the pharmacodynamics of fused active domain proteins, including alteration of tissue uptake, penetration, and diffusion. These pharmacodynamic parameters can be modulated by specific selection of the appropriate serum albumin binding peptide sequence (US 20040001827). A series of albumin binding peptides were identified by phage display screening (Dennis et al. (2002) "Albumin Binding As A General Strategy For Improving The Pharmacokinetics Of Proteins" J Biol Chem. 277:35035-35043; WO 01/45746). Compounds of the disclosure include ABP sequences taught by: (i) Dennis et al (2002) J Biol Chem. 277:35035-35043 at Tables III and IV, page 35038; (ii) US 20040001827 at [0076] SEQ ID NOS: 9-22; and (iii) WO 01/45746 at pages 12-13, SEQ ID NOS: z1-z14.

Albumin Binding (ABP)-Fabs were engineered by fusing an albumin binding peptide to the C-terminus of Fab heavy chain in 1:1 stoichiometric ratio (1 ABP / 1 Fab). It was shown that association of these ABP-Fabs with albumin increased their half life by more than 25 fold in rabbits and mice. The above described reactive Cys residues can therefore be introduced in these ABP-Fabs and used for site-specific conjugation with zirconium reagents followed by *in vivo* animal studies.

Exemplary albumin binding peptide sequences include, but are not limited to the amino acid sequences listed in SEQ ID NOS: 1-5:
CDKTHTGGGSQRLMEDICLPRWGCLWEDDF SEQ ID NO:1
QRLMEDICLPRWGCLWEDDF SEQ ID NO:2
QRLIEDICLPRWGCLWEDDF SEQ ID NO:3
RLIEDICLPRWGCLWEDD SEQ ID NO:4
DICLPRWGCLW SEQ ID NO:5

The albumin binding peptide (ABP) sequences bind albumin from multiple species (mouse, rat, rabbit, bovine, rhesus, baboon, and human) with Kd (rabbit) = 0.3 µM. The albumin binding peptide does not compete with ligands known to bind albumin and has a half life (T½) in rabbit of 2.3 hr. ABP-ThioFab proteins were purified on BSA-SEPHAROSE™ followed by biotin-maleimide conjugation and purification on Superdex-S200 column chromatography as described in previous sections. Purified biotinylated proteins were homogeneous and devoid of any oligomeric forms (Example 4).

Figure 6 shows the properties of Albumin Binding Peptide (ABP)-ThioFab variants. ELISA analyses were carried out to test the binding ability of ABP-hu4D5Fabv8-wt, ABP-hu4D5Fabv8-V110C and ABP-hu4D5Fabv8-A121C with rabbit albumin, streptavidin and HER2. Biotinylated ABP-ThioFabs are capable of binding to albumin and HER2 with similar affinity to that of wild type ABP-hu4D5Fabv8 as confirmed by ELISA (Figure 6) and BIAcore binding kinetics analysis (Table 3). An ELISA plate was coated with albumin, HER2 and SA as described. Binding of biotinylated ABP-ThioFabs to albumin, HER2 and SA was probed with anti-Fab HRP. Biotinylated ABP-ThioFabs were capable of binding to streptavidin compared to non biotinylated control ABP-hu4D5Fabv8-wt indicating that ABP-ThioFabs were conjugated with biotin maleimide like ThioFabs in a site specific manner as the same Cys mutants were used for both the variants (Figure 6).

**Table 3. BIAcore kinetic analysis for HER2 and rabbit albumin binding to biotinylated ABP- hu4D5Fabv8 wild type and ThioFabs**

| Antibody | kₒₙ (M⁻¹s⁻¹) | k_{off} (S⁻¹) | K_{d} (nM) |
|---|---|---|---|
| HER2 binding | | | |
| wild type | 4.57 x 10⁵ | 4.19 x 10⁻⁵ | 0.0917 |
| V110C | 4.18 x 10⁵ | 4.05 x 10⁻⁵ | 0.097 |
| A121C | 3.91 x 10⁵ | 4.15 x 10⁻⁵ | 0.106 |
| Rabbit albumin binding | | | |
| wild type | 1.66 x 10⁵ | 0.0206 | 124 |
| V110C | 2.43 x 10⁵ | 0.0331 | 136 |
| A121C | 1.70 x 10⁵ | 0.0238 | 140 |

| | | | |
|---|---|---|---|
| ABP = albumin binding peptide | | | |

Alternatively, an albumin-binding peptide may be linked to the antibody by covalent attachment through a linker moiety.

### ENGINEERING OF ABP-THIOFABS WITH TWO FREE THIOL GROUPS PER FAB

The above results indicate that all four (L-V15C, L-V110C, H-A88C and H-A121C) thioFab (cysteine engineered Fab antibodies) variants have reactive thiol groups that can be used for site specific conjugation with a label reagent, linker reagent, or zirconium-linker intermediate. L-V15C can be expressed and purified but with relatively low yields. However the expression and purification yields of L-V110C, H-A88C and H-A121C variants were similar to that of hu4D5Fabv8. Therefore these mutants can be used for further analysis and recombined to get more than one thiol group per Fab. Towards this objective, one thiol group on the light and one on heavy chain were constructed to obtain two thiol groups per Fab molecule (L-V110C/H-A88C and L-V110C/H-A121C). These two double Cys variants were expressed in an *E. coli* expression system and purified. The homogeneity of purified biotinylated ABP-ThioFabs was found to be similar to that of single Cys variants.

The effects of engineering two reactive Cys residues per Fab was investigated (Figure 7). The presence of a second biotin was tested by probing the binding of biotinylated ABP-ThioFab to SA using streptavidin-HRP (Figure 7). For HER2/Fab analysis, an ELISA plate was coated with HER2 and probed with anti-Fab HRP. For SA/Fab analysis, an ELISA plate was coated with SA and probed with anti-Fab HRP. For SA/SA analysis, an ELISA plate was coated with SA and probed with SA-HRP. Figure 7. ELISA analyses for the interaction of biotinylated ABP-hu4D5Fabv8 cys variants with HER2, streptavidin (SA). HER2/Fab, SA/Fab and SA/SA indicate that their interactions were monitored by anti-Fab-HRP, SA-HRP, respectively. SA/Fab monitors the presence of single biotin per Fab and more than one biotin per Fab is monitored by SA/SA analysis. Binding of HER2 with double cys mutants is similar to that of single Cys variants (Figure 7). However the extent of biotinylation on double Cys mutants was higher compared to single Cys variants due to more than one free thiol group per Fab molecule (Figure 7).

### ENGINEERING OF THIO IgG VARIANTS OF TRASTUZUMAB

Cysteine was introduced into the full-length monoclonal antibody, trastuzumab (HERCEPTIN®, Genentech Inc.) at certain residues. The single cys mutants H-A88C, HA121C and L-V110C of trastuzumab, and double cys mutants V110C-A121C and V110C-A121C of trastuzumab were expressed in CHO (Chinese Hamster Ovary) cells by transient fermentation in media containing 1 mM cysteine. The A88C mutant heavy chain sequence (450 aa) is SEQ ID NO:6. The A121C mutant heavy chain sequence (450 aa) is SEQ ID NO:7. The V110C mutant light chain sequence (214 aa) is SEQ ID NO:8.

According to one embodiment, the cysteine engineered thio-trastuzumab antibodies comprise one or more of the following variable region heavy chain sequences with a free cysteine amino acid (SEQ ID NOS: 9-16).

| Mutant | Sequence | SEQ ID NO: |
|---|---|---|
| A40C | WVRQCPGKGL | SEQ ID NO:9 |
| A88C | NSLRCEDTAV | SEQ ID NO:10 |
| S119C | LVTVCSASTKGPS | SEQ ID NO:11 |
| S120C | LVTVSCASTKGPS | SEQ ID NO:12 |
| A121C | LVTVSSCSTKGPS | SEQ ID NO:13 |
| S122C | LVTVSSACTKGPS | SEQ ID NO:14 |
| A175C | HTFPCVLQSSGLYS | SEQ ID NO:15 |
| S179C | HTFPAVLQCSGLYS | SEQ ID NO:16 |

According to another embodiment, the cysteine engineered thio-trastuzumab antibodies comprise one or more of the following variable region light chain sequences with a free cysteine amino acid (SEQ ID NOS: 17-27).

| Mutant | Sequence | SEQ ID NO: |
|---|---|---|
| V15C | SLSASCGDRVT | SEQ ID NO:17 |
| A43C | QKPGKCPKLLI | SEQ ID NO:18 |
| V110C | EIKRTCAAPSV | SEQ ID NO:19 |
| S114C | TCAAPCVFIFPP | SEQ ID NO:20 |
| S121C | FIFPPCDEQLK | SEQ ID NO:21 |
| S127C | DEQLKCGTASV | SEQ ID NO:22 |
| A144C | FYPRECKVQWK | SEQ ID NO:23 |
| A153C | WKVDNCLQSGN | SEQ ID NO:24 |
| N158C | ALQSGCSQESV | SEQ ID NO:25 |
| S168C | VTEQDCKDSTY | SEQ ID NO:26 |
| V205C | GLSSPCTKSFN | SEQ ID NO:27 |

The resulting full-length, thio-trastuzumab IgG variants were assayed for thiol reactivity and HER2 binding activity. Figure 13A shows a cartoon depiction of biotinylated antibody binding to immobilized HER2 and HRP labeled secondary antibody for absorbance detection. Figure 13B shows binding measurements to immobilized HER2 with detection of absorbance at 450 nm of (left to right): non-biotinylated wild type trastuzumab (Wt), biotin-maleimide conjugated thio-trastuzumab variants V110C (single cys), A121C (single cys), and V110C-A121C (double cys). Each thio IgG variant and trastuzumab was tested at 1, 10, and 100 ng. The measurements show that biotinylated anti-HER2 ThioMabs retain HER2 binding activity.

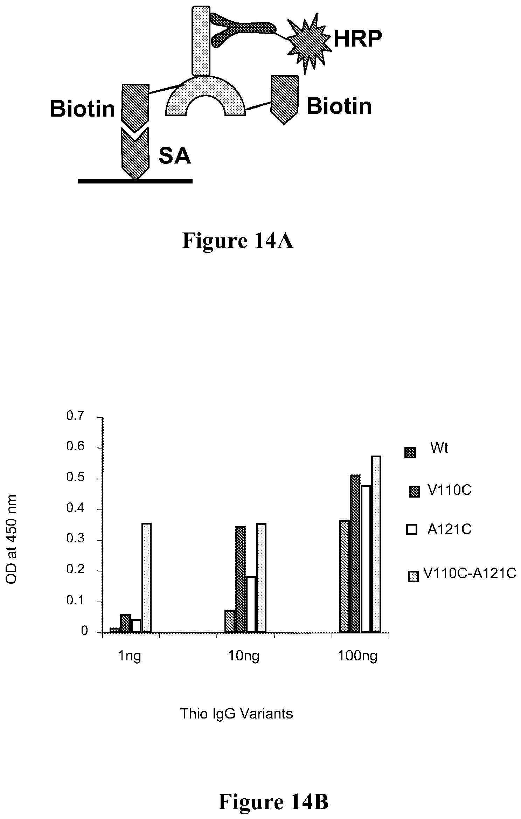

Figure 14A shows a cartoon depiction of a biotinylated antibody binding to immobilized HER2 with binding of biotin to anti-IgG-HRP for absorbance detection. Figure 14B shows binding measurements with detection of absorbance at 450 nm of biotin-maleimide conjugated thio-trastuzumab variants and non-biotinylated wild type trastuzumab in binding to streptavidin. From left to right: V110C (single cys), A121C (single cys), V110C/A121C (double cys), and trastuzumab. Each thio IgG trastuzumab variant and parent trastuzumab was tested at 1, 10, and 100 ng. The measurements show that the HER2 ThioMabs have high thiol reactivity.

Cysteine was introduced into the full-length 2H9 anti-EphB2R antibody at certain residues. The single cys mutant H-A121C of 2H9 was expressed in CHO (Chinese Hamster Ovary) cells by transient fermentation in media containing 1 mM cysteine. The A121C 2H9 mutant heavy chain sequence (450 aa) is SEQ ID NO:28.

Cysteine engineered thio-2H9 antibodies comprise the following Fc constant region heavy chain sequences with a free cysteine amino acid (SEQ ID NOS: 29-38).

| Mutant | Sequence | SEQ ID NO: |
|---|---|---|
| V273C | HEDPECKFNWYVDGVEVHNAKTKPR | SEQ ID NO:29 |
| V279C | HEDPEVKFNWYCDGVEVHNAKTKPR | SEQ ID NO:30 |
| V282C | HEDPEVKFNWYVDGCEVHNAKTKPR | SEQ ID NO:31 |
| V284C | HEDPEVKFNWYVDGVECHNAKTKPR | SEQ ID NO:32 |
| A287C | HEDPEVKFNWYVDGVEVHNCKTKPR | SEQ ID NO:33 |
| S324C | YKCKVCNKALP | SEQ ID NO:34 |
| S337C | IEKTICKAKGQPR | SEQ ID NO:35 |
| A339C | IEKTISKCKGQPR | SEQ ID NO:36 |
| S375C | KGFYPCDIAVE | SEQ ID NO:37 |
| S400C | PPVLDCDGSFF | SEQ ID NO:38 |

Figure 16 shows non-reducing (top) and reducing (bottom) denaturing SDS-PAGE (polyacrylamide gel electrophoresis) analysis of 2H9 ThioMab Fc variants (left to right, lanes 1-9): A339C; S337C; S324C; A287C; V284C; V282C; V279C; and V273C, with 2H9 wild type, after purification on immobilized Protein A. The lane on the right is a size marker ladder, indicating the intact proteins are about 150 kDa, heavy chain fragments about 50 kDa, and light chain fragments about 25 kDa. Figure 17A shows non-reducing (left) and reducing (right) denaturing polyacrylamide gel electrophoresis analysis of 2H9 ThioMab variants (left to right, lanes 1-4): L-V15C; S179C; S375C; S400C, after purification on immobilized Protein A. Figure 17B shows non-reducing (left) and reducing (+DTT) (right) denaturing polyacrylamide gel electrophoresis analysis of additional 2H9 and 3A5 ThioMab variants after purification on immobilized Protein A. The 2H9 ThioMab variants (in the Fab as well as Fc region) were expressed and purified as described. As seen in Figures 16, 17A and 17B, all the proteins are homogenous on SDS-PAGE followed by the reduction and oxidation procedure of Example 11 to prepare reactive ThioMabs for conjugation (Example 12).

Cysteine was introduced into the full-length 3A5 anti-MUC16 antibody at certain residues. The single cys mutant H-A121C of 3A5 was expressed in CHO (Chinese Hamster Ovary) cells by transient fermentation in media containing 1 mM cysteine. The A121C 3A5 mutant heavy chain sequence (446 aa) comprises SEQ ID NO:39.

Cysteine engineered thio-3A5 anti-MUC16 antibodies comprise the following variable region heavy chain sequences with a free cysteine amino acid (SEQ ID NOS: 40-44).

| Mutant | Sequence | SEQ ID NO: |
|---|---|---|
| F45C | NWIRQCPGNK | SEQ ID NO:40 |
| A90C | LNSCTTEDTAT | SEQ ID NO:41 |
| A121C | GQGTLVTVSACSTKGPSVFPL | SEQ ID NO:42 |
| A175C | HTFPCVLQSSGLYS | SEQ ID NO:43 |
| V176C | HTFPACLQSSGLYS | SEQ ID NO:44 |

Cysteine engineered thio-3A5 anti-MUC16 antibodies comprise the following variable region light chain sequences with a free cysteine amino acid (SEQ ID NOS: 45-49).

| Mutant | Sequence | SEQ ID NO: |
|---|---|---|
| L15C | FLSVSCGGRVT | SEQ ID NO:45 |
| A43C | QKPGNCPRLLI | SEQ ID NO:46 |
| V110C | EIKRTCAAPSV | SEQ ID NO:47 |
| A144C | FYPRECKVQWK | SEQ ID NO:48 |
| S168C | VTEQDCKDSTY | SEQ ID NO:49 |

### THIOL REACTIVITY OF THIOMABS

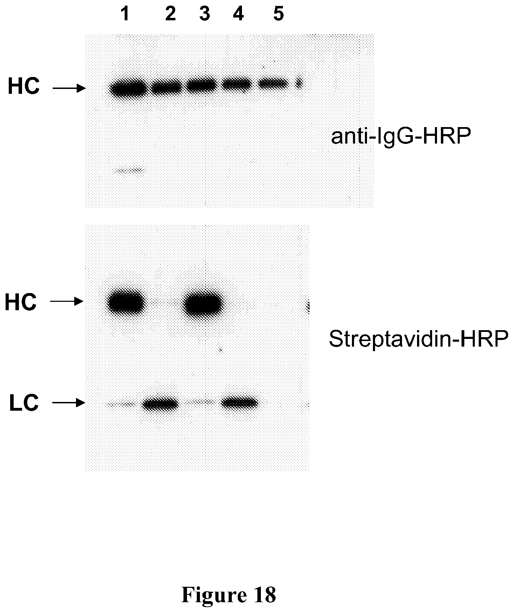

The thiol reactivity of full length, IgG cysteine engineered antibodies (ThioMabs) was measured by biotinylation and streptavidin binding. A western blot assay was set up to screen the ThioMab that is specifically conjugated with biotin-maleimide. In this assay, the antibodies are analyzed on reducing SDS-PAGE and the presence of Biotin is specifically probed by incubating with streptavidin-HRP. As seen from figure 18, the streptavidin-HRP interaction is either observed in heavy chain or light chain depending on which engineered cys variant is being used and no interaction is seen with wild type, indicating that ThioMab variants specifically conjugated the biotin at engineered Cys residue. Figure 18 shows denaturing gel analysis of reduced, biotinylated Thio-IgG variants after capture on immobilized anti-IgG-HRP (top gel) and streptavidin-HRP (bottom gel). Lane 1: 3A5 HA121C. Lane 2: 3A5 L-V110C. Lane 3: 2H9 H-A121C. Lane 4: 2H9 L-V110C. Lane 5: anti-EphB2R 2H9 parent, wild type. Each mutant (lanes 1-4) was captured by anti-IgG with HRP detection (top) indicating that selectivity and affinity were retained. Capture by immobilized streptavidin with HRP detection (bottom) confirmed the location of biotin on heavy and light chains. The location of cysteine mutation on the cysteine engineered antibodies in lanes 1 and 3 is the heavy chain. The location of cysteine mutation on the cysteine engineered antibodies in lanes 2 and 4 is the light chain. The cysteine mutation site undergoes conjugation with the biotin-maleimide reagent.

Analysis of the ThioMab cysteine engineered antibodies of Figure 18 and a 2H9 V15C variant by LC/MS gave quantitative indication of thiol reactivity (Table 5).

**Table 5 LC/MS quantitation of biotinylation of ThioMabs - Thiol reactivity**

| ThioMab variant | number of biotin per ThioMab |
|---|---|
| 2H9 wt | 0.0 |
| 2H9 L-V15C | 0.6 |
| 2H9 L-V110C | 0.5 |
| 2H9 H-A121C | 2.0 |
| 3A5 L-V110C | 1.0 |
| 3A5 H-A121C | 2.0 |

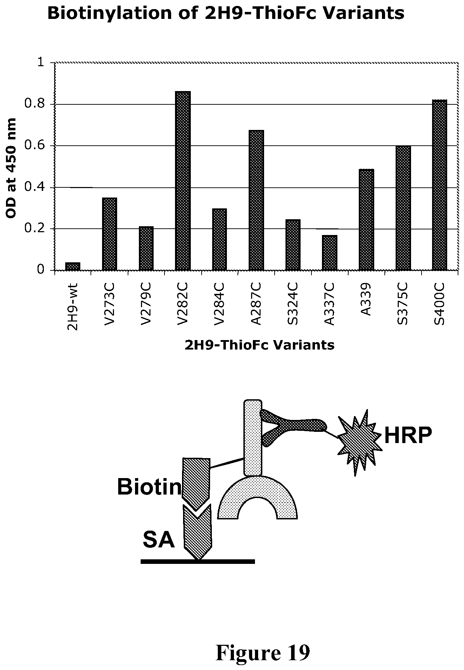

Cysteine engineering was conducted in the constant domain, i.e. Fc region, of IgG antibodies. A variety of amino acid sites were converted to cysteine sites and the expressed mutants, i.e. cysteine engineered antibodies, were assessed for their thiol reactivity. Biotinylated 2H9 ThioMab Fc variants were assessed for thiol reactivity by HRP quantitation by capture on immobilized streptavidin in an ELISA assay (Figure 19). An ELISA assay was established to rapidly screen the Cys residues with reactive Thiol groups. As depicted in Figure 19 schematic diagram, the streptavidin-biotin interaction is monitored by probing with anti-IgG-HRP followed by measuring absorbance at 450 nm. These results confirmed 2H9-ThioFc variants V282C, A287C, A339C, S375C and S400C had moderate to highest Thiol reactivity. The extent of biotin conjugation of 2H9 ThioMab Fc variants was quantitated by LS/MS analysis as reported in Table 6. The LS/MS analysis confirmed that the A282C, S375C and S400C variants had 100% biotin conjugation and V284C and A339C had 50% conjugation, indicating the presence of a reactive cysteine thiol group. The other ThioFc variants, and the parent, wild type 2H9, had either very little biotinylation or none.

**Table 6 LC/MS quantitation of biotinylation of 2H9 Fc ThioMabs**

| 2H9 ThioMab Fc variant | % biotinylation |
|---|---|
| V273C | 0 |
| V279C | 31 |
| V282C | 100 |
| V284C | 50 |
| A287C | 0 |
| S324C | 71 |
| S337C | 0 |
| A339C | 54 |
| S375C | 100 |
| S400C | 100 |
| (wild type 2H9) | 0 |

### THIOL REACTIVITY OF THIO-4D5 FAB LIGHT CHAIN VARIANTS

Screening of a variety of cysteine engineered light chain variant Fabs of the antiErbB2 antibody 4D5 gave a number of variants with a thiol reactivity value of 0.6 and higher (Table 7), as measured by the PHESELECTOR assay of Figure 8. The thiol reactivity values of Table 7 are normalized to the heavy chain 4D5 ThioFab variant (HC-A121C) which is set at 100%, assuming complete biotinylation of HC-A121C variant, and represented as per cent values.

**Table 7 Thiol reactivity per cent values of 4D5 ThioFab light chain variants**

| 4D5 ThioFab variant | Thiol reactivity value (%) |
|---|---|
| V15C | 100 |
| V110C | 95 |
| S114C | 78 |
| S121C | 75 |
| S127C | 75 |
| A153C | 82 |
| N158C | 77 |
| V205C | 78 |
| (HC-A121C) | 100 |
| (4D5 wild type) | 25 |

### ZIRCONIUM-LABELLING REAGENTS

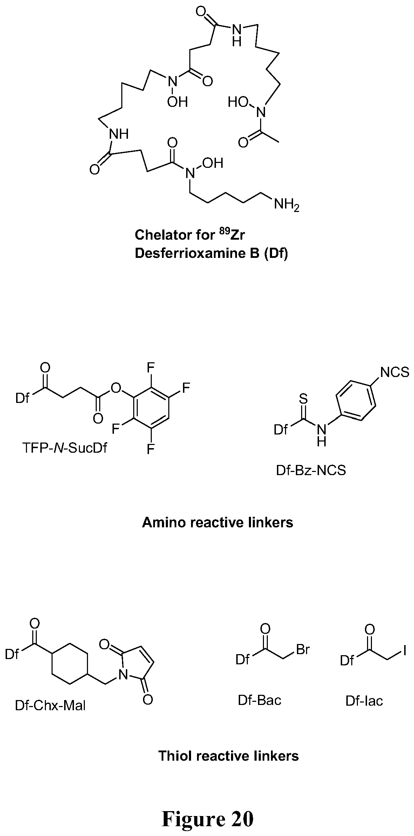

Exemplary bifunctional reagents based on desferrioxamine B (Df) are employed for the complexation of ⁸⁹Zr to antibodies, including monoclonal antibodies (mAbs). Desferrioxamine B (*N'*-{5-[acetyl(hydroxy)amino]pentyl}-*N*-[5-({4-[(5-aminopentyl)(hydroxy)amino] -4-oxobutanoyl} amino)pentyl] -N-hydroxysuccinamide (CAS Reg. No. 70-51-9); and also known as Deferoxamine, desferoxamine B, DFO-B, DFOA, DFB or desferal) is a bacterial siderophore produced by the actinobacter Streptomyces pilosus (Figure 20 top). Desferrioxamine B has medical applications as a chelating agent used to remove excess iron from the body (Miller, Marvin J. "Syntheses and therapeutic potential of hydroxamic acid based siderophores and analogs" (1989) Chemical Reviews 89 (7): 1563-1579). The mesylate salt of DFO-B is commercially available. Initial experiments were conducted with *N*-(*S*-acetyl)thioacetyl-Df (SATA-Df) and mAb decorated with maleimide groups, 4-[*N*-maleimidomethyl]cyclohexane-1-carboxylate (mAb-SMCC) attached to ε-amino group in lysine side chain (Meijs WE et al. "Zirconium-labeled monoclonal antibodies and their distribution in tumor-bearing nude mice" (1997) J. Nucl. Med. 38:112-8; Meijs WE et al. "A facile method for the labeling of proteins with zirconium isotopes" (1996) Nucl Med Biol. 23:439-48). However, the resulting thioether conjugates (mAb-SMCC-SATA-Df) were unstable in human serum at 37 °C (Verel I et al "89Zr immuno-PET: comprehensive procedures for the production of 89Zr-labeled monoclonal antibodies" (2003) J Nucl Med 44:1271-81). Another exemplary amino reactive bifunctional chelators, based on Df modified with succinic anhydride (Suc), was used to convert the amino group of Df to carboxylic acid and subsequently activated as 2,3,5,6-tetrafluorophenyl ester (TFP). TFP-N-Suc-Df (Figure 20 center) was coupled to lysine ε-amino groups of mAb and the purified mAb-N-Suc-Df was chelated with ⁸⁹Zr. The resulting ⁸⁹Zr-mAb-N-Suc-Df was stable at physiological conditions and its biodistribution was compared to mAb-SMCC-SATA-Df in mice (Verel I et al "89Zr immuno-PET: comprehensive procedures for the production of 89Zr-labeled monoclonal antibodies" (2003) J Nucl Med. 44:1271-81). However, the preparation of TFP-N-Suc-Df requires protection of hydroxamide groups as Fe(III) complex. The iron is removed by treatment with EDTA prior to chelation with ⁸⁹Zr, but the multistep method is tedious and possesses a danger of incomplete removal of the iron from the desferrioxamine and/or incomplete removal of EDTA from the conjugation buffer which may negatively impact the ⁸⁹Zr-chelation yield. Therefore, a heterobifunctional amino reactive reagent, *p*-isothiocyanatobenzyl-desferrioxaimine (Df-Bz-NCS) was recently developed for incorporation of Df into proteins via thiourea linkage, Figure 20 center (Perk LR et al. "Facile radiolabeling of monoclonal antibodies and other proteins with zirconium-89 or gallium-68 for PET Imaging using p-isothiocyanatobenzyl-desferrioxamine" (2008) Nature Protocols, published online:DOI:10.1038/nprot.2008.22; Perk LR et al. "p-Isothiocyanatobenzyl-desferrioxamine: a new bifunctional chelate for facile radiolabeling of monoclonal antibodies with zirconium-89 for immuno-PET imaging" (2009) European Journal Of Nuclear Medicine And Molecular Imaging). The antibody conjugates prepared using Df-Bz-NCS showed comparable stability and imaging properties to the reference conjugates prepared using TFP-N-Suc-Df. Since reliable methods for coupling of ⁸⁹Zr with antibodies through lysine ε-amino groups were developed the number of reported pre-clinical and clinical immunoPET studies with ⁸⁹Zr labeled antibodies has been rapidly increasing (Verel I, et al. "Long-lived positron emitters zirconium-89 and iodine-124 for scouting of therapeutic radioimmunoconjugates with PET" (2003) Cancer Biother Radiopharm. 18:655-61; Nagengast WB et al. "In vivo VEGF imaging with radiolabeled bevacizumab in a human ovarian tumor xenograft" (2007) J Nucl Med. 48:1313-9; Perk LR, et al. "(89)Zr as a PET surrogate radioisotope for scouting biodistribution of the therapeutic radiometals (90)Y and (177)Lu in tumor-bearing nude mice after coupling to the internalizing antibody cetuximab" (2005) J Nucl Med. 46:1898-906; Perk LR et al. "Quantitative PET imaging of Met-expressing human cancer xenografts with (89)Zr-labelled monoclonal antibody DN30" (2008) European Journal Of Nuclear Medicine And Molecular Imaging 35:1857-67; Perk LR et al. "Preparation and evaluation of (89)Zr-Zevalin for monitoring of (90)Y-Zevalin biodistribution with positron emission tomography" (2006) European Journal Of Nuclear Medicine And Molecular Imaging 33:1337-45; Borjesson PK et al. "Performance of immuno-positron emission tomography with zirconium-89-labeled chimeric monoclonal antibody U36 in the detection of lymph node metastases in head and neck cancer patients" (2006) Clin Cancer Res. 12:2133-40; Aerts HJ et al. "Disparity between in vivo EGFR expression and 89Zr-labeled cetuximab uptake assessed with PET" (2009) J Nucl Med. 50:123-31; Dijkers EC et al. "Development and Characterization of Clinical-Grade 89Zr-Trastuzumab for HER2/neu ImmunoPET Imaging" (2009) J Nucl Med 50(6):974-981).

Embodiments of zirconium complexes also include zirconium-binding (chelating) ligands such as DTPA (CAS Reg. No. 67-43-6), DOPA(1,4,7,10-tetraazacyclododecane-1,4,7,10-tetraacetic acid) (Liu, Shuang (2008) Advanced Drug Delivery Reviews 60(12): 1347-1370), cyclopentadienyl, and allyl groups (Erker, G. (1991) Pure and Applied Chemistry 63(6):797-806; Erker, G. (1990) Jour. of Organometallic Chem. 400(1-2):185-203).

Zirconium complexes (Z) and other radionuclides may be conjugated to antibodies (Ab), including monoclonal antibodies (mAbs) through ε-amino group in lysine side chain or through thiol group of cysteine. Since approximately 40 lysine side chains (Wang L et al "Structural characterization of the maytansinoid-monoclonal antibody immunoconjugate, huN901-DM1, by mass spectrometry" (2005) Protein Sci. 14:2436-46) or 8 cysteines (Hamblett KJ et al. "Effects of drug loading on the antitumor activity of a monoclonal antibody drug conjugate" (2004) Clin Cancer Res. 10:7063-70) are available for conjugation in a mAb, both approaches provide heterogeneity with respect to mAb conjugate ratios and the site of conjugation. The modification of a lysine residue within the binding site may decrease the biological activity of the conjugate (Cai W et al. "PET imaging of colorectal cancer in xenograft-bearing mice by use of an 18F-labeled T84.66 anti-carcinoembryonic antigen diabody" (2007) J Nucl Med. 48:304-10; Shively JE. "18F labeling for immuno-PET: where speed and contrast meet" (2007) J Nucl Med. 48:170-2; Tait JF et al "Improved detection of cell death in vivo with annexin V radiolabeled by site-specific methods" (2006) J Nucl Med. 47:1546-53; Schellenberger EA et al "Optical imaging of apoptosis as a biomarker of tumor response to chemotherapy" (2003) Neoplasia (New York, N.Y) 5:187-92), while the modification of cysteines in the hinge region provides a reduced plasma half-life (Hamblett KJ et al. "Effects of drug loading on the antitumor activity of a monoclonal antibody drug conjugate" (2004) Clin Cancer Res. 10:7063-70). These limitations can be avoided by using mAbs engineered to contain cysteine selectively positioned for the purpose of site-specific conjugation with a biochemical assay, PHESELECTOR (US 7521541; Junutula JR et al. "Rapid identification of reactive cysteine residues for site-specific labeling of antibody-Fabs" J Immunol Methods 2008;332:41-52) for the rapid identification of preferred amino acids in an antibody for mutation to cysteine. The resulting antibody (THIOMAB) is subsequently chemoselectively and site-specifically conjugated to cytotoxic drugs without any loss of binding affinity or detrimental effect on the antibody scaffold stability (Junutula JR et al. "Site-specific conjugation of a cytotoxic drug to an antibody improves the therapeutic index" (2008) Nat Biotechnol. 26:925-32).

From an imaging point of view, a high target affinity and minimal non-specific uptake are required for optimal image quality. Accordingly, site-specifically radiolabeled cysteine-engineered antibodies (THIOMABs) could provide tracers with unaltered binding affinity and scaffold stability which may minimize the non-specific uptake of metabolites outside the target tissue. One aspect of the present disclosure is a method for site-specific radiolabeling of THIOMABs using novel Df-based thiol reactive bifunctional reagents maleimidocyclohexyl-desferrioxamine (Df-Chx-Mal), bromoacetyl-desferrioxamine (Df-Bac) and iodoacetyl-desferrioxamine (Df-Iac) (Figure 20). Exemplary embodiments include where these reagents were site-specifically conjugated to trastuzumab THIOMAB (thio-trastuzumab), chelated with ⁸⁹Zr, and evaluated *in vitro* and *in vivo.*

One metastable isomer of zirconium is ⁸⁹Zr with a half-life of 78.4 hours with decay modes of beta (electron emission), positron (beta plus), and gamma radiation.

The radioisotope or other labels may be incorporated in the conjugate in known ways (Fraker et al (1978) Biochem. Biophys. Res. Commun. 80: 49-57; "Monoclonal Antibodies in Immunoscintigraphy" Chatal, CRC Press 1989). Carbon-14-labeled 1-isothiocyanatobenzyl-3-methyldiethylene triaminepentaacetic acid (MX-DTPA) is an exemplary chelating agent for conjugation of a radionuclide to the antibody (WO 94/11026).

### LINKERS

A "Linker" (L) is a bifunctional or multifunctional moiety which can be used to link one or more zirconium complex moieties (Z) and an antibody unit (Ab) to form antibody-zirconium conjugates (AZC) of Formula I. Antibody-zirconium conjugates (AZC) can be conveniently prepared using a Linker having reactive functionality for binding to zirconium and to the Antibody. A cysteine thiol of a cysteine engineered antibody (Ab) can form a bond with a functional group of a linker reagent, a zirconium label moiety or zirconium-linker intermediate.

In one aspect, a Linker has a reactive site which has an electrophilic group that is reactive to a nucleophilic cysteine present on an antibody. The cysteine thiol of the antibody is reactive with an electrophilic group on a Linker and forms a covalent bond to a Linker. Useful electrophilic groups include, but are not limited to, maleimide and haloacetamide groups.

Cysteine engineered antibodies may react with linker reagents or zirconium-linker intermediates, with electrophilic functional groups such as maleimide or α-halo carbonyl, according to the conjugation method at page 766 of Klussman, et al (2004), Bioconjugate Chemistry 15(4):765-773, and according to the protocols of Examples 17-19.

In another embodiment, the Z moieties are the same.

In yet another embodiment, the Z moieties are different.

Exemplary embodiments of the Formula I antibody-zirconium conjugate (AZC) compounds include: 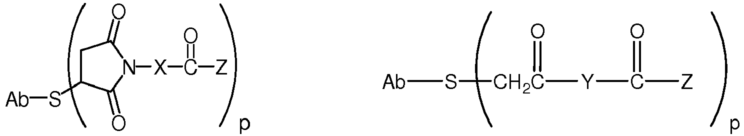 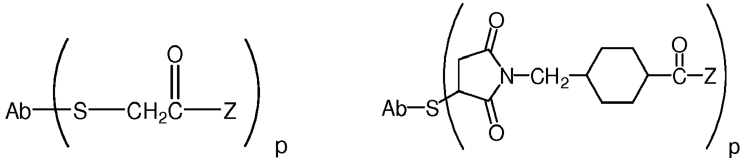 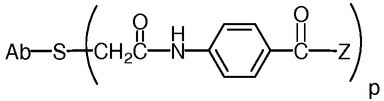 where X is: 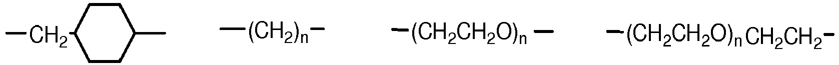 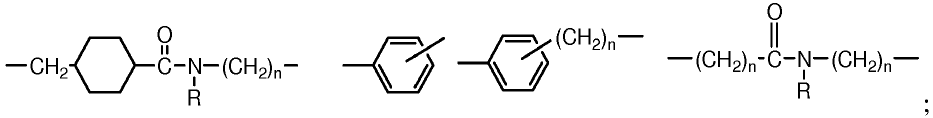 Y is: 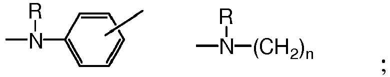 R is independently H or C₁-C₆ alkyl; and n is 1 to 12.

In another embodiment, a Linker has a reactive functional group which has a nucleophilic group that is reactive to an electrophilic group present on an antibody. Useful electrophilic groups on an antibody include, but are not limited to, aldehyde and ketone carbonyl groups. The heteroatom of a nucleophilic group of a Linker can react with an electrophilic group on an antibody and form a covalent bond to an antibody unit. Useful nucleophilic groups on a Linker include, but are not limited to, hydrazide, oxime, amino, hydrazine, thiosemicarbazone, hydrazine carboxylate, and arylhydrazide. The electrophilic group on an antibody provides a convenient site for attachment to a Linker.

In another embodiment, the Linker may be substituted with groups which modulated solubility or reactivity. For example, a charged substituent such as sulfonate (-SO₃⁻) or ammonium, may increase water solubility of the reagent and facilitate the coupling reaction of the linker reagent with the antibody or the zirconium moiety, or facilitate the coupling reaction of Ab-L (antibody-linker intermediate) with Z, or Z-L (zirconium-linker intermediate) with Ab, depending on the synthetic route employed to prepare the AZC.

The compounds of the disclosuredisclosure expressly contemplate, but are not limited to, AZC prepared with linker reagents: BMPEO, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate), and including bis-maleimide reagents: DTME, BMB, BMDB, BMH, BMOE, BM(PEO)₂, and BM(PEO)₄₃, which are commercially available from Pierce Biotechnology, Inc., Customer Service Department, P.O. Box 117, Rockford, IL. 61105 U.S.A. Bis-maleimide reagents allow the attachment of the thiol group of a cysteine engineered antibody to a thiol-containing zirconium moiety, label, or linker intermediate, in a sequential or concurrent fashion. Other functional groups besides maleimide, which are reactive with a thiol group of a cysteine engineered antibody, zirconium moiety, label, or linker intermediate include iodoacetamide, bromoacetamide, vinyl pyridine, disulfide, pyridyl disulfide, isocyanate, and isothiocyanate.

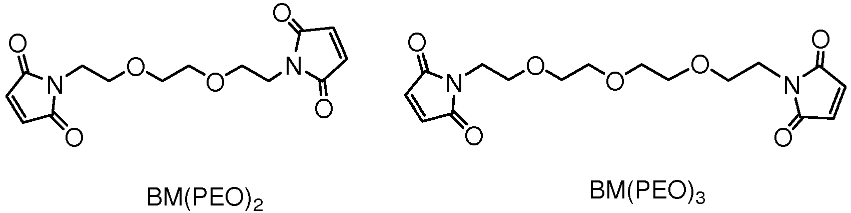

Useful linker reagents can also be obtained via other commercial sources, such as Molecular Biosciences Inc.(Boulder, CO), or synthesized in accordance with procedures described in Toki et al (2002) J. Org. Chem. 67:1866-1872; Walker, M.A. (1995) J. Org. Chem. 60:5352-5355; Frisch et al (1996) Bioconjugate Chem. 7:180-186; US 6214345; WO 02/088172; US 2003130189; US2003096743; WO 03/026577; WO 03/043583; and WO 04/032828.

Exemplary linker reagents include: 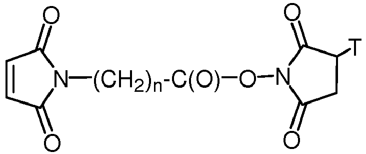 where n is an integer ranging from 1-10 and T is -H or -SO₃Na; 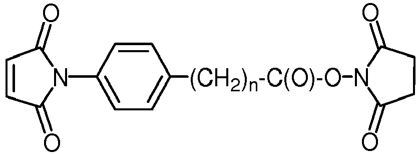 where n is an integer ranging from 0-3;

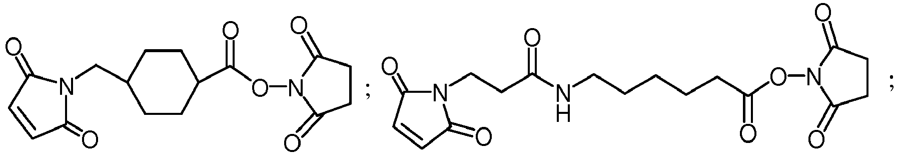

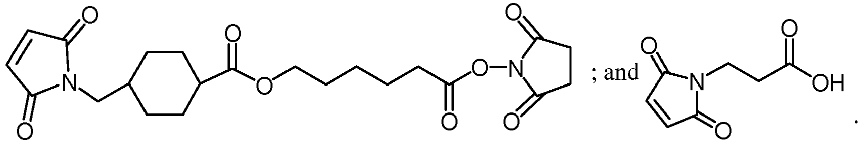

In another embodiment, linker L may be a dendritic type linker for covalent attachment of more than one zirconium moiety through a branching, multifunctional linker moiety to an antibody (Sun et al (2002) Bioorganic & Medicinal Chemistry Letters 12:2213-2215; Sun et al (2003) Bioorganic & Medicinal Chemistry 11:1761-1768). Dendritic linkers can increase the molar ratio of zirconium to antibody, i.e. loading of the AZC. Thus, where a cysteine engineered antibody bears only one reactive cysteine thiol group, a multitude of zirconium moieties may be attached through a dendritic linker.

The following exemplary embodiments of dendritic linker reagents allow up to nine nucleophilic zirconium moiety reagents to be conjugated by reaction with the chloroethyl nitrogen mustard functional groups: 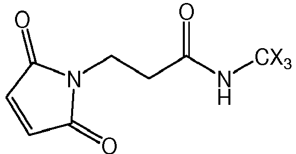 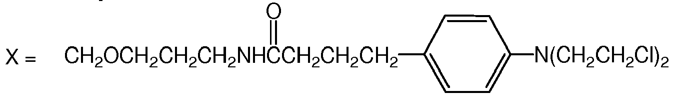 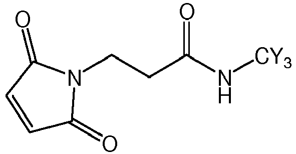 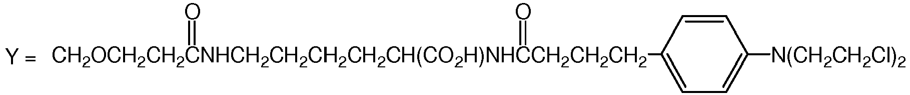 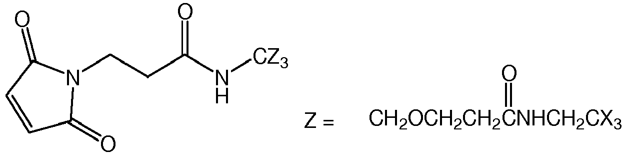 or

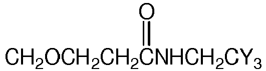

Other embodiments of branched, dendritic linkers include those with self-immolative 2,6-bis(hydroxymethyl)-p-cresol and 2,4,6-tris(hydroxymethyl)-phenol dendrimer units (WO 2004/01993; Szalai et al (2003) J. Amer. Chem. Soc. 125:15688-15689; Shamis et al (2004) J. Amer. Chem. Soc. 126:1726-1731; Amir et al (2003) Angew. Chem. Int. Ed. 42:4494-4499).

### DESFERRIOXAMINE-LABELLED, CYSTEINE-ENGINEERED ANTIBODIES

An aspect of the disclosuredisclosure is a desferrioxamine-labelled, cysteine-engineered antibody comprising a cysteine engineered antibody (Ab) conjugated through a free cysteine amino acid to a linker (L) and a desferrioxamine moiety (Df), having Formula II:

Ab-(L-Df)ₚ II

wherein L-Df is selected from: 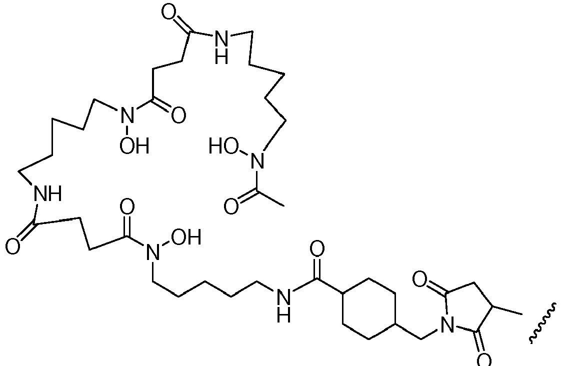 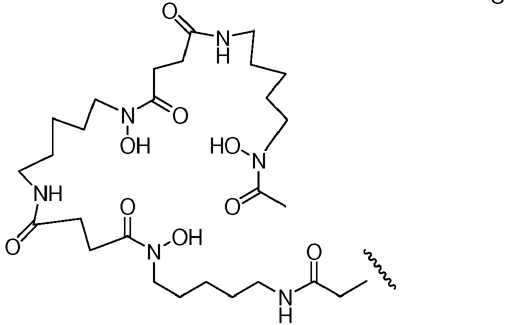 where the wavy line indicates the attachment to the antibody (Ab); and
p is 1 to 4.

### PREPARATION OF ANTIBODY-ZIRCONIUM CONJUGATES

The antibody-zirconium conjugates (AZC) of Formula I may be prepared by several routes, employing organic chemistry reactions, conditions, and reagents known to those skilled in the art, including: (1) reaction of a cysteine group of a cysteine engineered antibody with a linker reagent, to form antibody-linker intermediate Ab-L, via a covalent bond, followed by reaction with an activated zirconium label moiety Z; and (2) reaction of a nucleophilic group of a zirconium moiety with a linker reagent, to form zirconium label-linker intermediate Z-L, via a covalent bond, followed by reaction with a cysteine group of a cysteine engineered antibody. Conjugation methods (1) and (2) may be employed with a variety of cysteine engineered antibodies, zirconium label moieties, and linkers to prepare the antibody-zirconium conjugates of Formula I.

Antibody cysteine thiol groups are nucleophilic and capable of reacting to form covalent bonds with electrophilic groups on linker reagents and zirconium-linker intermediates including: (i) active esters such as NHS esters, HOBt esters, haloformates, and acid halides; (ii) alkyl and benzyl halides, such as haloacetamides; (iii) aldehydes, ketones, carboxyl, and maleimide groups; and (iv) disulfides, including pyridyl disulfides, via sulfide exchange. Nucleophilic groups on a zirconium lable moiety include, but are not limited to: amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents.

Under certain conditions, the cysteine engineered antibodies may be made reactive for conjugation with linker reagents by treatment with a reducing agent such as DTT (Cleland's reagent, dithiothreitol) or TCEP (tris(2-carboxyethyl)phosphine hydrochloride; Getz et al (1999) Anal. Biochem. Vol 273:73-80; Soltec Ventures, Beverly, MA). Full length, cysteine engineered monoclonal antibodies (ThioMabs) expressed in CHO cells were reduced with about a 50 fold excess of TCEP for 3 hrs at 37 °C to reduce disulfide bonds which may form between the newly introduced cysteine residues and the cysteine present in the culture media. The reduced ThioMab was diluted and loaded onto HiTrap S column in 10 mM sodium acetate, pH 5, and eluted with PBS containing 0.3M sodium chloride. Disulfide bonds were reestablished between cysteine residues present in the parent Mab with dilute (200 nM) aqueous copper sulfate (CuSO₄) at room temperature, overnight. Other oxidants, i.e. oxidizing agents, and oxidizing conditions, which are known in the art may be used. Ambient air oxidation is also effective. This mild, partial reoxidation step forms intrachain disulfides efficiently with high fidelity. An approximate 10 fold excess of zirconium-linker intermediate is added, mixed, and let stand for about an hour at room temperature to effect conjugation and form the ThioMab antibody-zirconium conjugate. The conjugation mixture is gel filtered and loaded and eluted through a HiTrap S column to remove excess zirconium-linker intermediate and other impurities.

Figure 15 shows the general process to prepare a cysteine engineered antibody expressed from cell culture for conjugation. Cysteine adducts, presumably along with various interchain disulfide bonds, are reductively cleaved to give a reduced form of the antibody. The interchain disulfide bonds between paired cysteine residues are reformed under partial oxidation conditions, such as exposure to ambient oxygen. The newly introduced, engineered, and unpaired cysteine residues remain available for reaction with linker reagents or zirconium-linker intermediates to form the antibody conjugates of the disclosure. The ThioMabs expressed in mammalian cell lines result in externally conjugated Cys adduct to an engineered Cys through -S-S- bond formation. Hence the purified ThioMabs have to be treated with reduction and oxidation procedures as described in Example 11 to produce reactive ThioMabs. These ThioMabs are used to conjugate with maleimide containing radiolabels, cytotoxic drugs, fluorophores, and other labels.

### PREPARATION AND ANALYSIS OF ⁸⁹Zr-Df-TRASTUZUMAB CONJUGATES

The protected active ester TFP-N-SucDf-Fe was prepared according to the previously described procedure (Verel I et al "89Zr Immuno-PET: Comprehensive Procedures For The Production Of 89Zr-Labeled Monoclonal Antibodies" (2003) J Nucl Med 44:1271-81) and conjugated to trastuzumab using a 5-fold molar excess of TFP-N-SucDf-Fe to yield N-SucDf-trastuzumab with an average of 1.6 molecules of desferrioxamine (Table 8). Df-Bz-SCN-trastuzumab was obtained by coupling an 8-fold molar excess of Df-Bz-SCN at pH 8.5 (Perk LR, et al. "Facile radiolabeling of monoclonal antibodies and other proteins with zirconium-89 or gallium-68 for PET Imaging using p-isothiocyanatobenzyl-desferrioxamine" (2008) Nature Protocols; published online:DOI:10.1038/nprot.2008.22). The reaction provided Df-Bz-SCN-trastuzumab decorated in average with 2.4 molecules of desferrioxamine (Table 1).

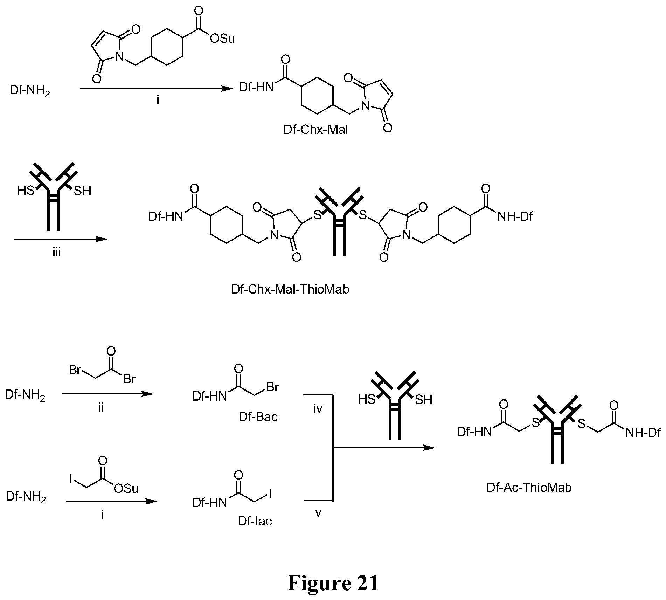

The novel maleimide based thiol reactive bifunctional linker Df-Chx-Mal was prepared from equimolar amounts of desferrioxamine mesylate and SMCC (Figure 21, Example 13). The reaction was complete within 30 min at room temperature and the product was isolated by precipitation upon addition of water in 45% yield and more than 95% purity. The reaction of an 8.5-fold molar excess of Df-Chx-Mal with freshly prepared thio-trastuzumab (Figure 21 Example 17) provided Df-Chx-Mal-thio-trastuzumab conjugate with exactly 2 molecules of desferrioxamine in 1 h (Table 1, Figure 21). Bromoacetyl desferrioxamine (BDf-Bac) was prepared by the reaction of equimolar amounts of desferrioxamine mesylate and bromoacetyl bromide at 0°C (Example 14). The product was obtained in 14% yield after HPLC purification. The alkylation of freshly prepared thio-trastuzumab (Figure 21, Example 16) with a 12-fold molar excess of Df-Bac provided the conjugate (Df-Ac-thio-trastuzumab) with 1.8 molecules of Df per antibody within 5 h (Table 8, Figure 21, Example 18). The low reactivity of bromide prompted us to explore the more reactive iodoacetyl derivative (Df-Iac). Df-Iac was prepared in 53% yield by the reaction of desferrioxamine mesylate with a slight excess of N-hydroxysuccinimidyl iodoacetate (Figure 21, Example 15). The product was obtained in more than 95% purity by precipitation from the reaction mixture. The subsequent reaction of an 11-fold excess of Df-Iac provided Df-Ac-thio-trastuzumab decorated with 1.8 molecules of Df within 2 h (Table 1, Figure 21, Example 19). Based on our experience, Df-Chx-Mal is the preferred reagent of the three compounds investigated. Notably, the reaction of Df-Chx-Mal was complete at a mild pH within 1 h as oppose to higher pH and longer reaction time required for the alkylation of thiol groups with Df-Bac and Df-Iac. Additionally, the lower reactivity of haloacetamides might have caused the incomplete loading of both available cysteines of thio-trastuzumab

**Table 8. Reaction conditions and yields of Df-linker-trastuzumab conjugates prepared using various reagents**

| Reagent | Temp. [°C] | pH | Reagent Excess | Reaction Time [hr] | Loading [Df/Mab] |
|---|---|---|---|---|---|
| Fe-Df-N-Suc-TFP | 37 | 8.5 | 5 | 1.5 | 1.6 |
| Df-Bz-SCN | 37 | 9 | 8 | 0.5 | 2.4 |
| Df-Chx-Mal | 25 | 7.5 | 8.5 | 1 | 2.0 |
| Df-Bac | 25 | 9 | 12 | 5 | 1.8 |
| Df-Iac | 25 | 9 | 11 | 2 | 1.8 |

The ⁸⁹Zr was chelated as 89-zirconium oxalate with all four variants of Df-trastuzumab using previously described experimental procedure (Verel I et al "89Zr immuno-PET: comprehensive procedures for the production of 89Zr-labeled monoclonal antibodies" (2003) J Nucl Med. 44:1271-81). The radiolabeled proteins were purified on a desalting column and the final solution was concentrated to the required volume by membrane filtration. The yield, purity, and final specific activity of the ⁸⁹Zr conjugates are summarized in Table 9. In general, the chelation yield was over 80% with the exception of the Df-N-Suc linker obtained in lower yield presumably due to the lower amount of Df per antibody molecule and/or incomplete removal of Fe(III) used to protect the chelator during activation and conjugation. After purification of the Df-trastuzumab variants using desalting column, the product purity was over 90% with a small amount (1-6%) of high molecular weight aggregates detected in each sample. Df-Chx-Mal-thio-trastuzumab provided the ⁸⁹Zr complex in 99% purity (Table 9) as opposed to Df-Ac conjugate which was contaminated with approximately 8% of a low molecular weight impurity and 2% of high molecular weight aggregates. The contaminant resisted removal using NAP-10 column but the removal was possible using repeated buffer exchange on Amicon filter.

**Table 9. Yields, specific activity and purity of radiolabeled ⁸⁹Zr-Df-linker-trastuzumab**

| Linker | Radiochemical yield [%] | Specific activity [mCi/mg] | Purity [%] |
|---|---|---|---|
| N-Suc | 60 | 2.2 | 98 |
| Bz-SCN | 81 | 2.9 | 94 |
| Chx-Mal | 87 | 3.4 | 99 |
| Ac | 84 | 3.2 | 90 |

### BIOLOGICAL ACTIVITY OF ⁸⁹Zr-Df-TRASTUZUMAB CONJUGATES

The biological activities of newly prepared site-specific Df-*linker*-thio-trastuzumab conjugates were determined using binding assay to BT474 breast cancer cell line by Scatchard analysis. The obtained *K_{D}* values were compared to non-modified trastuzumab (0.91 ± 0.20 nM). The *K_{D}* for the thio-trastuzumab conjugate containing Chx-Mal linker was 0.93 ± 0.15 nM and the values for the conjugates containing Ac linker were 1.22 ± 0.22 nM for conjugate prepared using Df-Bac and 0.87 ± 0.15 nM prepared using Df-Iac. The results of the biological activity analyses indicate that the modification of thio-trastuzumab did not affect the binding affinity of the antibody to HER2.

### IN VITRO SERUM STABILITY

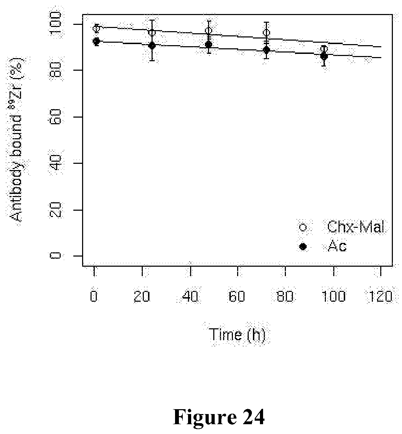

The previously reported Df-antibody conjugate with N-Suc and Bz-SCN linkers containing amide or thiourea linkages were stable *in vitro* over a 6 day period in serum at 37 °C (Verel I et al "89Zr immuno-PET: comprehensive procedures for the production of 89Zr-labeled monoclonal antibodies" J Nucl Med 2003;44:1271-81; Perk LR et al, (2009) European Journal Of Nuclear Medicine And Molecular Imaging 35(10): 1857-1867). The stability of ⁸⁹Zr-thio-trastuzumab conjugates with Chx-Mal and Ac linkers was determined in mouse serum at 37 °C. A significant loss of antibody bound ⁸⁹Zr was not observed within a 5 day period. Both thio conjugates were stable with an average loss of antibody bound ⁸⁹Zr 1.8% per day for ⁸⁹Zr-Df-Chx-Mal-thio-trastuzumab and 1.4% per day for ⁸⁹Zr-Df-Ac-thio-trastuzumab (Figure 24). Slow formation of high molecular weight species, presumably aggregates, was observed for both Df-Chx-Mal and Df-Ac linkers.

### IN VIVO MICROPET IMAGING

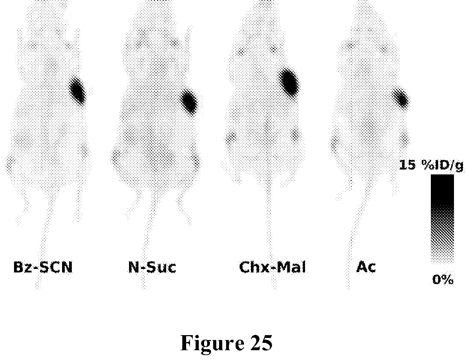

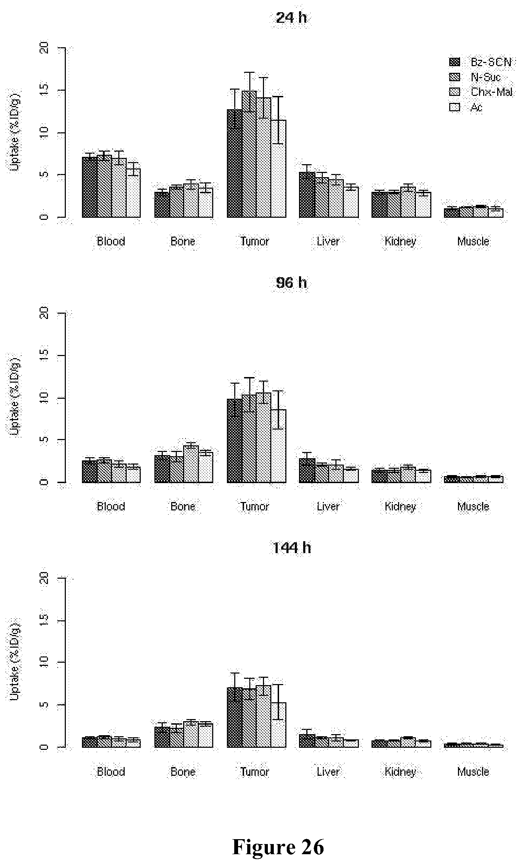

Twenty animals (5 animals per group) bearing subcutaneous BT474M1 xenografts (size -200 mm³) were injected intravenously with ⁸⁹Zr-trastuzumab. The amount of antibody injected per animal was 1.4 ± 0.29 mg/kg. The maximum intensity projection images of representative animals (at 96 h p.i.) are shown in Fig. 3. The ⁸⁹Zr-trastuzumab uptake in selected tissues is summarized in Fig 4. The images at 1 h (not shown) were dominated by the high blood pool uptake with the exception of Df-Bac where rapid hepatobiliary excretion of the lipophilic impurity resulted in an elevated uptake in intestine. The impurity was totally cleared within the first 24 h and elevated small and large intestine uptake was not detected at 24 h or later time after tracer injection. Although the tissue uptake of Df-Ac conjugate was resultantly slightly (-8%) lower, the tumor to blood ratios (Table 10) were not affected by the loss of injected radioactivity. The images at 96 h were dominated by the high tumor uptake with minor differences observed among the four different ⁸⁹Zr-trastuzumab variants (Figure 25). The tumor uptake was identical for each tracer reaching maximum values at 24 h post injection and maximum tumor-to-blood ratios at 144 h due to blood clearance (Table 10). The thiol based conjugate ⁸⁹Zr-Df-Chx-Mal-thio-trastuzumab exhibited elevated bone uptake (*P* < 0.05) compared to the amine based conjugates (Df-Bz-SCN and Df-N-Suc) at 96 and 144 h p.i. The bone uptake of Df-Ac-thio-trastuzumab was not significantly elevated (*P* = 0.20) compared to the Df-Bz-SCN and Df-N-Suc linkers but may become significant when corrected for the 8% loss of radioactivity during the first 24 h. The kidney uptake of each tracer was low (Figure 26) as expected for antibody based tracers but ⁸⁹Zr-Df-Chx-Mal-thio-trastuzumab was slightly higher compared to other linkers at 24, 96 and 144 h (*P* < 0.05).

**Table 10. Average tumor to blood ratios at 24, 96 and 144 h post injection**

| Linker | 24 h | 96 h | 144 h |
|---|---|---|---|
| N-Suc | 1.8 | 3.8 | 6.0 |
| Bz-SCN | 2.0 | 4.0 | 5.7 |
| Chx-Mal | 2.0 | 4.9 | 7.1 |
| Ac (Bac) | 2.0 | 4.7 | 6.1 |

BT474 (3+ expression level of HER2) xenografts exhibited lower absolute uptake of the tracer (15 %ID/g) than measured previously by Dijkers et al in SKOV3 (3+ expression level of HER2) 33.4 ± 7.7%ID/g (Dijkers EC, et al. "Development and Characterization of Clinical-Grade 89Zr-Trastuzumab for HER2/neu ImmunoPET Imaging" (2009) J Nucl Med 50(6):974-981). However, the tumor to blood ratio of 5.7-7.1 (Table 10) is comparable to the value obtained with SKOV3 (tumor to blood of 7.6). The difference in tumor uptake may be attributed to the tumor model and total dose of trastuzumab. A material with higher specific activity was used hence so significantly less antibody was injected (35 µg, 1.4 mg/kg) compared to the Dijkers et al study with SKOV3 (100 µg, 4 mg/kg). The difference in specific activity may have also contributed to lower bone uptake of free ⁸⁹Zr in the experiment herein of 2-3 %ID/g compared to SKOV3 model (5-10 %ID/g). Unfortunately, no teaching regarding the elevated bone uptake is provided by Dijkers et al. Zirconium is known to bind plasma proteins (Mealey J, Jr. "Turn-over of carrier-free zirconium-89 in man" (1957) Nature 179:673-4 and is later deposited in mineral bone (Fletcher CR. "The radiological hazards of zirconium-95 and niobium-95" (1969) Health Phys. 16:209-20; Shiraishi Y and Ichikawa R. "Absorption and retention of 144 Ce and 95 Zr- 95 Nb in newborn, juvenile and adult rats" (1972) Health Phys. 22:373-8). Since the injected material did not contain free ⁸⁹Zr, the bone uptake may originate from the breakdown of the ⁸⁹Zr-antibody or from ⁸⁹Zr non-specifically associated with antibody which could then trans-chelate to plasma proteins compared to ⁸⁹Zr bound to Df.

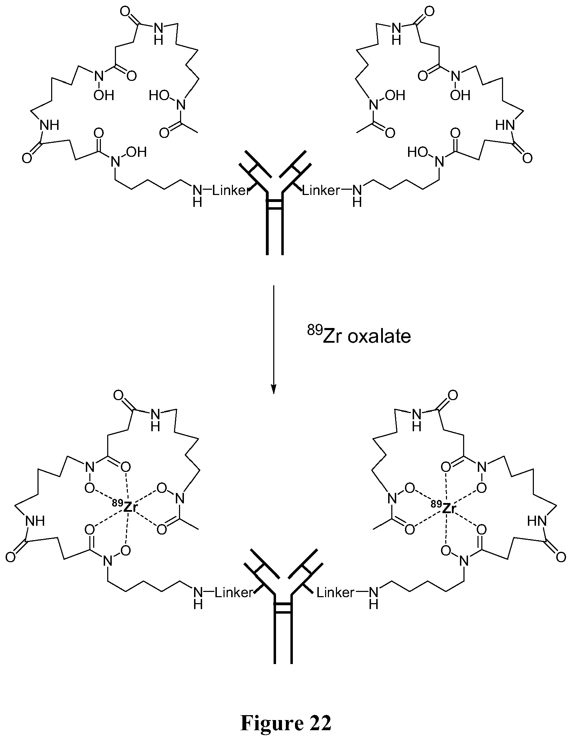

Three thiol specific reagents are exemplified herein for the chemoselective conjugation of desferrioxamine (Df) to monoclonal antibodies through the thiol group of cysteine of cysteine-engineered antibodies. The thiol-specific Df-reagents were obtained by the acylation of the amino group of desferrioxamine B in 14% (Df-Bac), 53% (Df-Iac) and 45% (Df-Chx-Mal) yields and conjugated to thio-trastuzumab resulting in site-specific modification on both engineered cysteines within 1-5 h. The binding activities of site-specific thio-trastuzumab conjugates to HER2 were identical to the activity of non-modified trastuzumab. The Df-modified thio-trastuzumabs (Df-Ac-thio-trastuzumab and Df-Chx-Mal-thio-trastuzumab) were chelated with ⁸⁹Zr (Figure 22) in yields exceeding 80% within 1 h comparable to lysine conjugates prepared using previously described Df-Bz-SCN and Df-N-Suc linkers. Both ⁸⁹Zr-Df-Ac-thio-trastuzumab and ⁸⁹Zr-Df-Chx-Mal-thio-trastuzumab showed comparable stability in mouse serum. Both compounds also showed PET imaging capabilities in BT474M1 breast cancer model comparable to lysine conjugates reaching 10-15 %ID/g of tumor uptake with a tumor to blood ratio in the range 6.1-7.1. Overall, the novel reagents are readily available, demonstrated good reactivity with thiol groups of the protein, and exhibited very good chelation properties with ⁸⁹Zr. The ⁸⁹Zr-labeled antibodies were stable in serum and showed excellent PET imaging properties. Df-Chx-Mal is a useful reagent for conjugation of Df to antibodies through cysteine side chain and showed several advantages over Df-Bac and Df-Iac. First, moderate pH 7.5 was required for complete conjugation of Df-Chx-Mal within 1 h as compared to pH 9 and 2 or 5 h required for Df-Bac and Df-Iac. Additionally, the site specifically ⁸⁹Zr labeled engineered THIOMAB conjugates can be used similarly as ¹⁸F labeled THIOFAB conjugates (Gill HS, et al. "A modular platform for the rapid site-specific radiolabeling of proteins with 18F exemplified by quantitative positron emission tomography of human epidermal growth factor receptor 2" (2009) Jour. of Med. Chem. 52:5816-25) as valuable tools for PET imaging applications in biomedical research.

### ADMINISTRATION OF ANTIBODY-ZIRCONIUM CONJUGATES

The antibody-zirconium conjugates (AZC) of the disclosure may be administered by any route appropriate to the condition to be treated. The AZC will typically be administered parenterally, i.e. infusion, subcutaneous, intramuscular, intravenous, intradermal, intrathecal and epidural.

### PHARMACEUTICAL FORMULATIONS

Pharmaceutical formulations of diagnostic antibody-zirconium conjugates (AZC) of the disclosure are typically prepared for parenteral administration, i.e. bolus, intravenous, intratumor injection with a pharmaceutically acceptable parenteral vehicle and in a unit dosage injectable form. An antibody-zirconium conjugate (AZC) having the desired degree of purity is optionally mixed with pharmaceutically acceptable diluents, carriers, excipients or stabilizers (Remington's Pharmaceutical Sciences (1980) 16th edition, Osol, A. Ed.), in the form of a lyophilized formulation or an aqueous solution.

Acceptable diluents, carriers, excipients, and stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG). For example, lyophilized anti-ErbB2 antibody formulations are described in WO 97/04801.

The active pharmaceutical ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semi permeable matrices of solid hydrophobic polymers containing the AZC, which matrices are in the form of shaped articles, e.g. films, or microcapsules. Examples of sustained-release matrices include polyesters, hydrogels (for example, poly(2-hydroxyethyl-methacrylate), or poly(vinyl alcohol)), polylactides (US 3773919), copolymers of L-glutamic acid and gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile, which is readily accomplished by filtration through sterile filtration membranes.

The formulations include those suitable for the foregoing administration routes. The formulations may conveniently be presented in unit dosage form and may be prepared by any of the methods well known in the art of pharmacy. Techniques and formulations generally are found in Remington's Pharmaceutical Sciences (Mack Publishing Co., Easton, PA). Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Aqueous suspensions of the disclosure contain the active materials in admixture with excipients suitable for the manufacture of aqueous suspensions. Such excipients include a suspending agent, such as sodium carboxymethylcellulose, croscarmellose, povidone, methylcellulose, hydroxypropyl methylcelluose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

The pharmaceutical compositions of AZC may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,3-butane-diol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.

The amount of active ingredient that may be combined with the carrier material to produce a single dosage form will vary depending upon the host treated and the particular mode of administration. For example, an aqueous solution intended for intravenous infusion may contain from about 3 to 500 µg of the active ingredient per milliliter of solution in order that infusion of a suitable volume at a rate of about 30 mL/hr can occur.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

Although oral administration of protein therapeutics are disfavored due to hydrolysis or denaturation in the gut, formulations of AZC suitable for oral administration may be prepared as discrete units such as capsules, cachets or tablets each containing a predetermined amount of the AZC.

The formulations may be packaged in unit-dose or multi-dose containers, for example sealed ampoules and vials, and may be stored in a freeze-dried (lyophilized) condition requiring only the addition of the sterile liquid carrier, for example water, for injection immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

The disclosure further provides veterinary compositions comprising at least one active ingredient as above defined together with a veterinary carrier therefore. Veterinary carriers are materials useful for the purpose of administering the composition and may be solid, liquid or gaseous materials which are otherwise inert or acceptable in the veterinary art and are compatible with the active ingredient. These veterinary compositions may be administered parenterally, orally or by any other desired route.

### LABELLED ANTIBODY IMAGING METHODS

In another embodiment of the disclosure, cysteine engineered antibodies may be labelled through the cysteine thiol with radionuclides, fluorescent dyes, bioluminescence-triggering substrate moieties, chemiluminescence-triggering substrate moieties, enzymes, and other detection labels for imaging experiments with diagnostic, pharmacodynamic, and therapeutic applications. Generally, the labelled cysteine engineered antibody, i.e. "biomarker" or "probe", is administered by injection, perfusion, or oral ingestion to a living organism, e.g. human, rodent, or other small animal, a perfused organ, or tissue sample. The distribution of the probe is detected over a time course and represented by an image.

### EXAMPLES

Preparation of Solvents and chemicals were purchased from Aldrich (Milwaukee, WI) unless stated otherwise. The following reversed-phase HPLC systems were used to analyze and purify the products. System A: Phenomenex BioSep-SEC-S 3000 (300 × 4.60 mm, 5 µm) 50 mM PBS 0.5 ml/min equipped with UV absorbance and radioactivity detector (PMT); System B Altima C-18 (100 × 22.0 mm, 5 µm) 0.05% TFA + 10-50% acetonitrile, 0-30 min, 24 mL/min, equipped with UV detector. Mass spectrometry analysis of low molecular weight products was performed on a PE Sciex API 150EX LCMS system equipped with an Onyx Monolithic C₁₈ column. LCMS analysis of proteins was performed on a TSQ Quantum Triple quadrupole mass spectrometer with extended mass range (Thermo Electron, Thermo Fisher Scientific Inc., USA). The protein samples for LCMS analysis were reduced by treatment with 20 mM dithiothreitol (DTT) at 37°C for 1 h to separate heavy and light chains. Samples were chromatographed on a PRLP-S 1000 Å microbore column (50 mm × 2.1 mm, Polymer Laboratories, Varian Inc., USA) heated to 75°C. A linear gradient from 30-40% B (solvent A, 0.05% TFA in water; solvent B, 0.04% TFA in acetonitrile) was used and the eluant was directly ionized using the electrospray source. Data were collected by the Xcalibur data system and deconvolution was performed using ProMass software (Novatia, Monmouth Junction, NJ). NMR spectra were recorded on Bruker Avance II 400 spectrometer at 298K and the chemical shifts are reported relative to TMS. Protein concentrations were measured at 280 nm using Eppendorf BioPhotometer (Westbury, NY). ⁸⁹Zr was obtained from Memorial Sloan-Kettering Cancer Center (New York, NY) as ⁸⁹Zr(IV) oxalate in 1M oxalic acid solution with specific activity 470-1195 Ci/mmol (Holland JP, et al (2009) "Standardized methods for the production of high specific-activity zirconium-89" Nucl Med Biol. 36:729-39). Heterobifunctional linker succinimidyl 4-[*N*-maleimidomethyl]cyclohexane-1-carboxylate (SMCC) was purchased from Pierce (Rockford, IL) and N-hydroxysuccinimidyl iodoacetate was obtained from Indofine Chemical Company (Hillsborough, NJ). NAP-10 columns were obtained from (GE Healthcare, USA) and Amicon Ultra-4 centrifugal filters (10,000 MWCO) from Millipore (Billerica, MA). Df-Bz-SCN was purchased Macrocyclics (Dallas, TX).

### Example 1 Preparation of Biotinylated ThioFab Phage

ThioFab-phage (5 x 10¹² phage particles) were reacted with 150 fold excess of biotin-PEO-maleimide ((+)-biotinyl-3-maleimidopropionamidyl-3,6-dioxaoctainediamine, Oda et al (2001) Nature Biotechnology 19:379-382, Pierce Biotechnology, Inc.) for 3 hours at room temperature. Excess biotin-PEO-maleimide was removed from biotin-conjugated phage by repeated PEG precipitations (3-4 times). Other commercially available biotinylation reagents with electrophilic groups which are reactive with cysteine thiol groups may be used, including Biotin-BMCC, PEO-Iodoacetyl Biotin, Iodoacetyl-LC-Biotin, and Biotin-HPDP (Pierce Biotechnology, Inc.), and N*^{α}*-(3- maleimidylpropionyl)biocytin (MPB, Molecular Probes, Eugene, OR). Other commercial sources for biotinylation, bifunctional and multifunctional linker reagents include Molecular Probes, Eugene, OR, and Sigma, St. Louis, MO.

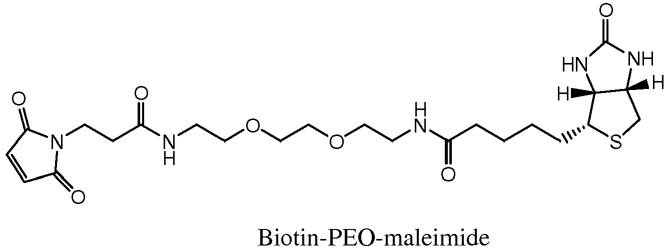

### Example 2 PHESELECTOR Assay

Bovine serum albumin (BSA), erbB2 extracellular domain (HER2) and streptavidin (100 µl of 2 µg/ml) were separately coated on Maxisorp 96 well plates. After blocking with 0.5% Tween-20 (in PBS), biotinylated and non-biotinylated hu4D5Fabv8-ThioFab-Phage (2x 10¹⁰ phage particles) were incubated for 1 hour at room temperature followed by incubation with horseradish peroxidase (HRP) labeled secondary antibody (anti-M13 phage coat protein, pVIII protein antibody). Figure 8 illustrates the PHESELECTOR Assay by a schematic representation depicting the binding of Fab or ThioFab to HER2 (top) and biotinylated ThioFab to streptavidin (bottom).

Standard HRP reaction was carried out and the absorbance was measured at 450 nm. Thiol reactivity was measured by calculating the ratio between OD₄₅₀ for streptavidin/OD₄₅₀ for HER2. A thiol reactivity value of 1 indicates complete biotinylation of the cysteine thiol. In the case of Fab protein binding measurements, hu4D5Fabv8 (2-20 ng) was used followed by incubation with HRP labeled goat polyclonal anti-Fab antibodies.

### Example 3a Expression and Purification of ThioFabs

ThioFabs were expressed upon induction in 34B8, a non-suppressor *E. coli* strain (Baca et al (1997) Journal Biological Chemistry 272(16): 10678-84). The harvested cell pellet was resuspended in PBS (phosphate buffered saline), total cell lysis was performed by passing through a microfluidizer and the ThioFabs were purified by affinity chromatography with protein G SEPHAROSE™ (Amersham).

ThioFabs L-V15C, L-V110C, H-A88C, and H-A121C were expressed and purified by Protein-G SEPHAROSE™ column chromatography. Oligomeric-Fab was present in fractions 26 to 30, and most of the monomeric form was in fractions 31-34. Fractions consisting of the monomeric form were pooled and analyzed by SDS-PAGE along with wild type hu4D5Fabv8and analyzed on SDS-PAGE gel in reducing (with DTT or BME) and non-reducing (without DTT or BME) conditions. Gel filtration fractions of A121C-ThioFab were analyzed on non-reducing SDS-PAGE.

ThioFabs were conjugated with biotin-PEO-maleimide as described above and the biotinylated-ThioFabs were further purified by Superdex-200™ (Amersham) gel filtration chromatography, which eliminated the free biotin-PEO-maleimide and the oligomeric fraction of ThioFabs. Wild type hu4D5Fabv8 and hu4D5Fabv8 A121C-ThioFab (0.5 mg in quantity) were each and separately incubated with 100 fold molar excess of biotin-PEO-maleimide for 3 hours at room temperature and loaded onto a Superdex-200 gel filtration column to separate free biotin as well as oligomeric Fabs from the monomeric form.

### Example 3b Analysis of ThioFabs

Enzymatic digest fragments of biotinylated hu4D5Fabv8 (A121C) ThioFab and wild type hu4D5Fabv8 were analyzed by liquid chromatography electrospray ionization mass spectroscopy (LS-ESI-MS) The difference between the 48294.5 primary mass of biotinylated hu4D5Fabv8 (A121C) and the 47737.0 primary mass of wild type hu4D5Fabv8 was 557.5 mass units. This fragment indicates the presence of a single biotin-PEO-maleimide moiety (C₂₃H₃₆N₅O₇S₂). Table 4 shows assignment of the fragmentation values which confirms the sequence.

**Table 4. LC-ESI-Mass spec analysis of biotinylated hu4D5Fabv8 ThioFab A121C after tryptic digestion**

| **Amino acid** | **b Fragment** | **y Fragment** |
|---|---|---|
| A (Alanine) | 72 | |
| M (Methionine) | 203 | 2505 |
| D (Aspartic acid) | 318 | 2374 |
| Y (Tyrosine) | 481 | 2259 |
| W (Tryptophan) | 667 | 2096 |
| G (Glycine) | 724 | 1910 |
| Q (glutamine) | 852 | 1853 |
| G (Glycine) | 909 | 1725 |
| T (Threonine) | 1010 | 1668 |
| L (Leucine) | 1123 | 1567 |
| V (Valine) | 1222 | 1454 |
| T (Threonine) | 1323 | 1355 |
| V (Valine) | 1422 | 1254 |
| S (Serine) | 1509 | 1155 |
| S (Serine) | 1596 | 1068 |
| **C (Cysteine) + biotin** | **2242** | **981** |
| S (Serine) | 2329 | 335 |
| T (Threonine) | 2430 | 248 |
| K (Lysine) | | 175 |

Before and after Superdex-200 gel filtration, SDS-PAGE gel analyses, with and without reduction by DTT or BME, of biotinylated ABP- hu4D5Fabv8-A121C, biotinylated ABP- hu4D5Fabv8-V110C, biotinylated double Cys ABP-hu4D5Fabv8-(V110C-A88C), and biotinylated double Cys ABP-hu4D5Fabv8-(V110C-A121C) were conducted.

Mass spectroscopy analysis (MS/MS) of of hu4D5Fabv8-(V110C)-BMPEO-DM1 (after Superdex-200 gel filtration purification): Fab+1 51607.5, Fab 50515.5. This data shows 91.2% conjugation. MS/MS analysis of hu4D5Fabv8-(V110C)-BMPEO-DM1 (reduced): LC 23447.2, LC+1 24537.3, HC (Fab) 27072.5. This data shows that all DM1 conjugation is on the light chain of the Fab.

### Example 11 Reduction/Oxidation of ThioMabs for Conjugation

Full length, cysteine engineered monoclonal antibodies (ThioMabs) expressed in CHO cells were reduced with about a 50 fold excess of TCEP (tris(2-carboxyethyl)phosphine hydrochloride; Getz et al (1999) Anal. Biochem. Vol 273:73-80; Soltec Ventures, Beverly, MA) for 3 hrs at 37 °C. The reduced ThioMab (Figure 15) was diluted and loaded onto a HiTrap S column in 10 mM sodium acetate, pH 5, and eluted with PBS containing 0.3M sodium chloride. The eluted reduced ThioMab was treated with 200 nM aqueous copper sulfate (CuSO₄) at room temperature, overnight. Ambient air oxidation was also effective.

### Example 13 N-[4-(N-maleimidomethyl)cyclohexane- 1-carboxyl]desferrioxamine (Df-Chx-Mal)

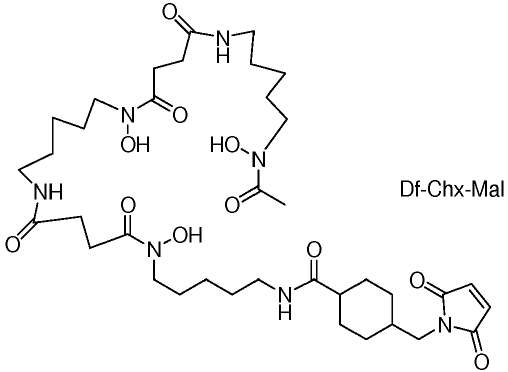

4-[*N*-Maleimidomethyl]cyclohexane-1-carboxylate (SMCC, 40 mg, 0.12 mmol), desferrioxamine mesylate (78 mg, 0.12 mmol) and *N,N*-diisopropylethylamine (22 µL, 0.13 mmol) were dissolved in a mixture of DMF (2.0 mL) and 0.2 mL water (Figure 21). The resulting turbid solution was stirred at room temperature for 30 min. Water (8 mL) was added and the precipitated product was isolated by filtration, washed with water, and dried at reduced pressure to yield 42 mg (45%) of N-[4-(N-maleimidomethyl)cyclohexane-1-carboxyl]desferrioxamine (Df-Chx-Mal) as a white solid (Figure 20 bottom). ¹H NMR (400 MHz, d₆-DMSO) δ 0.87-0.90 (m, 2H), 1.20-1.26 (m, 8H), 1.35-1.41 (m, 6H), 1.45-1.55 (m, 8H), 1.60-1.70 (m, 4H), 1.97 (s, 3H, acetyl), 2.26-2.29 (m, 4H), 2.56-2.60 (m, 4H), 2.95-3.05 (m, 6H), 3.24 (d, J = 7.0 Hz, 2H), 3.44-3.48 (m, 6H), 7.00 (s, 2H, maleimide), 7.62 (t, J = 5.4 Hz, 1H, amide), 7.75 (m, 2H, amide), 9.59 (s, 2H, hydroxyl), 9.64 (s, 1H, hydroxyl). MS ESI (m/z): [M+H]⁺ calculated for C₃₇H₆₂N₇O₁₁ 780.44; found 780.6.

### Example 14 N-bromoacetyldesferrioxamine (Df-Bac)

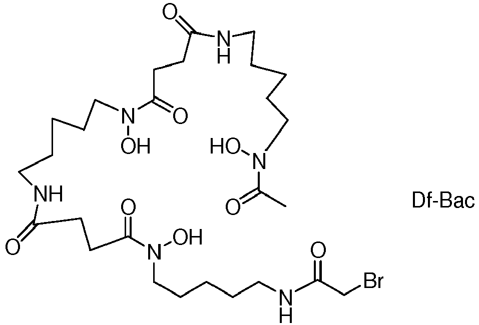

A solution of bromoacetyl bromide (27 µL, 0.30 mmol) in DMF (1 mL) was added dropwise in 5 min into a cooled (0°C) mixture of desferrioxamine mesylate (200 mg, 0.30 mmol) and *N,N*-diisopropylethylamine (106 µL, 0.60 mmol) in DMF (5 mL) after which the reaction mixture was stirred at 0°C for 4 h (Figure 21). Water (10 mL) was added and the product was isolated using HPLC (System B, retention time 7.5 min) to yield 29 mg (14%) of N-bromoacetyldesferrioxamine (Df-Bac) as a white solid (Figure 20 bottom). ¹H NMR (400 MHz, d₆-DMSO) δ 1.18-1.26 (m, 6H), 1.35-1.42 (m, 6H), 1.45-1.55 (m, 6H), 1.97 (s, 3H, acetyl), 2.24-2.30 (m, 4H), 2.54-2.59 (m, 4H), 2.96-3.07 (m, 6H), 3.44-3.47 (m, 6H), 3.82 (s, 2H, bromoacetyl), 7.74 (m, 2H, amide), 8.21 (t, 1H, amide), 9.59 (s, 2H, hydroxyl), 9.63 (s, 1H, hydroxyl). MS ESI (m/z): [M+H]⁺ calculated for C₂₇H₅₀BrN₆O₉ 681.27, 683.27; found 681.1, 683.0.

### Example 15 N-iodoacetyldesferrioxamine (Df-Iac)

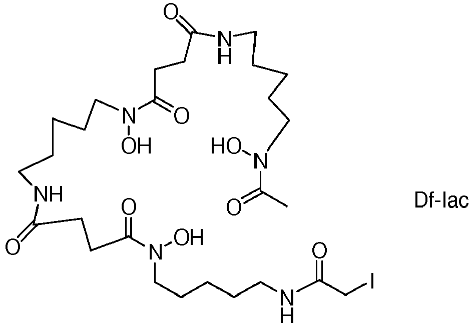

Desferrioxamine mesylate (200 mg, 0.30 mmol) and *N,N*-diisopropylethylamine (53 µL, 0.30 mmol) were mixed in DMF (4 mL) and water (0.4 mL). *N*-hydroxysuccinimidyl iodoacetate (93 mg, 0.33 mmol) was added and the resulting mixture was stirred at room temperature for 1 h (Figure 21). Water (8 mL) was added and the precipitated product was separated, washed with water, and dried at reduced pressure to yield 115 mg (53%) of N-iodoacetyldesferrioxamine (Df-Iac) as a white solid (Figure 20 bottom). ¹H NMR (400 MHz, d₆-DMSO) δ 1.20-1.25 (m, 6H), 1.35-1.42 (m, 6H), 1.47-1.54 (m, 6H), 1.97 (s, 3H, acetyl), 2.25-2.29 (m, 4H), 2.56-2.59 (m, 4H), 2.98-3.03 (m, 6H), 3.45 (m, 6H), 3.61 (s, 2H, iodoacetyl), 7.75 (m, 2H, amide), 8.17 (t, 1H, amide), 9.57 (s, 2H, hydroxyl), 9.61 (s, 1H, hydroxyl). MS ESI (m/z): [M+H]⁺ calculated for C₂₇H₅₀IN₆O₉ 729.26; found 729.1.

### Example 16 Thio-trastuzumab

The construction, expression, and purification of THIOMAB with Cys substitution at Ala¹¹⁴ (Kabat numbering) in heavy chain was described previously (Junutula JR, et al "Site-specific conjugation of a cytotoxic drug to an antibody improves the therapeutic index" (2008) Nat Biotechnol 26:925-32). The isolated thio-trastuzumab was prepared for conjugation by a reduction and re-oxidation procedure to remove disulfide adducts bound to Cys¹¹⁴. First, the protein was reduced for 24 h by treatment with a 40-fold molar excess of DTT and 2 mM EDTA in 88 mM Tris buffer pH 7.5. To remove DTT prior to re-oxidation, the thio-trastuzumab solution was adjusted to pH 5 by the addition of 10 mM sodium succinate buffer. The solution was then loaded on an ion exchange column (HiTrap SP FF, GE Healthcare) that had been sterilized and equilibrated with 10 mM sodium succinate buffer pH 5. The column was washed with 10 mM sodium succinate buffer (10 mL) and the thio-trastuzumab was then eluted with 3 mL of 50 mM Tris, 150 mM NaCl buffer with pH 7.5. thio-trastuzumab re-oxidization was achieved by treatment with a 25-fold molar excess of dehydroascobric acid (100 mM in *N,N*-dimethylacetamide (DMA)) in 75 mM Tris, 150 mM NaCl pH 7.5 buffer at 25°C for 3.5 h. After re-oxidation, the thio-trastuzumab was conjugated to desferrioxamine without further purification. MS ESI (m/z): found light chain 23440.0, heavy chain 50627.3.

### Example 17 Df-Chx-Mal-thio-trastuzumab

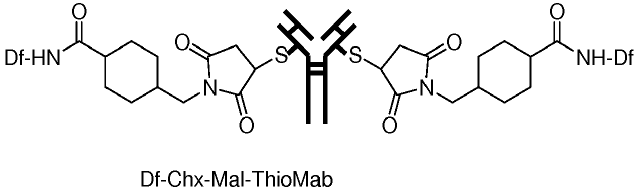

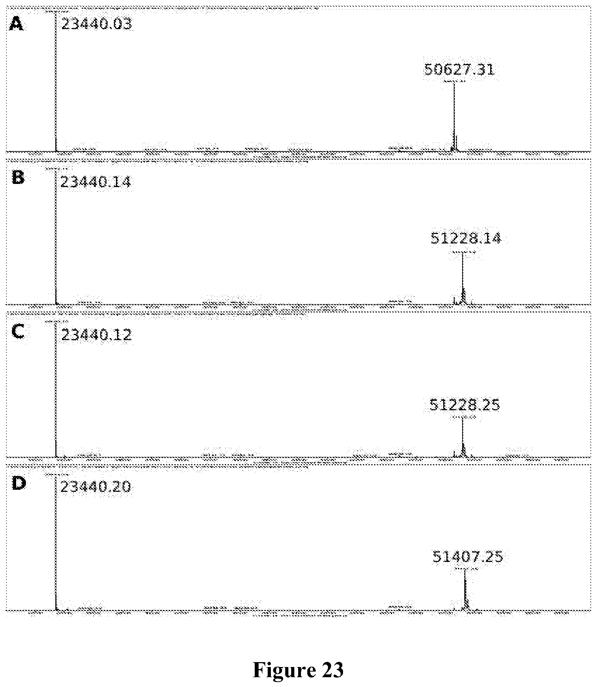

The 2 mM stock solution of the bifunctional chelator was prepared by dissolving Df-Chx-Mal (1.5 mg, 2 µmol) in a 1:1 mixture (1 mL) of DMF and DMA by heating to 44°C for 30 min, the stock solution was then aliquoted and stored at -80 °C (Figure 21). An aliquot of the stock solution (220 µL, 0.440 µmol) was then added to the solution of thio-trastuzumab (7.5 mg, 52 nmol) in 50 mM Tris, 150 mM NaCl buffer pH 7.5 (1.5 mL) and incubated at room temperature for 1 h. The solution was then buffer exchanged on an Amicon Ultra-4 filter into 0.25 M sodium acetate buffer to obtain 1 mL of Df-Chx-Mal-thio-trastuzumab conjugate solution at a concentration of 6 mg/mL. MS ESI (m/z): found light chain 23440.2, heavy chain 51407.3 (Figure 23, D).

### Example 18 Df-Ac-thio-trastuzumab using Df-Bac

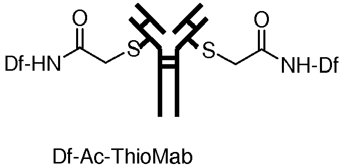

The 12 mM stock solution of the bifunctional chelator was prepared by dissolving Df-Bac (8 mg, 12 µmol) in 1 mL DMA (Figure 21). The stock solution was then aliquoted and stored at -80 °C. The re-oxidized thio-trastuzumab was buffer exchanged on an Amicon Ultra-4 filter into 0.05 M sodium borate buffer pH 9. An aliquot of the Df-Bac stock solution (35 µL, 0.410 µmol) was added to the solution of thio-trastuzumab (4.9 mg, 34 nmol) in 0.05 M sodium borate buffer pH 9 (1 mL) and incubated at room temperature for 5 h. The reaction mixture was loaded on a NAP-10 column, and the Df-Ac-thio-trastuzumab was eluted with 1.5 mL of 0.25 M sodium acetate buffer to obtain the product at a concentration of 3.2 mg/mL. MS ESI (m/z): found light chain 23440.1, heavy chain 51228.1 (Figure 23, B).

### Example 19 Df-Ac-thio-trastuzumab using Df-Iac

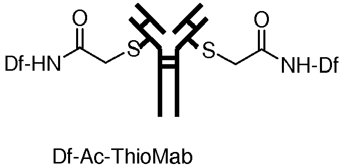

The 11 mM stock solution of the bifunctional chelator was prepared by dissolving Df-Iac (8 mg, 11 µmol) in DMSO (1 mL), the stock solution was then aliquoted and stored at - 80°C (Figure 21). The thio-trastuzumab solution (3.2 mL) was adjusted to pH 9 with the addition of 0.5 mL of 0.1 M sodium carbonate. An aliquot of the stock solution (110 µL, 1.20 µmol) was then added to the solution of thio-trastuzumab (16 mg, 110 nmol) in 50 mM Tris, 150 mM NaCl, 0.0125 M sodium carbonate buffer with pH 9 (4 mL) and incubated at room temperature for 2 h. The solution was then buffer exchanged on an Amicon Ultra-4 filter into 0.25 M sodium acetate buffer to obtain 1 mL of Df-Ac-thio-trastuzumab conjugate solution at a concentration of 8 mg/mL. MS ESI (m/z): found light chain 23440.1, heavy chain 51228.3 (Figure 23, C).

### Example 20 General procedure for preparation of ⁸⁹Zr chelates

The solution of ⁸⁹Zr(IV) oxalate (2-4 mCi, 100 µL) in 1 M oxalic acid was mixed with 2 M solution of Na₂CO₃ (45 µL) and incubated at room temperature for 3 min after which 0.5 M HEPES buffer (0.15 mL) was added (Figure 22). The Df-thio-trastuzumab conjugate (1 mg, 7 nmol) was diluted with 0.25 M sodium acetate/0.5% gentisic acid to a final volume of 0.356 mL and added to the ⁸⁹Zr solution. Finally, a second portion of HEPES buffer (0.350 mL) was added resulting in 1 mL of total volume. The mixture was incubated at room temperature for 1 h. To remove free ⁸⁹Zr the radiolabeled protein was purified using a NAP-10 desalting column. The NAP-10 column was equilibrated with 20 mL of 0.25M sodium acetate/0.5% gentisic acid. The reaction mixture was loaded on the NAP-10 column, and the ⁸⁹Zr-Df-thio-trastuzumab was eluted with 1.5 mL of 0.25M sodium acetate/0.5% gentisic acid buffer (1.5 mL). If needed, the ⁸⁹Zr-Df-thio-trastuzumab was concentrated using Amicon Ultra-4 filter to the desired volume. The product was analyzed by SEC HPLC (System A).

### Example 21 In vitro serum stability

A solution of ⁸⁹Zr-Df-thio-trastuzumab conjugate 0.5-1.5 mCi (1 mg) in 0.25 M sodium acetate/0.5% gentisic acid buffer (0.1 mL) was added to fresh mouse serum (0.9 mL) and incubated at 37°C for 0-96 h. Samples (20 µL) of the serum solution were analyzed using SEC HPLC (System A), with results shown in Figure 24.

### Example 22 Animal models

Beige nude XID mice of age 6-8 weeks were obtained from Harlan Sprague Dawley (Livermore, CA). Three days prior to cell inoculation, the mice were implanted (s. c., left flank) with a 0.36 mg 60-day sustained release 17β-estradiol pellets (Innovative Research of America) to maintain serum estrogen level. Mice were inoculated in the mammary fat pad with 5 × 10⁶ BT474M1 cells in 50% phenol red-free matrigel. BT474M1 is a subclone of human breast tumor cell line BT474 that was obtained from California Pacific Medical Center. Animal care and treatment followed protocols approved by Genentech's Institutioned Animal Care and Use Committee which is accredited by the Association for Assessment and Accreditation of Laboratory Animal Care (AAALAC).

### Example 23 MicroPET Imaging

Mice were anesthetized with approx. 3% sevoflurane to effect and injected i.v. via the tail vein with approximately 0.1 mCi of ⁸⁹Zr-radiolabeled trastuzumab in isotonic solution (100-130 µL) and returned to the cage for recovery. The PET imaging (Figure 25) was performed on an Inveon PET/CT scanner at 1, 24, 96 and 144 h post tracer injection as follows. Animals anesthetized with sevoflurane were placed head-first, prone position on the scanner bed and static 15 or 30 min scans were acquired. Body temperature was measured by a rectal probe and maintained with warm air. Full-body iterative image reconstructions were obtained using maximum a posteriori algorithm (MAP, hyperparameter beta (β) 0.05) and corrected for signal attenuation using the tissue density obtained from CT. Projections were created with ASIPro software (Siemens Preclinical Solutions) and used to obtain quantitative activity levels in each organ of interest using region-of-interest analysis.

Statistical analysis: The plots of Figure 26 were constructed with R software version 2.4.1 (R Foundation for Statistical Computing, Vienna, Austria). Statistical significance was determined using a two-tailed Student's t-test or ANOVA and P values of less than 0.05 were considered significant; data are presented as mean ± s.d. if not stated otherwise.

## Claims

1. A method of making a zirconium-labelled, cysteine-engineered antibody comprising a cysteine engineered antibody (Ab) conjugated through a free cysteine amino acid to a linker (L) and a zirconium complex (Z), having Formula I:
Ab-(L-Z)ₚ I
where p is 1 to 2;
the method comprising a first method of making a desferrioxamine-labelled, cysteine-engineered antibody comprising a cysteine engineered antibody (Ab) conjugated through a free cysteine amino acid to a linker (L) and a desferrioxamine moiety (Df), having Formula II:
Ab-(L-Df)ₚ II
wherein L-Df is selected from: 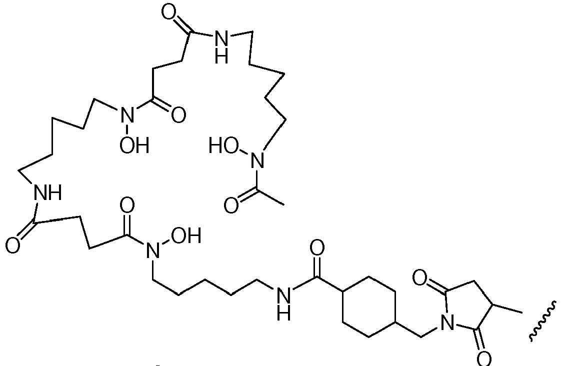 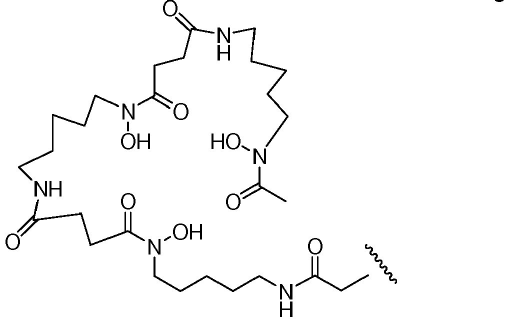 where the wavy line indicates the attachment to the antibody (Ab); and
p is 1 to 2;
the first method comprising reacting a composition selected from the structures: 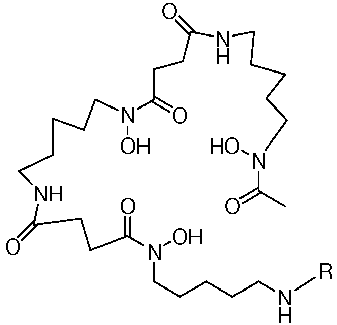 wherein R is selected from: 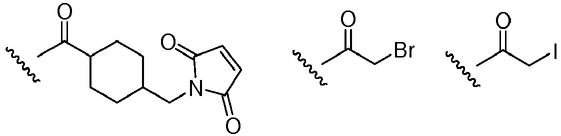 with a cysteine-engineered antibody having one or more free cysteine amino acids,
whereby the desferrioxamine-labelled, cysteine-engineered antibody is formed;
the method further comprising complexing a zirconium reagent with the desferrioxamine-labelled, cysteine-engineered antibody comprising a cysteine engineered antibody (Ab) conjugated through a free cysteine amino acid to a linker (L) and a desferrioxamine moiety (Df), having Formula II:
Ab-(L-Df)ₚ II
wherein L-Df is selected from: 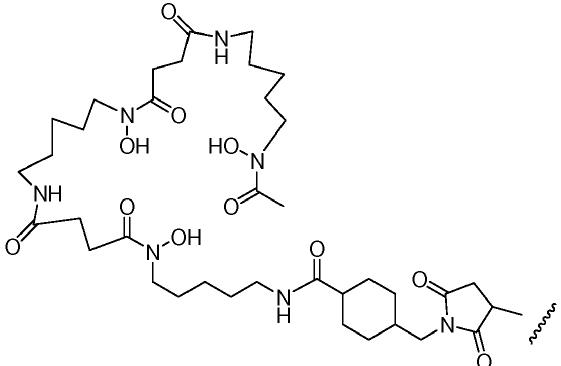 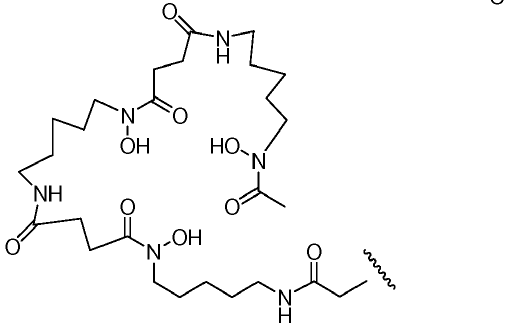 where the wavy line indicates the attachment to the antibody (Ab); and
p is 1 to 2;
whereby the zirconium-labelled, cysteine-engineered antibody is formed;
wherein the cysteine engineered antibody is prepared by a process comprising replacing one or more amino acid residues of a parent antibody with the one or more free cysteine amino acids, where the parent antibody selectively binds to an antigen and the cysteine engineered antibody selectively binds to the same antigen as the parent antibody,
wherein the cysteine engineered antibody comprises a sequence in the heavy chain selected from SEQ ID NO: 13:
LVTVSSCSTKGPS SEQ ID NO:13
where the cysteine in SEQ ID NO: 13 is the free cysteine amino acid.

2. The method of claim 1 wherein the zirconium reagent is ⁸⁹zirconium oxalate.

## Patentansprüche

1. Verfahren zur Herstellung eines Zirkonium-markierten, Cystein-modifizierten Antikörpers, umfassend einen Cystein-modifizierten Antikörper (Ak), der durch eine freie Cystein-Aminosäure an einen Linker (L) und einen Zirkonium-Komplex (Z) konjugiert ist, der die folgende Formel I aufweist:
Ak-(L-Z)ₚ I
wobei p 1 bis 2 ist;
wobei das Verfahren ein erstes Verfahren zur Herstellung eines Desferrioxamin-markierten, Cystein-modifizierten Antikörpers, umfassend einen Cystein-modifizierten Antikörper (Ak), der durch eine freie Cystein-Aminosäure an einen Linker (L) und eine Desferrioxamin-Gruppierung (Df) konjugiert ist, umfasst, der die folgende Formel II aufweist:
Ak-(L-Df)ₚ II
wobei L-Df aus den Folgenden ausgewählt ist: 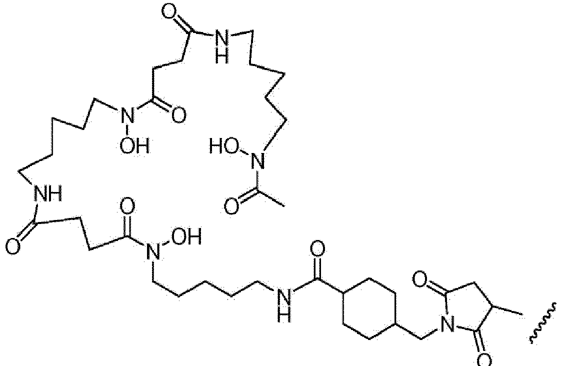 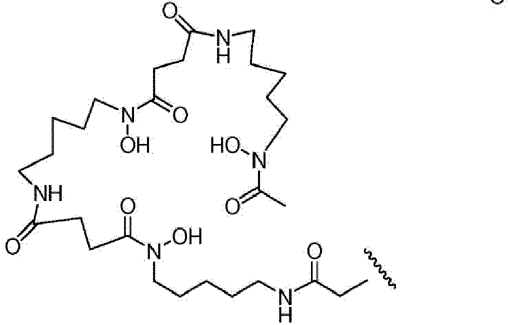 wobei die Wellenlinie für die Bindung an den Antikörper (Ak) steht; und
p 1 bis 2 ist;
wobei das erste Verfahren das Umsetzen einer Zusammensetzung, ausgewählt aus den folgenden Strukturen: 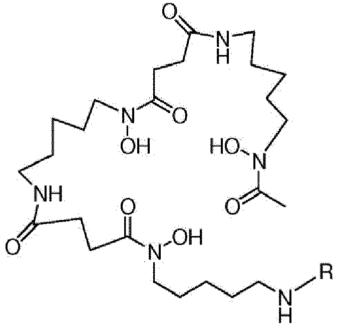 wobei R aus den Folgenden ausgewählt ist: 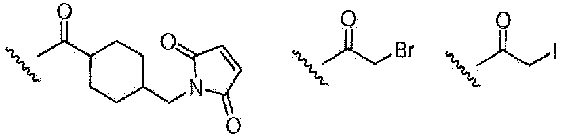 mit einem Cystein-modifizierten Antikörper mit einer oder mehreren freien Cystein-Aminosäuren umfasst,
wodurch der Desferrioxamin-markierte, Cystein-modifizierte Antikörper gebildet wird;
wobei das Verfahren ferner das Komplexieren eines Zirkonium-Reagens mit dem Desferrioxamin-markierten, Cystein-modifizierten Antikörper, umfassend einen Cystein-modifizierten Antikörper (Ak), der durch eine freie Cystein-Aminosäure an einen Linker (L) und eine Desferrioxamin-Gruppierung (Df) konjugiert ist, umfasst, der die Formel II aufweist:
Ak-(L-Df)ₚ II
wobei L-Df aus den Folgenden ausgewählt ist: 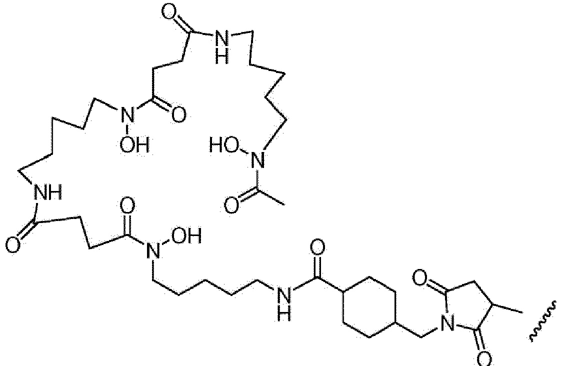 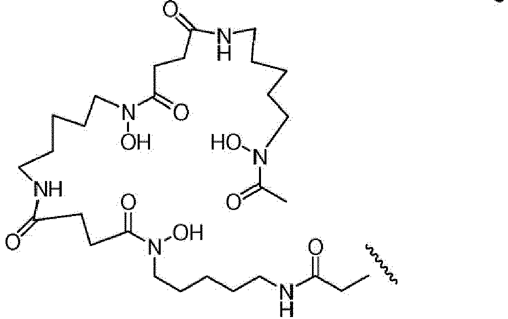 wobei die Wellenlinie für die Bindung an den Antikörper (Ak) steht; und
p 1 bis 2 ist;
wodurch der Zirkonium-markierte, Cystein-modifizierte Antikörper gebildet wird;
wobei der Cystein-modifizierte Antikörper nach einem Verfahren hergestellt wird, welches das Ersetzen eines oder mehrerer Aminosäurereste eines Ausgangsantikörpers durch die eine oder mehrere der freien Cystein-Aminosäuren umfasst, wobei der Ausgangsantikörper selektiv an ein Antigen bindet, und der Cystein-modifizierte Antikörper selektiv an dasselbe Antigen wie der Ausgangsantikörper bindet,
wobei der Cystein-modifizierte Antikörper eine Sequenz in der Schwerkette aufweist, die aus Seq.-ID Nr. 13 ausgewählt ist:
LVTVSSCSTKGPS Seq.-ID Nr. 13
wobei das Cystein in Seq.-ID Nr. 13 die freie Cystein-Aminosäure ist.

2. Verfahren nach Anspruch 1, wobei das Zirkonium-Reagens ⁸⁹Zirkoniumoxalat ist.

## Revendications

1. Procédé de préparation d'un anticorps modifié par cystéine, marqué par le zirconium comprenant un anticorps (Ab) modifié par cystéine conjugué par le biais d'un acide aminé libre de la cystéine à un lieur (L) et à un complexe de zirconium (Z) répondant à la formule I :
Ab-(L-Z)ₚ I
dans laquelle p représente 1 à 2 ;
le procédé comprenant un premier procédé de préparation d'un anticorps modifié par cystéine, marqué par la déféroxamine comprenant un anticorps (Ab) modifié par cystéine conjugué par le biais d'un acide aminé libre de la cystéine à un lieur (L) et à un fragment de déféroxamine (Df) répondant à la formule II :
Ab-(L-Df)ₚ II
dans laquelle L-Df est choisi parmi : 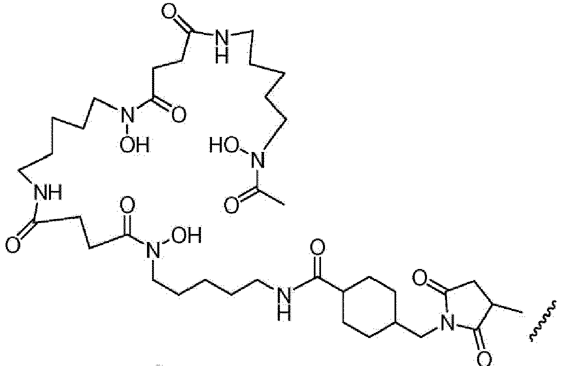 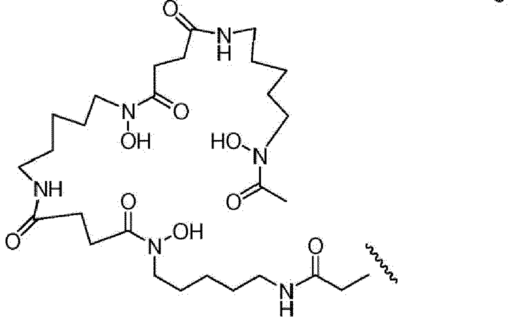 où la ligne ondulée indique l'attachement à l'anticorps (Ab) ; et
p représente 1 à 2 ;
le premier procédé comprenant la mise en réaction d'une composition choisie parmi les structures : 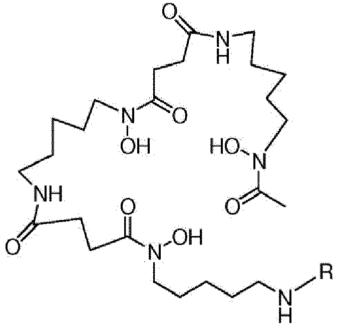 où R est choisi parmi : 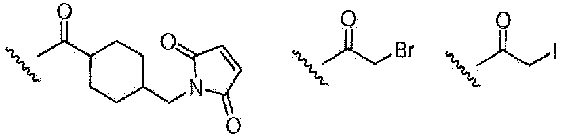 avec un anticorps modifié par cystéine ayant un ou plusieurs acides aminés libres de la cystéine,
l'anticorps modifié par cystéine, marqué par la déféroxamine étant ainsi formé ;
le procédé comprenant en outre la complexation d'un réactif à base de zirconium avec l'anticorps modifié par cystéine, marqué par la déféroxamine comprenant un anticorps (Ab) modifié par cystéine conjugué par le biais d'un acide aminé libre de la cystéine à un lieur (L) et à un fragment de déféroxamine (Df) répondant à la formule II :
Ab-(L-Df)ₚ II
dans laquelle L-Df est choisi parmi : 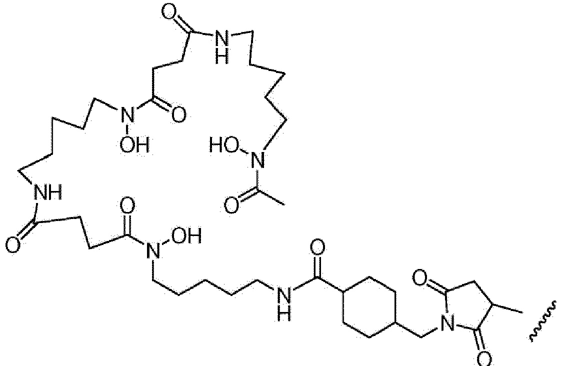 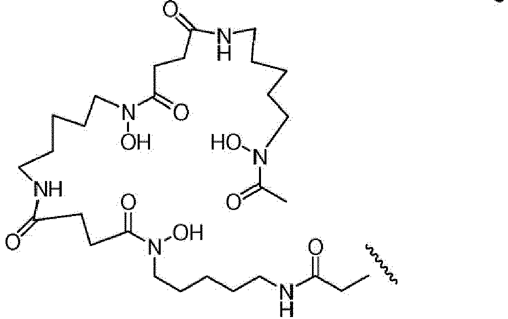 où la ligne ondulée indique l'attachement à l'anticorps (Ab) ; et
p représente 1 à 2 ;
l'anticorps modifié par cystéine, marqué par le zirconium étant ainsi formé ;
l'anticorps modifié par cystéine étant préparé par un procédé comprenant le remplacement d'un ou plusieurs résidus d'acides aminés d'un anticorps parent par le ou les acides aminés libres de la cystéine, l'anticorps parent se liant sélectivement à un antigène et l'anticorps modifié par cystéine se liant sélectivement au même antigène que l'anticorps parent,
l'anticorps modifié par cystéine comprenant une séquence dans la chaîne lourde choisie parmi la SEQ ID NO: 13 :
LVTVSSCSTKGPS SEQ ID NO: 13
la cystéine dans la SEQ ID NO: 13 étant l'acide aminé libre de la cystéine.

2. Procédé selon la revendication 1, dans lequel le réactif à base de zirconium est l'oxalate de ⁸⁹zirconium.


### Sequence Listing

<110> GENENTECH, INC. et al.
<120> ZIRCONIUM-RADIOLABELED, CYSTEINE ENGINEERED ANTIBODY CONJUGATES
<130> P2066R1C1P1-WO
<140>
   <141>
<150> US 12/612,912
   <151> 2009-11-05
<150> US 12/399,241
   <151> 2009-03-06
<150> US 11/233,258
   <151> 2005-09-22
<150> US 60/696,353
   <151> 2005-06-30
<150> US 60/612,468
   <151> 2004-09-23
<160> 49
<210> 1
   <211> 30
   <212> PRT
   <213> Artificial sequence
<220>
   <223> chemically synthesized
<400> 1
   Cys Asp Lys Thr His Thr Gly Gly Gly Ser Gln Arg Leu Met Glu
   1               5                   10                  15
   Asp Ile Cys Leu Pro Arg Trp Gly Cys Leu Trp Glu Asp Asp Phe
                   20                  25                  30
<210> 2
   <211> 20
   <212> PRT
   <213> Artificial sequence
<220>
   <223> chemically synthesized
<400> 2
   Gln Arg Leu Met Glu Asp Ile Cys Leu Pro Arg Trp Gly Cys Leu
   1               5                   10                  15
   Trp Glu Asp Asp Phe
                   20
<210> 3
   <211> 20
   <212> PRT
   <213> Artificial sequence
<220>
   <223> chemically synthesized
<400> 3
   Gln Arg Leu Ile Glu Asp Ile Cys Leu Pro Arg Trp Gly Cys Leu
   1               5                   10                  15
   Trp Glu Asp Asp Phe
                   20
<210> 4
   <211> 18
   <212> PRT
   <213> Artificial sequence
<220>
   <223> chemically synthesized
<400> 4
   Arg Leu Ile Glu Asp Ile Cys Leu Pro Arg Trp Gly Cys Leu Trp
   1 5 10 15
   Glu Asp Asp
<210> 5
   <211> 11
   <212> PRT
   <213> Artificial sequence
<220>
   <223> chemically synthesized
<400> 5
   Asp Ile Cys Leu Pro Arg Trp Gly Cys Leu Trp
   5 10
<210> 6
   <211> 450
   <212> PRT
   <213> Artificial sequence
<220>
   <223> artificial protein
<400> 6
   Glu Val Gln Leu Val Glu Ser Gly Gly Gly Leu Val Gln Pro Gly
   1 5 10 15
   Gly Ser Leu Arg Leu Ser Cys Ala Ala Ser Gly Phe Asn Ile Lys
   20 25 30
   Asp Thr Tyr Ile His Trp Val Arg Gln Ala Pro Gly Lys Gly Leu
   35 40 45
   Glu Trp Val Ala Arg Ile Tyr Pro Thr Asn Gly Tyr Thr Arg Tyr
   50 55 60
   Ala Asp Ser Val Lys Gly Arg Phe Thr Ile Ser Ala Asp Thr Ser
   65 70 75
   Lys Asn Thr Ala Tyr Leu Gln Met Asn Ser Leu Arg Cys Glu Asp
   80 85 90
   Thr Ala Val Tyr Tyr Cys Ser Arg Trp Gly Gly Asp Gly Phe Tyr
   95 100 105
   Ala Met Asp Tyr Trp Gly Gln Gly Thr Leu Val Thr Val Ser Ser
   110 115 120
   Ala Ser Thr Lys Gly Pro Ser Val Phe Pro Leu Ala Pro Ser Ser
   125 130 135
   Lys Ser Thr Ser Gly Gly Thr Ala Ala Leu Gly Cys Leu Val Lys
   140 145 150
   Asp Tyr Phe Pro Glu Pro Val Thr Val Ser Trp Asn Ser Gly Ala
   155 160 165
   Leu Thr Ser Gly Val His Thr Phe Pro Ala Val Leu Gln Ser Ser
   170 175 180
   Gly Leu Tyr Ser Leu Ser Ser Val Val Thr Val Pro Ser Ser Ser
   185 190 195
   Leu Gly Thr Gln Thr Tyr Ile Cys Asn Val Asn His Lys Pro Ser
   200 205 210
   Asn Thr Lys Val Asp Lys Lys Val Glu Pro Lys Ser Cys Asp Lys
   215 220 225
   Thr His Thr Cys Pro Pro Cys Pro Ala Pro Glu Leu Leu Gly Gly
   230 235 240
   Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser
   260 265 270
   His Glu Asp Pro Glu Val Lys Phe Asn Trp Tyr Val Asp Gly Val
   275 280 285
   Glu Val His Asn Ala Lys Thr Lys Pro Arg Glu Glu Gln Tyr Asn
   290 295 300
   Ser Thr Tyr Arg Val Val Ser Val Leu Thr Val Leu His Gln Asp
   305 310 315
   Trp Leu Asn Gly Lys Glu Tyr Lys Cys Lys Val Ser Asn Lys Ala
   320 325 330
   Leu Pro Ala Pro Ile Glu Lys Thr Ile Ser Lys Ala Lys Gly Gln
   335 340 345
   Pro Arg Glu Pro Gln Val Tyr Thr Leu Pro Pro Ser Arg Glu Glu
   350 355 360
   Met Thr Lys Asn Gln Val Ser Leu Thr Cys Leu Val Lys Gly Phe
   365 370 375
   Tyr Pro Ser Asp Ile Ala Val Glu Trp Glu Ser Asn Gly Gln Pro
   380 385 390
   Glu Asn Asn Tyr Lys Thr Thr Pro Pro Val Leu Asp Ser Asp Gly
   395 400 405
   Ser Phe Phe Leu Tyr Ser Lys Leu Thr Val Asp Lys Ser Arg Trp
   410 415 420
   Gln Gln Gly Asn Val Phe Ser Cys Ser Val Met His Glu Ala Leu
   425 430 435
   His Asn His Tyr Thr Gln Lys Ser Leu Ser Leu Ser Pro Gly Lys
   440 445 450
<210> 7
   <211> 450
   <212> PRT
   <213> Artificial sequence
<220>
   <223> chemically synthesized
<400> 7
   Glu Val Gln Leu Val Glu Ser Gly Gly Gly Leu Val Gln Pro Gly
   1               5                   10                  15
   Gly Ser Leu Arg Leu Ser Cys Ala Ala Ser Gly Phe Asn Ile Lys
                   20                  25                  30
   Asp Thr Tyr Ile His Trp Val Arg Gln Ala Pro Gly Lys Gly Leu
                   35                  40                  45
   Glu Trp Val Ala Arg Ile Tyr Pro Thr Asn Gly Tyr Thr Arg Tyr
                   50                  55                  60
   Ala Asp Ser Val Lys Gly Arg Phe Thr Ile Ser Ala Asp Thr Ser
                   65                  70                  75
   Lys Asn Thr Ala Tyr Leu Gln Met Asn Ser Leu Arg Ala Glu Asp
                   80                  85                  90
   Thr Ala Val Tyr Tyr Cys Ser Arg Trp Gly Gly Asp Gly Phe Tyr
                   95                  100                 105
   Ala Met Asp Tyr Trp Gly Gln Gly Thr Leu Val Thr Val Ser Ser
                   110                 115                 120
   Cys Ser Thr Lys Gly Pro Ser Val Phe Pro Leu Ala Pro Ser Ser
                   125                 130                 135
   Lys Ser Thr Ser Gly Gly Thr Ala Ala Leu Gly Cys Leu Val Lys
                   140                 145                 150
   Asp Tyr Phe Pro Glu Pro Val Thr Val Ser Trp Asn Ser Gly Ala
                   155                 160                 165
   Leu Thr Ser Gly Val His Thr Phe Pro Ala Val Leu Gln Ser Ser
                   170                 175                 180
   Gly Leu Tyr Ser Leu Ser Ser Val Val Thr Val Pro Ser Ser Ser
                   185                 190                 195
   Leu Gly Thr Gln Thr Tyr Ile Cys Asn Val Asn His Lys Pro Ser
                   200                 205                 210
   Asn Thr Lys Val Asp Lys Lys Val Glu Pro Lys Ser Cys Asp Lys
                   215                 220                 225
   Thr His Thr Cys Pro Pro Cys Pro Ala Pro Glu Leu Leu Gly Gly
                   230                 235                 240
   Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser
   260 265 270
   His Glu Asp Pro Glu Val Lys Phe Asn Trp Tyr Val Asp Gly Val
   275 280 285
   Glu Val His Asn Ala Lys Thr Lys Pro Arg Glu Glu Gln Tyr Asn
   290 295 300
   Ser Thr Tyr Arg Val Val Ser Val Leu Thr Val Leu His Gln Asp
   305 310 315
   Trp Leu Asn Gly Lys Glu Tyr Lys Cys Lys Val Ser Asn Lys Ala
   320 325 330
   Leu Pro Ala Pro Ile Glu Lys Thr Ile Ser Lys Ala Lys Gly Gln
   335 340 345
   Pro Arg Glu Pro Gln Val Tyr Thr Leu Pro Pro Ser Arg Glu Glu
   350 355 360
   Met Thr Lys Asn Gln Val Ser Leu Thr Cys Leu Val Lys Gly Phe
   365 370 375
   Tyr Pro Ser Asp Ile Ala Val Glu Trp Glu Ser Asn Gly Gln Pro
   380 385 390
   Glu Asn Asn Tyr Lys Thr Thr Pro Pro Val Leu Asp Ser Asp Gly
   395 400 405
   Ser Phe Phe Leu Tyr Ser Lys Leu Thr Val Asp Lys Ser Arg Trp
   410 415 420
   Gln Gln Gly Asn Val Phe Ser Cys Ser Val Met His Glu Ala Leu
   425 430 435
   His Asn His Tyr Thr Gln Lys Ser Leu Ser Leu Ser Pro Gly Lys
   440 445 450
<210> 8
   <211> 214
   <212> PRT
   <213> Artificial sequence
<220>
   <223> chemically synthesized
<400> 8
   Asp Ile Gln Met Thr Gln Ser Pro Ser Ser Leu Ser Ala Ser Val
   1 5 10 15
   Gly Asp Arg Val Thr Ile Thr Cys Arg Ala Ser Gln Asp Val Asn
   20 25 30
   Thr Ala Val Ala Trp Tyr Gln Gln Lys Pro Gly Lys Ala Pro Lys
   35 40 45
   Leu Leu Ile Tyr Ser Ala Ser Phe Leu Tyr Ser Gly Val Pro Ser
   50 55 60
   Arg Phe Ser Gly Ser Arg Ser Gly Thr Asp Phe Thr Leu Thr Ile
   65 70 75
   Ser Ser Leu Gln Pro Glu Asp Phe Ala Thr Tyr Tyr Cys Gln Gln
   80 85 90
   His Tyr Thr Thr Pro Pro Thr Phe Gly Gln Gly Thr Lys Val Glu
   95 100 105
   Ile Lys Arg Thr Cys Ala Ala Pro Ser Val Phe Ile Phe Pro Pro
   110 115 120
   Ser Asp Glu Gln Leu Lys Ser Gly Thr Ala Ser Val Val Cys Leu
   125 130 135
   Leu Asn Asn Phe Tyr Pro Arg Glu Ala Lys Val Gln Trp Lys Val
   140 145 150
   Asp Asn Ala Leu Gln Ser Gly Asn Ser Gln Glu Ser Val Thr Glu
   155 160 165
   Gln Asp Ser Lys Asp Ser Thr Tyr Ser Leu Ser Ser Thr Leu Thr
   170 175 180
   Leu Ser Lys Ala Asp Tyr Glu Lys His Lys Val Tyr Ala Cys Glu
   185 190 195
   Val Thr His Gln Gly Leu Ser Ser Pro Val Thr Lys Ser Phe Asn
   200 205 210
   Arg Gly Glu Cys
<210> 9
   <211> 10
   <212> PRT
   <213> Artificial sequence
<220>
   <223> chemically synthesized
<400> 9
   Trp Val Arg Gln Cys Pro Gly Lys Gly Leu
   5 10
<210> 10
   <211> 10
   <212> PRT
   <213> Artificial sequence
<220>
   <223> chemically synthesized
<400> 10
   Asn Ser Leu Arg Cys Glu Asp Thr Ala Val
   1               5                   10
<210> 11
   <211> 13
   <212> PRT
   <213> Artificial sequence
<220>
   <223> chemically synthesized
<400> 11
   Leu Val Thr Val Cys Ser Ala Ser Thr Lys Gly Pro Ser
                   5                   10
<210> 12
   <211> 13
   <212> PRT
   <213> Artificial sequence
<220>
   <223> chemically synthesized
<400> 12
   Leu Val Thr Val Ser Cys Ala Ser Thr Lys Gly Pro Ser
                   5                   10
<210> 13
   <211> 13
   <212> PRT
   <213> Artificial sequence
<220>
   <223> chemically synthesized
<400> 13
   Leu Val Thr Val Ser Ser Cys Ser Thr Lys Gly Pro Ser
                   5                   10
<210> **14**
   <211> 13
   <212> PRT
   <213> Artificial sequence
<220>
   <223> chemically synthesized
<400> 14
   Leu Val Thr Val Ser Ser Ala Cys Thr Lys Gly Pro Ser
   5 10
<210> 15
   <211> 14
   <212> PRT
   <213> Artificial sequence
<220>
   <223> chemically synthesized
<400> 15
   His Thr Phe Pro Cys Val Leu Gln Ser Ser Gly Leu Tyr Ser
   5 10
<210> 16
   <211> 14
   <212> PRT
   <213> Artificial sequence
<220>
   <223> chemically synthesized
<400> 16
   His Thr Phe Pro Ala Val Leu Gln Cys Ser Gly Leu Tyr Ser
   5 10
<210> 17
   <211> 11
   <212> PRT
   <213> Artificial sequence
<220>
   <223> chemically synthesized
<400> 17
   Ser Leu Ser Ala Ser Cys Gly Asp Arg Val Thr
   5 10
<210> 18
   <211> 11
   <212> PRT
   <213> Artificial sequence
<220>
   <223> chemically synthesized
<400> 18
   Gln Lys Pro Gly Lys Cys Pro Lys Leu Leu Ile
   5 10
<210> 19
   <211> 11
   <212> PRT
   <213> Artificial sequence
<220>
   <223> chemically synthesized
<400> 19
   Glu Ile Lys Arg Thr Cys Ala Ala Pro Ser Val
                   1               5               10
<210> 20
   <211> 12
   <212> PRT
   <213> Artificial sequence
<220>
   <223> chemically synthesized
<400> 20
   Thr Cys Ala Ala Pro Cys Val Phe Ile Phe Pro Pro
                       5                   10
<210> 21
   <211> 11
   <212> PRT
   <213> Artificial sequence
<220>
   <223> chemically synthesized
<400> 21
   Phe Ile Phe Pro Pro Cys Asp Glu Gln Leu Lys
                       5                   10
<210> 22
   <211> 11
   <212> PRT
   <213> Artificial sequence
<220>
   <223> chemically synthesized
<400> 22
   Asp Glu Gln Leu Lys Cys Gly Thr Ala Ser Val
                       5                   10
<210> 23
   <211> 11
   <212> PRT
   <213> Artificial sequence
<220>
   <223> chemically synthesized
<400> 23
   Phe Tyr Pro Arg Glu Cys Lys Val Gln Trp Lys
   5 10
<210> 24
   <211> 11
   <212> PRT
   <213> Artificial sequence
<220>
   <223> chemically synthesized
<400> 24
   Trp Lys Val Asp Asn Cys Leu Gln Ser Gly Asn
   5 10
<210> 25
   <211> 11
   <212> PRT
   <213> Artificial sequence
<220>
   <223> chemically synthesized
<400> 25
   Ala Leu Gln Ser Gly Cys Ser Gln Glu Ser Val
   5 10
<210> 26
   <211> 11
   <212> PRT
   <213> Artificial sequence
<220>
   <223> chemically synthesized
<400> 26
   Val Thr Glu Gln Asp Cys Lys Asp Ser Thr Tyr
   5 10
<210> 27
   <211> 11
   <212> PRT
   <213> Artificial sequence
<220>
   <223> chemically synthesized
<400> 27
   Gly Leu Ser Ser Pro Cys Thr Lys Ser Phe Asn
   5 10
<210> 28
   <211> 450
   <212> PRT
   <213> Artificial sequence
<220>
   <223> chemically synthesized
<400> 28
   Glu Val Gln Leu Val Glu Ser Gly Gly Gly Leu Val Gln Pro Gly
   1               5                   10                  15
   Gly Ser Leu Arg Leu Ser Cys Ala Ala Ser Gly Tyr Thr Phe Thr
                   20                  25                  30
   Ser Tyr Trp Met His Trp Val Arg Gln Ala Pro Gly Lys Gly Leu
                   35                  40                  45
   Glu Trp Val Gly Phe Ile Asn Pro Ser Thr Gly Tyr Thr Asp Tyr
                   50                  55                  60
   Asn Gln Lys Phe Lys Asp Arg Phe Thr Ile Ser Ala Asp Thr Ser
                   65                  70                  75
   Lys Asn Thr Ala Tyr Leu Gln Met Asn Ser Leu Arg Ala Glu Asp
                   80                  85                  90
   Thr Ala Val Tyr Tyr Cys Thr Arg Arg Pro Lys Ile Pro Arg His
                   95                  100                 105
   Ala Asn Val Phe Trp Gly Gln Gly Thr Leu Val Thr Val Ser Ser
                   110                 115                 120
   Cys Ser Thr Lys Gly Pro Ser Val Phe Pro Leu Ala Pro Ser Ser
                   125                 130                 135
   Lys Ser Thr Ser Gly Gly Thr Ala Ala Leu Gly Cys Leu Val Lys
                   140                 145                 150
   Asp Tyr Phe Pro Glu Pro Val Thr Val Ser Trp Asn Ser Gly Ala
                   155                 160                 165
   Leu Thr Ser Gly Val His Thr Phe Pro Ala Val Leu Gln Ser Ser
                   170                 175                 180
   Gly Leu Tyr Ser Leu Ser Ser Val Val Thr Val Pro Ser Ser Ser
                   185                 190                 195
   Leu Gly Thr Gln Thr Tyr Ile Cys Asn Val Asn His Lys Pro Ser
                   200                 205                 210
   Asn Thr Lys Val Asp Lys Lys Val Glu Pro Lys Ser Cys Asp Lys
                   215                 220                 225
   Thr His Thr Cys Pro Pro Cys Pro Ala Pro Glu Leu Leu Gly Gly
                   230                 235                 240
   Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser
                   260                 265                 270
   His Glu Asp Pro Glu Val Lys Phe Asn Trp Tyr Val Asp Gly Val
                   275                 280                 285
   Glu Val His Asn Ala Lys Thr Lys Pro Arg Glu Glu Gln Tyr Asn
                   290                 295                 300
   Ser Thr Tyr Arg Val Val Ser Val Leu Thr Val Leu His Gln Asp
   305 310 315
   Trp Leu Asn Gly Lys Glu Tyr Lys Cys Lys Val Ser Asn Lys Ala
   320 325 330
   Leu Pro Ala Pro Ile Glu Lys Thr Ile Ser Lys Ala Lys Gly Gln
   335 340 345
   Pro Arg Glu Pro Gln Val Tyr Thr Leu Pro Pro Ser Arg Glu Glu
   350 355 360
   Met Thr Lys Asn Gln Val Ser Leu Thr Cys Leu Val Lys Gly Phe
   365 370 375
   Tyr Pro Ser Asp Ile Ala Val Glu Trp Glu Ser Asn Gly Gln Pro
   380 385 390
   Glu Asn Asn Tyr Lys Thr Thr Pro Pro Val Leu Asp Ser Asp Gly
   395 400 405
   Ser Phe Phe Leu Tyr Ser Lys Leu Thr Val Asp Lys Ser Arg Trp
   410 415 420
   Gln Gln Gly Asn Val Phe Ser Cys Ser Val Met His Glu Ala Leu
   425 430 435
   His Asn His Tyr Thr Gln Lys Ser Leu Ser Leu Ser Pro Gly Lys
   440 445 450
<210> 29
   <211> 25
   <212> PRT
   <213> Artificial sequence
<220>
   <223> chemically synthesized
<400> 29
   His Glu Asp Pro Glu Cys Lys Phe Asn Trp Tyr Val Asp Gly Val
   1 5 10 15
   Glu Val His Asn Ala Lys Thr Lys Pro Arg
   20 25
<210> 30
   <211> 25
   <212> PRT
   <213> Artificial sequence
<220>
   <223> chemically synthesized
<400> 30
   His Glu Asp Pro Glu Val Lys Phe Asn Trp Tyr Cys Asp Gly Val
   1               5                   10                  15
   Glu Val His Asn Ala Lys Thr Lys Pro Arg
                   20                  25
<210> 31
   <211> 25
   <212> PRT
   <213> Artificial sequence
<220>
   <223> chemically synthesized
<400> 31
   His Glu Asp Pro Glu Val Lys Phe Asn Trp Tyr Val Asp Gly Cys
   1               5                   10                  15
   Glu Val His Asn Ala Lys Thr Lys Pro Arg
                   20                  25
<210> 32
   <211> 25
   <212> PRT
   <213> Artificial sequence
<220>
   <223> chemically synthesized
<400> 32
   His Glu Asp Pro Glu Val Lys Phe Asn Trp Tyr Val Asp Gly Val
   1               5                   10                  15
   Glu Cys His Asn Ala Lys Thr Lys Pro Arg
                   20                  25
<210> 33
   <211> 25
   <212> PRT
   <213> Artificial sequence
<220>
   <223> chemically synthesized
<400> 33
   His Glu Asp Pro Glu Val Lys Phe Asn Trp Tyr Val Asp Gly Val
   1               5                   10                  15
   Glu Val His Asn Cys Lys Thr Lys Pro Arg
                   20                  25
<210> 34
   <211> 11
   <212> PRT
   <213> Artificial sequence
<220>
   <223> chemically synthesized
<400> 34
   Tyr Lys Cys Lys Val Cys Asn Lys Ala Leu Pro
   5 10
<210> 35
   <211> 13
   <212> PRT
   <213> Artificial sequence
<220>
   <223> artificial protein
<400> 35
   Ile Glu Lys Thr Ile Cys Lys Ala Lys Gly Gln Pro Arg
   5 10
<210> 36
   <211> 13
   <212> PRT
   <213> Artificial sequence
<220>
   <223> chemically synthesized
<400> 36
   Ile Glu Lys Thr Ile Ser Lys Cys Lys Gly Gln Pro Arg
   5 10
<210> 37
   <211> 11
   <212> PRT
   <213> Artificial sequence
<220>
   <223> chemically synthesized
<400> 37
   Lys Gly Phe Tyr Pro Cys Asp Ile Ala Val Glu
   5 10
<210> 38
   <211> 11
   <212> PRT
   <213> Artificial sequence
<220>
   <223> chemically synthesized
<400> 38
   Pro Pro Val Leu Asp Cys Asp Gly Ser Phe Phe
   5 10
<210> 39
   <211> 446
   <212> PRT
   <213> Artificial sequence
<220>
   <223> chemically synthesized
<400> 39
   Asp Val Gln Leu Gln Glu Ser Gly Pro Gly Leu Val Asn Pro Ser
   1 5 10 15
   Gln Ser Leu Ser Leu Thr Cys Thr Val Thr Gly Tyr Ser Ile Thr
   20 25 30
   Asn Asp Tyr Ala Trp Asn Trp Ile Arg Gln Phe Pro Gly Asn Lys
   35 40 45
   Leu Glu Trp Met Gly Tyr Ile Asn Tyr Ser Gly Tyr Thr Thr Tyr
   50 55 60
   Asn Pro Ser Leu Lys Ser Arg Ile Ser Ile Thr Arg Asp Thr Ser
   65 70 75
   Lys Asn Gln Phe Phe Leu His Leu Asn Ser Val Thr Thr Glu Asp
   80 85 90
   Thr Ala Thr Tyr Tyr Cys Ala Arg Trp Asp Gly Gly Leu Thr Tyr
   95 100 105
   Trp Gly Gln Gly Thr Leu Val Thr Val Ser Ala Cys Ser Thr Lys
   110 115 120
   Gly Pro Ser Val Phe Pro Leu Ala Pro Ser Ser Lys Ser Thr Ser
   125 130 135
   Gly Gly Thr Ala Ala Leu Gly Cys Leu Val Lys Asp Tyr Phe Pro
   140 145 150
   Glu Pro Val Thr Val Ser Trp Asn Ser Gly Ala Leu Thr Ser Gly
   155 160 165
   Val His Thr Phe Pro Ala Val Leu Gln Ser Ser Gly Leu Tyr Ser
   170 175 180
   Leu Ser Ser Val Val Thr Val Pro Ser Ser Ser Leu Gly Thr Gln
   185 190 195
   Thr Tyr Ile Cys Asn Val Asn His Lys Pro Ser Asn Thr Lys Val
   200 205 210
   Asp Lys Lys Val Glu Pro Lys Ser Cys Asp Lys Thr His Thr Cys
   215 220 225
   Pro Pro Cys Pro Ala Pro Glu Leu Leu Gly Gly Pro Ser Val Phe
   230 235 240
   Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro
   260 265 270
   Glu Val Lys Phe Asn Trp Tyr Val Asp Gly Val Glu Val His Asn
   275 280 285
   Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly
   305 310 315
   Lys Glu Tyr Lys Cys Lys Val Ser Asn Lys Ala Leu Pro Ala Pro
   320 325 330
   Ile Glu Lys Thr Ile Ser Lys Ala Lys Gly Gln Pro Arg Glu Pro
   335 340 345
   Gln Val Tyr Thr Leu Pro Pro Ser Arg Glu Glu Met Thr Lys Asn
   350 355 360
   Gln Val Ser Leu Thr Cys Leu Val Lys Gly Phe Tyr Pro Ser Asp
   365 370 375
   Ile Ala Val Glu Trp Glu Ser Asn Gly Gln Pro Glu Asn Asn Tyr
   380 385 390
   Lys Thr Thr Pro Pro Val Leu Asp Ser Asp Gly Ser Phe Phe Leu
   395 400 405
   Tyr Ser Lys Leu Thr Val Asp Lys Ser Arg Trp Gln Gln Gly Asn
   410 415 420
   Val Phe Ser Cys Ser Val Met His Glu Ala Leu His Asn His Tyr
   425 430 435
   Thr Gln Lys Ser Leu Ser Leu Ser Pro Gly Lys
   440 445
<210> 40
   <211> 10
   <212> PRT
   <213> Artificial sequence
<220>
   <223> chemically synthesized
<400> 40
   Asn Trp Ile Arg Gln Cys Pro Gly Asn Lys
   5 10
<210> 41
   <211> 11
   <212> PRT
   <213> Artificial sequence
<220>
   <223> chemically synthesized
<400> 41
   Leu Asn Ser Cys Thr Thr Glu Asp Thr Ala Thr
   5 10
<210> 42
   <211> 21
   <212> PRT
   <213> Artificial sequence
<220>
   <223> chemically synthesized
<400> 42
   Gly Gln Gly Thr Leu Val Thr Val Ser Ala Cys Ser Thr Lys Gly
   1 5 10 15
   Pro Ser Val Phe Pro Leu
   20
<210> 43
   <211> 14
   <212> PRT
   <213> Artificial sequence
<220>
   <223> chemically synthesized
<400> 43
   His Thr Phe Pro Cys Val Leu Gln Ser Ser Gly Leu Tyr Ser
   5 10
<210> 44
   <211> 14
   <212> PRT
   <213> Artificial sequence
<220>
   <223> chemically synthesized
<400> 44
   His Thr Phe Pro Ala Cys Leu Gln Ser Ser Gly Leu Tyr Ser
   5 10
<210> 45
   <211> 11
   <212> PRT
   <213> Artificial sequence
<220>
   <223> chemically synthesized
<400> 45
   Phe Leu Ser Val Ser Cys Gly Gly Arg Val Thr
   5 10
<210> 46
   <211> 11
   <212> PRT
   <213> Artificial sequence
<220>
   <223> chemically synthesized
<400> 46
   Gln Lys Pro Gly Asn Cys Pro Arg Leu Leu Ile
   5 10
<210> 47
   <211> 11
   <212> PRT
   <213> Artificial sequence
<220>
   <223> chemically synthesized
<400> 47
   Glu Ile Lys Arg Thr Cys Ala Ala Pro Ser Val
   5 10
<210> 48
   <211> 11
   <212> PRT
   <213> Artificial sequence
<220>
   <223> artificial protein
<400> 48
   Phe Tyr Pro Arg Glu Cys Lys Val Gln Trp Lys
                   5                   10
<210> 49
   <211> 11
   <212> PRT
   <213> Artificial sequence
<220>
   <223> chemically synthesized
<400> 49
   Val Thr Glu Gln Asp Cys Lys Asp Ser Thr Tyr
                   5                   10